# EUROPEAN PATENT SPECIFICATION

(11) **EP 3 994 282 B1**
(45) Date of publication and mention of the grant of the patent: **02.09.2026**
(21) Application number: 20736685.7
(22) Date of filing: 03.07.2020
(51) Int. Cl.: C12Q 1/6886

(54) **DETERMINING INDIVIDUAL HLA PATTERNS, USE AS PROGNOSTICATORS, TARGET GENES AND THERAPEUTIC AGENTS**
BESTIMMUNG INDIVIDUELLER HLA-MUSTER, VERWENDUNG ALS PROGNOSTIKATOREN, ZIELGENE UND THERAPEUTISCHE MITTEL
DÉTERMINATION DE MOTIFS DE HLA INDIVIDUELS, UTILISATION EN TANT QU'OUTILS PRONOSTIQUES, GÈNES CIBLES ET AGENTS THÉRAPEUTIQUES

(30) Priority: 05.07.2019 DE 102019004747
(43) Date of publication of application: 11.05.2022
(73) Proprietor: Intellexon GmbH, 82343 Pöcking (DE)
(72) Inventor: WÜRFEL, Wolfgang, 85235 Odelzhausen (DE); WIRTZ, Ralph M., 50677 Köln (DE); WINTERHALTER, Christoph, 82343 Pöcking (DE)
(74) Representative: Samson & Partner Patentanwälte mbB
(86) International application number: PCT/EP2020/068814
(87) International publication number: WO 2021/004934

(56) References cited:
- EP-A1- 3 284 479
- EP-A2- 2 808 402
- WO-A1-2009/040220
- WO-A1-2020/077128
- WO-A2-2016/183041
- CN-A- 109 801 678
- US-A1- 2004 209 296
- US-A1- 2010 279 889
- US-A1- 2016 097 105
- US-A1- 2016 265 050
- US-A1- 2017 067 118
- US-A1- 2019 175 709
- JIM SHEU ET AL: "HLA-G and Immune Evasion in Cancer Cells", JOURNAL OF THE FORMOSAN MEDICAL ASSOCIATION., vol. 109, no. 4, 1 April 2010 (2010-04-01), HK, pages 248 - 257, XP055486135, ISSN: 0929-6646, DOI: 10.1016/S0929-6646(10)60050-2

## Description

### TECHNICAL FIELD OF THE INVENTION

The present invention relates to *in vitro* methods of determining the individual HLA patterns (adult and/or embryonic) in body samples, in particular tissue or blood samples of cancer patients and/or patients suffering disorders related to autoimmune disease and to methods of stratifying said patients for tailored treatments.

### BACKGROUND OF THE INVENTION

The chemical or biological tumor therapy has not yet focused on individual tumor cell types of a single patient but still detects in general all rapidly dividing cells, independent of whether or not they are malignant and independent of the individual patient.

In the final result, this type of therapy is often ineffective and/or accompanied by severe side-effects. A more targeted, highly individual therapy would be extremely useful.

The basis for the well-regulated existence of an entire organism is the communication between the cells or the cellular dialogue. This dialogue and its regulation enable to maintain the existence of an entire organism even though cells constantly die and/or are reproduced. As a result of this dialogue, the differentiation of cells is also regulated, as known from stem cell research. This dialogue even enables the well-regulated cooperation of two different cell clones even if one shows an extremely fast growth as is the case during pregnancy.

The basis of the cellular dialogue in humans is the MHC (major histocompatibility complex) with its HLA groups. The identification of a cell by HLA groups is the basis of every cellular communication. The cellular communication develops in cooperation with specific receptors, such as the killer-immunoglobulin-like receptors (KIR) on the natural killer cells (NK cells) or the LILR (leukocyte immunoglobulin-like receptors), with subsequent involvement of further factors, such as cytokines, growth factors, etc.

Various HLA groups are known which can be described as follows:
HLA groups A, B, and C (MHC I): they identify substantially all adult and somatic cells.
HLA groups D (DR, DP, DQ, etc.; MHC II): They play an important role in immunocompetent cells and/or in the antigen presentation.
HLA groups E, F and G: They identify embryonic cells, in particular on the so-called front of invasion.

In addition, the MHC complex also comprises further substances such as the complement factors which belong to class III.

A tumor cell basically has a genetic code the same as that of any other cell of the entire organism. Therefore, it does not have any information other than that of the entire organism with respect to cell division and cell differentiation. As a result, every malignant tumor disease is unique and individual, i.e. specific to the respective organism.

In some tumor diseases, additional genetic information is introduced into the cell from outside, e.g. by viral vectors, such as the so-called oncogenes. However, oncogenes can also form an integral part of the genetic material from procreation on. Oncogenes and/or the activation thereof and other external factors can permanently affect the biology of a tumor cell. Yet, the tumor cells stay involved in the cellular dialogue of the entire organism and the regularities valid therein.

Organisms having a high cellular differentiation, such as human beings, "pay" for their high differentiation by a loss of multipotency or totipotency. In the case of organ losses, restitution ad integrum is no longer possible but only the repair by connective tissue. When a living being is not so strongly differentiated, such as the starfish, the loss of totipotency or multipotency is not so distinct and therefore when an arm is lost, for example, a new arm can grow again even though it is smaller.

Totipotency is basically encoded in the genetic material of higher living beings as well. This is proved by the simple fact that this genetic material formerly had to control the development from a fertilized egg cell to a differentiated organism. Cloning experiments also show that "resetting" of the genetic material ("reprogramming") of even highly differentiated cells, such as the udder cells of the cloned sheep "Dolly", "to zero" is possible in the nucleus. In the final analysis, this also applies to the procreation and/or fertilization of an egg cell where the genetic material of two relatively old individuals (father and mother to be) is reset "to zero" and is encoded again for the development of a new living being.

Correspondingly, it is clear that a tumor cell is also provided with genetic material that fundamentally codes for all growth and differentiation processes which are at all possible in an entire organism, i.e. also for the mechanisms of the initial embryonic implantation, of the early embryo-maternal cell dialog and of the subsequent embryonic-fetal development.

Every tumor cell loses its differentiation in varying degrees (is thus "dedifferentiated") and makes its "way back" in varying degrees. Essential characteristics of this "way back" are the loss of cellular differentiation and the loss of specific cell performances and also the (re)gaining of uncontrolled cell growth.

It is known that tumor cells can express typical embryonic HLA groups on their surface. Although the respective investigations are still fragmentary, this expression of embryonic HLA groups contributes to the circumstance that tumor cells evade the attack of the unspecific immune defense of the own organism. The expression of these typical HLA groups on the surface enables cells to activate corresponding receptors e.g. on the NK cells but also the lymphocytes and further immunocompetent cells, and therefore there is not only no attack of the unspecific immune defense, i.e. the NK cells and lymphocytes, but also in the individual case tumor cells (and also embryonic cells) are able to "let the immune defense work for them", namely by a synthesis of growth factors and cytokines which are beneficial to the own development.

Here, e.g. the phenomenon of TAM (tumor-associated macrophages) or MDSC (tolerogenic "myeloid-derived suppressor cells") has to be mentioned. They even support the tumor growth in the micro-environment of a malignant tumor (i.e. "are turned"). The same applies similarly to cytokines, such as MIF (macrophage migration inhibition factor) which is likely to be produced in the tumor (likely by NK cells) and has a proangiogenetic effect, thus supporting the proliferation and migration of tumor cells.

Tumor cells need not be very resistant. As is known, they are more sensitive to chemotherapy and also more sensitive to radiation than "healthy" and differentiated standard cells. Their cell division rate is not particularly high either. The danger resulting from a malignant tumor cell is above all that it is able on the basis of the cellular communication to "enforce" the progressing uncontrolled growth.

This circumstance is also illustrated by the fact that according to current knowledge metastases predominantly form because malignant cells which can be referred to as "malignant stem cells" spread and colonize. Should this be correct, such "malignant stem cells" should by means of the cellular communication with the adjacent tissue also be locally able to evade the growth control and the differentiation pressure. Even if formed from a dedifferentiation, stem cells would behave like stem cells in general, which in this case directs the focus in particular on the mode of functioning of the embryomaternal communication (of the embryomaternal dialogue).

The malignant degeneration of a cell is a unique process which is specific to every individual. This is not altered by the fact that there are pathologically well classifiable (always recurring) tumor types across individuals. This circumstance is rather a proof for the fact that a malignant tumor does not form by every dedifferentiation and every "way back". It is rather likely that only certain constellations can "survive" on the "way back", thus resulting in the typical tumor entities across individuals.

The dedifferentiation or "degeneration" of a cell is presumably a comparatively ubiquitous process in every entire organism. However, it almost never leads to the formation of a tumor disease since only some few of these cells have the cell-biological and also communicative preconditions (from cell to cell) that are necessary for the survivability. The cells which show survivability use the two above mentioned mechanisms, presumably in a combined form. On the one hand, they use the recovery of the embryomaternal communication to evade an attack of the immune system and even get support from this communication in the course of cell growth; on the other hand, they can evade an attack of the specific immune defense while being protected by the completely or partially expressed (originally adult) HLA patterns (and also those which correspond to those of the own mother (see below)), thus expanding.

The "acquired" immunity develops during pregnancy and means that there is not only a tolerance with respect to the body's own HLA groups but also always with respect to the foreign adult HLA groups of one's own mother.

According to current knowledge tumor cells basically express the same adult HLA groups as all other somatic cells of the entire organism as transmembrane spanning proteins presenting peptides as antigens in a cleft of constituted by the extracellular alpha 1 and alpha 2 domain, while being bound to beta-microglobulin as co-factor in the complex. As a result, it is protected from an attack of the specific immune defense. This also applies in principle when parts of the original HLA pattern are lost "on their way back", are expressed less densely or are available in a changed, i.e. corrupted, form (which is not atypical for tumor cells).

It is here useful to make a comparison with an embryo's embryonic cells which were flushed out in the maternal organism and persist therein (this is referred to as "microchimerism). The embryonic surface structures, in particular HLA-G, -E and -F, on the embryonic cells (predominantly placental or trophoblastoid) prevent the mother's immune system from attacking the cells.

If a certain differentiation of the embryonic cells has already taken place (here usually the embryo's genuine cells), they are specifically incorporated into corresponding organs. This very strongly reflects the behavior of malignant tumors which often prefer a certain metastasis formation pattern.

When the embryonic surface structures of the embryo cells are maintained, the embryonic cells are presumably not attacked throughout the mother's life - similar to tumor cells.

If during pregnancy very many embryonic cells flush out into the maternal organism, this tolerance can even lead to an attempt to "take over" the mother's body, i.e. a "graft-versus-host" reaction results, as in the case of the quite threatening HELLP syndrome (hemolysis, elevated liver enzymes, low platelet count), for example.

However, there can also be counter-reactions to microchimerism, which are presumably due to the fact that the embryonic cells at least partially lose their HLA-G, E, F protection, optionally accompanied by further differentiation.

Then, there are inflammatory counter-reactions of the maternal organism since the embryonic cells show "adult" HLA structures when differentiating which differ from those of the maternal organism. As a typical result, connective tissue forms around the site of inflammation and this can cause certain immune diseases (cf. Hashimoto's thyreoiditis).

This inflammatory counter-reaction of the organism does not take place in the case of tumor cells since the tumor cells form HLA characteristics on the surface during further differentiation, said characteristics not differing from those of the host organism.

This complete or incomplete expression of the adult HLA patterns prevents an attack of the specific immune defense even when the tumor cell stands out by additional antigen expressions (or overexpressions) as known and described for tumor cells. The protection resulting from the complete or incomplete expression of the original adult HLA patterns is obviously very effective such that a tumor cell can express its specific antigen patterns (thus "standing out" as a result) without an effective attack of the (specific) immune response, i.e. also of the B lymphocytes and T lymphocytes occurring. It is remarkable that the tumor antigen expression patterns are relatively specific to individual tumor types. It is also possible that changed and/or mutated MHC-/HLA groups make the antigen presentation cascade (APM (antigen processing machinery)) more and more faulty and therefore typical, human-associated or own antigens are hardly presented or are not presented (any more). However the molecular mechanisms underlying such putative processes are unclear and have not yet been identified.

When an entire organism is confronted with a somatic cell whose HLA expression pattern strongly or fully differs from that of the organism or the mother, it has to be assumed that there will be an attack of the immune defense accompanied by the formation of memory cells. The "memory" is, of course, predominantly directed against divergent HLA groups. However, it is known that such an attack of the immune system followed by a destruction of the foreign cells is also accompanied by the formation of antibodies against other surface structures. In individual cases, antibodies are even formed against ubiquitous cell constituents, such as phospholipids ("antiphospholipid syndrome").

Against this background, it is necessary to consider both mechanisms as an entirety, namely as an entirety of how the "tumor cell on its way back" communicates with the other cells and the immune system and/or how lost communication characteristics of the embryonic period are recovered so as to develop from this understanding a therapy concept considering these basic principles and corresponding medicaments.

Besides the specific immune defense (i.e. T- and B-lymphocytes), monocytes and the macrophages resulting from them also play a role in tumor growth. However, macrophages can only be activated when cells of the unspecific immune defense (such as NK killer cells) or the specific immune defense (such as T-cells or B-cells) are present. However, this requires "priming" where antigen-presenting cells (such as the dendritic cells) present mutated or "foreign" proteins, thus leading to the formation of cytotoxic T-cells. Here, numerous cytokines, such as interferon γ (IFN-γ) or the tumor necrosis factor (TNF-α) play a role as well.

A comparison between embryonic cells and tumor cells offers itself in the case of the macrophages as well. Macrophages can be found in the basal endometrium in the case of an establishing pregnancy. They usually have an inhibitory effect on the invasion behavior of the embryo and form so to speak a "protective wall" between the implanting embryo and the myometrium. On the other hand, the embryo secretes macrophage migration inhibiting factors, i.e. factors which limit and inhibit the attack of the macrophages. This applies likewise to malignant tumors (see above).

In conclusion the current understanding is based on a direct cell-to-cell communication between tumor cells and immune cells based on membrane bound MHC I mediated peptide presentation and subsequent binding to specific receptors on immune cells (such as KIR and LILR).

The molecular basis for the communication between tumor cells and the immune system is still poorly understood. With the raise of antibody based treatments attacking the so called "CheckPoint" the determination of the respective target structures (such as quantitation of PD-L1 expression on mRNA or protein basis) and/or determination of tumor mutational burden and/or MSI status has been shown to have some impact when predicting response to treatment or prolonged survival (DFS, MFS, DSS or OS).

The term "predicting an outcome" of a disease, as used herein, is meant to include both a prediction of an outcome of a patient undergoing a given therapy and a prognosis of a patient who is not treated. The term "predicting an outcome" may, in particular, relate to the risk of a patient suffering an event, such as metastasis or death, preferably within a given time frame.

In conclusion, the medical need to better classify cancer patients for their individual risk of recurrence and response to chemotherapy or alternative treatment options is high for multiple kinds of tumors.

This may involve on the one hand the determination of tumor cell specific characteristic such as mutations (KRAS, NRAS, EGFR, cMET, HER2, ESR1, FGRF3, etc.), molecular subtyping transcripts (such as ESR1 PGR, ERBB2; proliferation genes such as MKI67, RACGAP1, BIRCS, MYBL1, FOXM1; Keratins such as KRT4, KRT5, KRT17, KRT18, KRT19, KRT20, etc.; EMT markers such as SNAI1, SNAI2, FOXA1 etc.), immune genes (such as CD3, CD8, CD19, CD68, CD168, CSF1R, IGKC, IGHM, IFNG), check point genes (such as PD-L1, PD-L2, CD86, CD80, L-ICOS, B7-H3, B7-H4; PD1, CTLA4, CD28, ICOS), on the other hand the determination of HLA expression patterns ("HLA typing") as described in this disclosure.

For instance, for the very heterogeneous groups of both bladder cancer stages the optimal therapeutic procedure is not clear as the prognosis cannot be reliably assessed by current clinicopathological features.

The current worldwide applied standard methodology for the detection of the receptor status of cancers, e.g., breast cancers, is immunohistochemistry (IHC) from formalin-fixed and paraffin-embedded (FFPE) biopsy or resection tissue. In breast cancer, administration of endocrine or targeted systemic treatment (e.g., with trastuzumab) is mostly based on IHC. However, IHC-based approaches to determine the individual risk by testing a plurality of markers do not result in a reliable prognostic subclassification of bladder cancer that could help to resolve the diagnostic/therapeutic dilemma described above. Furthermore, testing by IHC generally suffers from a lack of sensitivity.

US 2016/265050 A1 relates to methods for determining whether a subject shows an immune response. US 2004/209296 A1 relates to methods for selecting tumors expressing HLA-G which are sensitive to anticancer treatment, and uses thereof. Both documents provide technical background to the present disclosure.

Therefore, there is clearly a need for a reliable, objective, quantitative and reproducible test system for the molecular subtyping of bladder cancer, which enables reliable individual risk assessment, facilitates the selection of suitable tumor treatment regimens (i.e., patient stratification), and allows prognosis and prediction of therapy success. Moreover, such test system should allow for decentralized testing that is suitable for a significant proportion of cancer patients.

These and other objects are solved by the present invention, which will be described in the following.

### SUMMARY OF THE INVENTION

The present invention is defined in the appended claims. In particular, it relates to *in vitro* methods of determining the individual HLA patterns (adult and/or embryonic) in body samples (tissue or blood samples) of cancer patients and/or patients suffering disorders related to autoimmune disease and to methods of stratifying said patients for tailored treatments.

Disclosed, but not part of the claimed invention, are kits and their uses, as well as to nucleic acid molecules as prognostic biomarkers for neoplastic disease such as cancer, autoimmune disease, infectious disease and conditions related to pregnancy. Disclosed, but not part of the claimed invention, are therapeutic agents and to methods of producing therapeutic agents.

The terms "sample", "biological sample", or "clinical sample", as used herein, refer to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, liquor cerebrospinalis, tear fluid, or cells there from. Biological samples may also include sections of tissues such as frozen or fixed sections taken for histological purposes or microdissected cells or extracellular parts thereof. A biological sample to be analyzed is tissue material from a neoplastic lesion taken by aspiration or punctuation, excision or by any other surgical method leading to biopsy or resected cellular material. Such a biological sample may comprise cells obtained from a patient. The cells may be found in a cell "smear" in solid tumor material, in a lavage fluid, or in a body fluid. The sample may be a processed sampel, e.g. a sample, which has been frozen, fixed, embedded or the like. A preferred type of sample is a formaline fixed paraffin embedded (FFPE) sample. Preparation of FFPE samples are standard medical practice and these samples can be conserved for long periods of time.

The term "patient", as used herein, refers to any organism such as vertebrate, particularly any mammal, including both a human and another mammal, e.g., an animal such as a rodent, a rabbit, or a monkey. The rodent may be a mouse, rat, hamster, guinea pig, or chinchilla. Preferably, the patient is a human.

Disclosed is a method of determining individual HLA patterns of a tumor, comprising: determining a first expression level of RNA transcript encoding a first region of a first HLA gene ; determining a second expression level of RNA transcript of a second region of a second HLA gene; and comparing the determined first and second expression levels to obtain an individual HLA pattern, wherein the first HLA gene and the second HLA gene are selected from the group consisting of genes encoding HLA-A, HLA-B, HLA-C, HLA-D, HLA-E, HLA-F, HLA-G, HLA-H- HLA-J.

In some embodiments, the first HLA gene and the second HLA gene may encode different HLA groups. For instance, the first HLA gene may encode one selected from the group consisting of HLA-A, HLA-B, HLA-C, HLA-D, HLA-E, HLA-F, HLA-G, HLA-H, HLA-J. Similarly, the second HLA gene may encode another one selected from the group consisting of HLA-A, HLA-B, HLA-C, HLA-D, HLA-E, HLA-F, HLA-G, HLA-H, HLA-J.

In such cases, the comparing of the first and second expression level may be termed "intergenic".

According to the present invention, the first HLA gene may encode a classical HLA gene, i.e. selected from the group consisting of HLA-A, HLA-B and HLA-C; while the second HLA gene may encode a non-classical HLA gene or pseudogene, i.e. selected from the group consisting of HLA-D, HLA-E, HLA-F, HLA-G, HLA-H, and HLA-J.

In particular, the present disclosure relates to *an in vitro* method not part of the claimed invention, of determining individual HLA patterns of a tumor in a patient, e.g. a cancer patient, said method comprising determining the expression level of RNA transcript of at least one gene selected from the group consisting of adult HLA groups (e.g. HLA-A, HLA-B, HLA-C; MHC I), HLA groups D (DR, DP, DQ, etc.; MHCII), "embryonic" HLA's (e.g. HLA-E, HLA-F, HLA-G), HLA pseudogenes (e.g. HLA-H, HLA-J) in a sample, e.g. of the tumor tissue or blood of a cancer patient.

According to the present invention, the term "RNA transcript" shall relate to transcription products in sense and/or antisense direction.

Preferably, the term includes and relates to "mRNA" which means "messenger RNA" and relates to a "transcript" which encodes a peptide or protein. mRNA typically comprises a 5' non translated region (5'-UTR), a protein or peptide coding region and a 3' non translated region (3'-UTR). mRNA has a limited halftime in cells and *in vitro.*

In other cases, such as for an antisense RNA (aRNA) transcript, the RNA may not encode for a peptide or protein. It may however be complementary to the mRNA and thereby regulate the translation of a corresponding sense mRNA into a peptide or protein. For purposes of the present disclosure, an antisense RNA transcript may be considered as relating to a further "region" (an antisense region) of the respective "HLA gene", although it is not directly translated into a HLA group protein or peptide.

In some cases, the first expression level and the second expression level may both relate to sense RNA transcripts. In other cases, the first expression level may relate to sense RNA transcript, whereas the second expression level may relate to antisense RNA transcript (or inversely). Preferably, but not necessarily, the sense and antisense transcripts relate to a same HLA group. For instance, the antisense transcript may be at least partially complementary to the sense transcript of the same HLA group.

The term "expression level" refers, e.g., to a determined level of gene expression. The term "pattern of expression levels" refers to a determined level of gene expression compared either to a reference gene, e.g. housekeeper, or inversely regulated genes, or to a computed average expression value, e.g. in DNA-chip analyses. A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other. Expression levels may also be assessed relative to expression in different tissues, e.g. expression of a gene in cancerous tissue vs. non-cancerous tissue.

The term "expression level", as used herein, refers to the expression of a particular gene (e.g., HLA-E, HLA-F, HLA-G) so as to produce transcript and/or protein. According to the present invention, the expression level is determined on the RNA transcript level, in particular mRNA level (transcriptional level), for example, by measuring the transcribed mRNA (e.g., via northern blot), by reverse transcription (RT) quantitative PCR (RT-qPCR) or by directly staining the mRNA (e.g., via *in situ* hybridization).

In some embodiments, the expression level is normalized against the (mean) expression level of one or more reference genes in the sample of the tumor. The term "reference gene", as used herein, is meant to refer to a gene which has a relatively invariable level of expression on the RNA transcript/mRNA level in the system which is being examined, i.e. cancer. Such gene may be referred to as housekeeping gene. In some embodiments, the one or more reference genes are selected from the group comprising CALM2, B2M, RPL37A, GUSB, HPRT1 and GAPDH, preferably CALM2 and/or B2M. Other suitable reference genes are known to a person skilled in the art.

Each of the first and second region may comprise an exon-exon-boundary or may comprise a portion of no more than one exon (i.e. not comprise an exon-exon boundary).

For instance, one of the first region and second region (e.g. the first region) may span portions of two exons (i.e. comprise an exon-exon-boundary) and the other one of the first region and second region (e.g. the second region) comprises a portion of no more than one exon (i.e. not comprise an exon-exon boundary.

Alternatively, the first region may comprise an exon-exon-boundary (i.e. span portions of two exons) and the second region may comprise an exon-exon-boundary. The first region and the second region may or may not comprise portions of a common exon. For instance, the first region may comprise the boundary between exon 2 and exon 3 (i.e. comprise the exon 2 / exon 3 boundary. In such cases, the second region may comprise portions of any exon (such as exon 1, exon 2, exon 3, exon 4 etc.) or it may comprise any exon-exon-boundary (such as exon 3 / exon 4 boundary, exon 4 / exon 5 boundary). The group of exon-exon boundaries also includes boundaries formed by exon skipping, such exon 2 / exon 4 - boundary etc.

In a further alternative, the first region comprises a portion of no more than one exon and the second region comprises a portion of no more than one exon.

In some embodiments, the first region may encode a signal peptide region of a HLA group and the second region may encode a transmembrane region of the of a HLA group.

In general, the method of determining individual HLA patterns may also comprise determining whether the individual HLA pattern is predominantly soluble or membrane-bound based on the comparison of the first and second expression levels. In particular, if the expression level of a region encoding a signal peptide region of a HLA group exceeds the expression level of a region encoding a transmembrane region of the HLA group, it may be determined that the individual HLA pattern is predominantly soluble. If the expression level of a region encoding a transmembrane region of a HLA group is essentially equal to or exceeds the expression level of a region encoding a signal peptide region of the HLA group, it may be determined that the individual HLA pattern is predominantly membrane-bound.

In general, the method of determining individual HLA pattern may also comprise determining HLA isoforms based on the comparison of the first and second expression levels. In particular, if the expression level of a region encoding a portion of a first exon exceeds the expression level of a region encoding a portion of a second exon, it may be determined that the individual HLA pattern comprises one or more isoforms, which comprise the first exon and do not comprise the second exon.

In general, the method of determining individual HLA pattern may also comprise determining one or more further expression levels (e.g. a third expression level) for one or more further regions (e.g. a third region) of HLA groups and wherein the comparing is further based on the determined further expression levels to obtain the individual HLA pattern.

The term "RNA expression level" refers to a determined level of the converted DNA gene sequence information into transcribed RNA, the initial unspliced RNA transcript or the mature mRNA. RNA expression can be monitored by measuring the levels of either the entire RNA of the gene or subsequences.

In some embodiments, determining the expression level of RNA transcript comprises determining whether the expression level of RNA transcript is lower or higher than a defined expression threshold of RNA transcript (= dichotomization). In cases where the expression level is equal to the defined expression threshold, the expression level may be considered to belong to the group of expression levels that are higher than the defined expression threshold. Thus, the wording "higher than a defined expression threshold", as used herein, includes expression levels that are higher than or equal to the defined expression threshold. Expression levels that are "higher than a defined expression threshold" may also be referred to as "expression-positive", whereas expression levels that are "lower than a defined expression threshold" may also be referred to as "expression-negative".

In some embodiments, the expression levels of RNA transcripts encoding the homologous region of the signal-peptide of one or more HLA's is determined and set into relation to the expression levels encoding for the homologous transmembrane region of one or more HLA's and/or the divergent cytoplasmic tail of one or more HLA's is set into relation, whereby the ratio of secreted alpha domains versus transmembrane localized HLA's can be determined for individual HLA's and HLA isoforms.

The step of "determining the expression level of RNA transcript" may comprise (i) measuring the expression level of RNA transcript and (ii) analyzing the measured expression level of RNA transcript (e.g., by comparison to a reference expression level, such as a defined expression threshold), wherein the order of measuring the expression level of RNA transcript may or may not be independent of the order of analyzing the measured expression level of RNA transcript.

In some embodiments, the expression levels of RNA transcript of at least one, two, three or four genes selected from the group consisting of HLA-A, HLA-B, HLA-C, HLA-D, HLA-E, HLA-F, HLA-G and HLA pseudogenes (HLA-H and/or HLA-J) are determined.

In some embodiments, the obtained individual HLA pattern may be indicative of the presence and/or absence and/or level of expression of one or more isoforms of HLA groups. For instance, known isoforms of HLA-G include HLA-G1, HLA-G2, HLA-G3, HLA-G4, HLA-G5, HLA-G6 and HLA-G7. The same applies to the further embryonic HLA groups HLA-E and HLA-F as well as to HLA pseudogenes HLA-H and HLA-J. In such embodiments, further currently unknown isoforms of HLA groups may be determined to be present.

The obtained individual HLA pattern indicative of the presence and/or level of expression of one or more isoforms of HLA groups may further by used for identifying a molecular subtype of a tumor and/or in a method of producing a therapeutic agent.

Additionally or alternatively, the indication of the presence and/or absence and/or level of expression of isoforms, in particular soluble isoforms, may be used for (a) stabilizing the implantation of an embryo in assisted reproduction, e.g. in vitro fertilisation, IVF, (b) reducing the risk of transplant rejection, e.g. in a host-versus-graft reaction, and/or (c) reducing the risk of or minimizing the impact of autoimmune surges. Such use may in particular comprise producing a medium or therapeutic agent comprising soluble and biologically active isoforms of HLA-E, HLA-F and/or HLA-G. Examples of such soluble and biologically active isoforms include HLA-G5.

In some embodiments, the method further comprises determining the expression level of RNA transcripts of at least one gene selected from the group of immune genes (such as CD3, CD8, CD19, CD68, CD168, CSF1R, IGKC, IGHM, IFNG) in a sample of the tumor.

As one embodiment of the disclosure the combination of HLA typing with determination of check point characteristics as exemplified by protein based and/or mRNA based assessment of PD-L1, PD-L2, CD86, CD80, L-ICOS, B7-H3, B7-H4; PD1, CTLA4, CD28 and/or ICOS is of particular interest in case of targeting check point genes by specific inhibitors such as humanized antibodies in a clinical situation of advanced cancer. The HLA typing provides HLA expression pattern information that adds value of solely quantitating check point target genes for response prediction towards chemotherapeutic agents and/or check point inhibitors (such as anti-PD1 or anti-PD-L1 or anti-CTLA4 drugs).

As one embodiment of the disclosure the combination of HLA typing with quantitation of immune cell infiltrates as exemplified by protein based and/or mRNA based assessment of immune genes (such as CD3, CD8, CD19, CD68, CD168, CSF1R, IGKC, IGHM, IFNG) adds value of solely quantitating check point target genes for response prediction towards chemotherapeutic agents and or anti check point drugs, particularly when predicting the response to neoadjuvant treatment strategies.

According to one or more embodiments , the immune check point inhibitor comprises at least one selected from the group consisting of: antibody, modified antibody format, antibody derivative or fragment retaining target binding properties, antibody-based binding protein, oligopeptide binder and antibody mimetic.

"Antibodies", also synonymously called "immunoglobulins" (Ig), are generally comprising four polypeptide chains, two heavy (H) chains and two light (L) chains, and are therefore multimeric proteins, or an equivalent Ig homologue thereof (e.g., a camelid nanobody, which comprises only a heavy chain, single domain antibodies (dAbs) which can be either be derived from a heavy or light chain); including full length functional mutants, variants, or derivatives thereof (including, but not limited to, murine, chimeric, humanized and fully human antibodies, which retain the essential epitope binding features of an Ig molecule, and including dual specific, bispecific, multispecific, and dual variable domain immunoglobulins; Immunoglobulin molecules can be of any class (e.g., IgG, IgE, IgM, IgD, IgA, and IgY), or subclass (e.g., IgG1, IgG2, IgG3, IgG4, IgA1, and IgA2) and allotype.

An "antibody-based binding protein", as used herein, may represent any protein that contains at least one antibody-derived V_{H}, V_{L}, or C_{H} immunoglobulin domain in the context of other non-immunoglobulin, or non-antibody derived components. Such antibody-based proteins include, but are not limited to (i) F_{c}-fusion proteins of binding proteins, including receptors or receptor components with all or parts of the immunoglobulin C_{H} domains, (ii) binding proteins, in which V_{H} and or V_{L} domains are coupled to alternative molecular scaffolds, or (iii) molecules, in which immunoglobulin V_{H}, and/or V_{L}, and/or C_{H} domains are combined and/or assembled in a fashion not normally found in naturally occurring antibodies or antibody fragments.

An "antibody derivative or fragment", as used herein, relates to a molecule comprising at least one polypeptide chain derived from an antibody that is not full length, including, but not limited to (i) a Fab fragment, which is a monovalent fragment consisting of the variable light (V_{L}), variable heavy (V_{H}), constant light (C_{L}) and constant heavy 1 (C_{H}1) domains; (ii) a F(ab')2 fragment, which is a bivalent fragment comprising two Fab fragments linked by a disulfide bridge at the hinge region; (iii) a heavy chain portion of a F_{ab} (F_{d}) fragment, which consists of the V_{H} and C_{H}1 domains; (iv) a variable fragment (Fᵥ) fragment, which consists of the V_{L} and V_{H} domains of a single arm of an antibody, (v) a domain antibody (dAb) fragment, which comprises a single variable domain; (vi) an isolated complementarity determining region (CDR); (vii) a single chain Fᵥ Fragment (scFᵥ); (viii) a diabody, which is a bivalent, bispecific antibody in which V_{H} and V_{L} domains are expressed on a single polypeptide chain, but using a linker that is too short to allow for pairing between the two domains on the same chain, thereby forcing the domains to pair with the complementarity domains of another chain and creating two antigen binding sites; and (ix) a linear antibody, which comprises a pair of tandem Fᵥ segments (V_{H}-C_{H}1-V_{H}-C_{H}1) which, together with complementarity light chain polypeptides, form a pair of antigen binding regions; and (x) other non-full length portions of immunoglobulin heavy and/or light chains, or mutants, variants, or derivatives thereof, alone or in any combination. In any case, said derivative or fragment retains target binding properties
The term "modified antibody format", as used herein, encompasses antibody-drug-conjugates, Polyalkylene oxide-modified scFv, Monobodies, Diabodies, Camelid Antibodies, Domain Antibodies, bi- or trispecific antibodies, IgA, or two IgG structures joined by a J chain and a secretory component, shark antibodies, new world primate framework + non-new world primate CDR, IgG4 antibodies with hinge region removed, IgG with two additional binding sites engineered into the CH3 domains, antibodies with altered Fc region to enhance affinity for Fc gamma receptors, dimerised constructs comprising CH3+VL+VH, and the like.

The term "antibody mimetic", as used herein, refers to proteins not belonging to the immunoglobulin family, and even non-proteins such as aptamers, or synthetic polymers. Some types have an antibody-like beta-sheet structure. Potential advantages of "antibody mimetics" or "alternative scaffolds" over antibodies are better solubility, higher tissue penetration, higher stability towards heat and enzymes, and comparatively low production costs.

Some antibody mimetics can be provided in large libraries, which offer specific binding candidates against every conceivable target. Just like with antibodies, target specific antibody mimetics can be developed by use of High Throughput Screening (HTS) technologies as well as with established display technologies, just like phage display, bacterial display, yeast or mammalian display. Currently developed antibody mimetics encompass, for example, ankyrin repeat proteins (called DARPins), C-type lectins, A-domain proteins of S. aureus, transferrins, lipocalins, 10th type III domains of fibronectin, Kunitz domain protease inhibitors, ubiquitin derived binders (called affilins), gamma crystallin derived binders, cysteine knots or knottins, thioredoxin A scaffold based binders, SH-3 domains, stradobodies, "A domains" of membrane receptors stabilised by disulfide bonds and Ca2+, CTLA4-based compounds, Fyn SH3, and aptamers (peptide molecules that bind to a specific target molecules).

According to one or more embodiments of the disclosure, the immune check point inhibitor comprises at least one selected from the group as set forth in **Table 1.** In **Table 1,** DART designates 'Dual-Affinity Re-Targeting'; mAb designates 'monoclonal antibody'; NA designates 'not applicable'.

**Table 1: Immune check point inhibitors**

| **Target** | **Drug name** | **Drug type** | **mAb isotype** |
|---|---|---|---|
| CTLA-4 | Ipilimumab | Human mAb | IgG1k |
| | Tremelimum ab | Human mAb | IgG2k |
| | AGEN-1884 | Human mAb | IgG1 |
| PD-1 | Pembrolizum ab | Humanized mAb | IgG4k |
| | Nivolumab | Human mAb | IgG4k |
| | PDR001 | Humanized mAb | IgG4 |
| | SHR1210 | Humanized mAb | IgG4k |
| | Cemiplimab | Human mAb | *IgG4* |
| | REGN28 10 | *Human mAb* | IgG4 |
| | Pidilizumab | Humanized mAb | IgG1k |
| | AMP 514 | Humanized mAb | IgG4k |
| | BGB A317 | Humanized mAb | IgG4 |
| | PF-06801591 | mAb | - |
| | AMP224 | Fusion protein of PD-L2 and Fc domain of human IgG | NA |
| PD-L1 | Atezolizuma b | Humanized mAb | IgG1k |
| | Durvalumab | Human mAb | IgG1k |
| | Avelumab | Human mAb | IgG1k |
| | CK-301 | Checkpoint Therapeutics | fully human antibody |
| | BMS 936559 | Human mAb | IgG4 |
| 7-H3 | MGA-271 | Humanized mAb | IgG1 |
| | MGD-009 | B7-H3 x CD3 DART protein | NA |
| LAG-3 | IMP-321 | LAG-3 and human IgG1 fusion protein | NA |
| | BMS-986016 | mAb | - |
| | LAG-525 | Humanized mAb | IgG4 |
| TIM-3 | TSR-022 | Humanized mAb | IgG4 |
| | MBG-453 | mAb | - |
| VISTA | CA-170 | Small-molecule antagonist | NA |
| GITR | TRX-518 | Humanized mAb | IgG1 |
| | INCAGN018 76 | Human mAb | IgG1 |
| | GWN-323 | Human mAb | IgG1 |
| | MEDI1873 | Human mAb | IgG1 |
| | MK-4166 | Human mAb | IgG1 |
| | MK-1248 | mAb | - |
| | BMS986156 | mAb | - |
| CD27 | Varlilumab | Human | IgG1k |
| CD70 | SGN-CD70A | mAb | - |
| CD40 | ISF35 | Adenovirus vector | NA |
| | RO70097890 | mAb | - |
| OX40 | MEDI-6469 | mAb | Murine IgG1 |
| | MOXR-0916 | Humanized mAb | IgG1 |
| | PF-04518600 | Human mAb | IgG2 |
| | MEDI-0562 | Humanized mAb | IgG1 |
| 4-1BB | Urelumab | Human mAb | IgG4k |
| | Utomilumab | Human mAb | IgG2 |

In another aspect, the disclosure relates to use of the method as described above in the treatment of cancer, the use comprising, as a first step, stratifying a cancer patient for tumor treatment and, as a second step, providing the selected anti HLA tumor treatment regimen to the cancer patient.

"Stratifying a cancer patient for tumor treatment" in accordance with the present invention comprises the allocation of the cancer patient to a patient group having a particular molecular tumor subtype, which then allows the medical practitioner to select the most suitable tumor treatment regimen.

In some embodiments of this disclosure said treatments comprise the usage of humanized antibodies or the RNA or protein based immunization raised against specific HLA isoforms thereof for patients suffering neoplastic diseases.

In some embodiments, said use may comprise the production of soluble HLA domains ex vivo. For instance, said production may comprise combining either naturally occurring alpha domains as synthetic monomers (α1, α2, α3) or multimers in naturally occurring order (e.g. α1α2α3, α1α2, α1α3) or de novo order (α2α3) or de novo concatemers (e.g. α1α1α1, α2α2α2, α3α3α3, α1α1α2, α1α1α3, α1α1α1α1α2α2α2α3α3α3, etc.).

Said therapeutic agents, may be for application to patients suffering disorders related to autoimmune diseases or pregnant women being at potential risk of premature abortion to increase immune tolerance for diminishing autoimmune symptoms and enabling continuation of pregnancy until birth.

In general, a method of producing a therapeutic agent may comprise determining an individual HLA pattern using a method as described above and producing a therapeutic agent.

In some embodiments, the therapeutic agent may comprise proteins, protein domains and/or polypeptides such that the therapeutic agent binds specifically the determined individual HLA pattern. As a result of such binding of the therapeutic agent, binding or interaction between the determined individual HLA pattern and ligands or receptors, e.g. on immunocompetent cells, may be blocked.

For instance, the therapeutic agent may comprise soluble HLA domains or antibodies based on the determined individual HLA pattern.

In some embodiments, the therapeutic agent may comprise nucleic acids, in particular RNA. Such RNA may encode an antigen, which may be synthesized in by the immune system after injection of the therapeutic agent, in line with known RNA vaccination techniques. As a result of such translation of the therapeutic agent into an antigen, the response of immunocompetent cells may be triggered despite the determined individual HLA pattern.

For instance, the therapeutic agent may comprise soluble HLA domains or antibodies based on the determined individual HLA pattern.

In some embodiments all of the aforementioned synthetic alpha domain combinations may occur in *CIS* (i.e. by combining alpha domains of only one singular HLA gene such as HLA-G or HLA-F or HLA-E or HLA-A) or in *TRANS* (i.e. by combining alpha domains of more than one HLA gene such as HLA-G with HLA-E or HLA-F or HLA-A; HLA-A with HLA-E or HLA-F or HLA-G; etc,).

In some embodiments the aforementioned synthetic alpha domains of HLA-A, HLA-B, HLA-C, HLA-D, HLA-E, HLA-F are biotechnological engineered to contain additional Cysteins in positions similar to the substitutions within the HLA-G to obtain soluble HLA alpha domains capable of dimerization and/or multimerization for decreased diffusion and increased local depots of applied HLA alpha domains.

In yet another aspect, the disclosure relates to a kit which is not part of the claimed invention, for identifying a molecular subtype of a tumor, e.g. in a bladder cancer patient, by means of reverse transcription (RT) quantitative PCR (RT-qPCR), said kit comprising at least one pair of primers and at least one probe that are specific for a gene selected from the group consisting of HLA-A, HLA-B, HLA-C, HLA-D, HLA-E, HLA-F, HLA-G, HLA-H, HLA-J.

It also relates to the use of a kit as described above for identifying a molecular subtype of a tumor. The term "bladder cancer" relates to a type of cancer originating from bladder or urethral tissue. In some embodiments, the bladder cancer is non-muscle-invasive bladder cancer (NMIBC) or muscle-invasive bladder cancer (MIBC). Occasionally, bladder cancer is metastatic. Common sites of metastasis include bone, liver, lung and brain. Bladder cancer occurs in humans and other mammals. While the majority of human cases occur in men, female bladder cancer can also occur. Generally, treatment of bladder cancer may include surgery, medications (such as immunotherapy and/or chemotherapy and/or immunotherapy by BCG), radiation and/or targeted therapy.

Nearly all bladder cancers start in the urothelium. As the cancer grows into or through the other layers in the bladder, it then becomes more advanced, wherein the classification into stages follows the progression of infiltration of the tumor in deeper tissue layers. The stages are defined as: Ta (pTa): Non-invasive papillary carcinoma, Tis (pTis): Non-invasive flat carcinoma (flat carcinoma in situ, or CIS), T1 (pT1): The tumor has grown from the layer of cells lining the bladder into the connective tissue below but is still considered being a NMIBC, T2 (pT2): The tumor has grown into the muscle layer (MIBC), T3 (pT3): The tumor has grown through the muscle layer of the bladder and into the fatty tissue layer that surrounds it; T4 (pT4): The tumor has spread beyond the fatty tissue and into nearby organs or structures. It may be growing into any of the following: the stroma (main tissue) of the prostate, the seminal vesicles, uterus, vagina, pelvic wall, or abdominal wall.

"Primer pairs" and "probes", within the meaning of the invention, shall have the ordinary meaning of this term which is well known to the person skilled in the art of molecular biology. In a preferred embodiment of the invention "primer pairs" and "probes", shall be understood as being polynucleotide molecules having a sequence identical, complementary, homologous, or homologous to the complement of regions of a target polynucleotide which is to be detected or quantified. In some embodiments, nucleotide analogues are also comprised for usage as primers and/or probes. Probe technologies used for kinetic or real time PCR applications could be e.g. TaqMan^{®} systems obtainable at Roche Molecular Diagnostics, extension probes such as Scorpion^{®} Primers, Dual Hybridisation Probes, Amplifluor^{®} obtainable at Chemicon International, Inc, or Minor Groove Binders.

In some embodiments, the kit comprises specific pairs of primers and specific probes for at least two, three or four genes selected from the group consisting of HLA-A, HLA-B, HLA-C, HLA-D, HLA-E, HLA-F, HLA-G, HLA-H, HLA-J.

In some embodiments, the kit comprises:
- at least one pair of HLA-G -specific primers and at least one HLA-E -specific probe;
- at least one pair of HLA-G -specific primers and at least one HLA-F -specific probe; and/or
- at least one pair of HLA-F-specific primers and at least one HLA-G-specific probe.

In some embodiments, the kit comprises at least one pair of HLA-group-specific primers and at least one HLA-group-specific probe, wherein the pair of primes and the probe are both specific for a gene selected from the group consisting of HLA-A, HLA-B, HLA-C, HLA-D, HLA-E, HLA-F, HLA-G, HLA-H, HLA-J.

Preferably, primers for use in accordance with the present disclosure have a length of 15 to 30 nucleotides, in particular deoxyribonucleotides. In some embodiments, the primers are designed so as to (1) be specific for the target mRNA-sequence (e.g., HLA-E, HLA-F or HLA-G), (2) provide an amplicon size of less than 120 bp (preferably less than 100 bp), (3) detect all known protein-encoding splicing variants, (4) not include known polymorphisms (e.g., single nucleotide polymorphisms, SNPs), (5) be mRNA-specific (consideration of exons/introns; preferably no amplification of DNA), (6) have no tendency to dimerize and/or (7) have a melting temperature Tₘ in the range of from 58°C to 62°C (preferably, Tₘ is approximately 60°C).

As used herein, the term "nucleotide" includes native (naturally occurring) nucleotides, which include a nitrogenous base selected from the group consisting of adenine (A), thymidine (T), cytosine (C), guanine (G) and uracil (U), a sugar selected from the group of ribose, arabinose, xylose, and pyranose, and deoxyribose (the combination of the base and sugar generally referred to as a "nucleoside"), and one to three phosphate groups, and which can form phosphodiester internucleosidyl linkages. Further, as used herein, "nucleotide" refers to nucleotide analogues. As used herein, "nucleotide analogue" shall mean an analogue of A, G, C, T or U (that is, an analogue of a nucleotide comprising the base A, G, C, T or U) which is recognized by DNA or RNA polymerase (whichever is applicable) and incorporated into a strand of DNA or RNA (whichever is appropriate). Examples of such nucleotide analogues include, without limitation, 5-propynyl pyrimidines (i.e., 5-propynyl-dTTP and 5-propynyl-dCTP), 7-deaza purines (i.e., 7-deaza-dATP and 7-deaza-dGTP), aminoallyl-dNTPs, biotin-AA-dNTPs, 2-amino-dATP, 5-methyl-dCTP, 5-iodo-dUTP, 5-bromo-dUTP, 5-fluoro-dUTP, N4-methyl-dCTP, 2-thio-dTTP, 4-thio-dTTP and alpha-thio-dNTPs. Also included are labelled analogues, e.g. fluorescent analogues such as DEAC-propylenediamine (PDA)-ATP, analogues based on morpholino nucleoside analogues as well as locked nucleic acid (LNA) analogues.

The wording "specific for the target mRNA-sequence", as used in connection with primers for use in accordance with the present invention, is meant to refer to the ability of the primer to hybridize (i.e. anneal) to the cDNA of the target mRNA-sequence under appropriate conditions of temperature and solution ionic strength, in particular PCR conditions. The conditions of temperature and solution ionic strength determine the stringency of hybridization. Hybridization requires that the two nucleic acids (i.e. primer and cDNA) contain complementary sequences, although depending on the stringency of the hybridization, mismatches between bases are possible. In some embodiments, "appropriate conditions of temperature and solution ionic strength" refer to a temperature in the range of from 58°C to 62°C (preferably a temperature of approximately 60°C) and a solution ionic strength commonly used in PCR reaction mixtures. In some embodiments, the sequence of the primer is 80%, preferably 85%, more preferably 90%, even more preferably 95%, 96%, 97%, 98%, 99% or 100% complementary to the corresponding sequence of the cDNA of the target mRNA-sequence, as determined by sequence comparison algorithms known in the art.

For instance, the primer may hybridize to the cDNA of the target mRNA-sequence under stringent or moderately stringent hybridization conditions. "Stringent hybridization conditions", as described herein, may involve hybridizing at 68°C in 5x SSC/5x Denhardt's solution/1,0% SDS, and washing in 0,2x SSC/0,1 % SDS at room temperature, or involve the art-recognized equivalent thereof (e.g., conditions in which a hybridization is carried out at 60°C in 2,5x SSC buffer, followed by several washing steps at 37°C in a low buffer concentration, and remains stable). "Moderately stringent hybridization conditions", as defined herein, involve including washing in 3x SSC at 42°C, or the art-recognized equivalent thereof. The parameters of salt concentration and temperature can be varied to achieve the optimal level of identity between the primer and the target nucleic acid. Guidance regarding such conditions is available in the art, for example, by J. Sambrook et al. eds., 2000, Molecular Cloning: A Laboratory Manual, 3rd Edition, Cold Spring Harbor Laboratory Press, Cold Spring Harbor; and Ausubel et al. eds., 1995, Current Protocols in Molecular Biology, John Wiley and Sons, N.Y.

In some embodiments, the probe hybridizes to the (amplified) cDNA of the target mRNA-sequence under stringent or moderately stringent hybridization conditions as defined above.

Preferably, probes for use in accordance with the present disclosure have a length of 20 to 35 nucleotides, in particular deoxyribonucleotides. In some embodiments, the probes are designed so as to (1) be specific for the target mRNA-sequence (e.g., HLA-E, HLA-F or HLA-G), (2) not include known polymorphisms (e.g., single nucleotide polymorphisms, SNPs) and/or (3) have a melting temperature Tₘ, which is approximately 5°C to 8°C higher than the melting temperature Tₘ of the corresponding primer(s).

The wording "specific for the target mRNA-sequence", as used in connection with probes for use in accordance with the present invention, is meant to refer to the ability of the probe to hybridize (i.e. anneal) to the (amplified) cDNA of the target mRNA-sequence under appropriate conditions of temperature and solution ionic strength, in particular PCR conditions. The conditions of temperature and solution ionic strength determine the stringency of hybridization. Hybridization requires that the two nucleic acids (i.e. probe and cDNA) contain complementary sequences, although depending on the stringency of the hybridization, mismatches between bases are possible. In some embodiments, "appropriate conditions of temperature and solution ionic strength" refer to a temperature in the range of from 63°C to 70°C and a solution ionic strength commonly used in PCR reaction mixtures. In some embodiments, the sequence of the probe is 80%, preferably 85%, more preferably 90%, even more preferably 95%, 96%, 97%, 98%, 99% or 100% complementary to the corresponding sequence of the (amplified) cDNA of the target mRNA-sequence, as determined by sequence comparison algorithms known in the art.

In some embodiments, the method comprises the use of HLA-A-specific primers comprising or having the sequences of SEQ ID NOs: 1, 2, 3; and/or HLA-B/C-specific comprising or having the sequences of SEQ ID NOs: 4, 5, 6; and/or HLA-G-specific primers comprising or having the sequences of SEQ ID Nos: 7 through 27; and/or HLA-H-specific primers comprising or having the sequences of SEQ ID Nos: 28 through 33.

In some embodiments, the quantitative PCR is fluorescence-based quantitative real-time PCR.

In some embodiments, detection of the probe is based on amplification-mediated probe displacement.

In some embodiments, the probe is a dual-label probe comprising a fluorescence reporter moiety and a fluorescence quencher moiety.

In some embodiments, the kit further comprises a reverse transcriptase and a DNA polymerase.

In some embodiments, the reverse transcriptase and the DNA polymerase are provided in the form of an enzyme-mix which allows a one-step reverse transcription (RT) quantitative PCR (RT-qPCR).

In some embodiments, the kit further comprises at least one pair of reference gene-specific primers and at least one reference gene-specific probe.

In some embodiments, the reference gene is one or more selected from the group consisting of CALM2, B2M, RPL37A, GUSB, HPRT1 and GAPDH. As used herein, CALM2 refers to calmodulin-2, phosphorylase kinase, delta (Ref.Seq. (mRNA): NM_001743), B2M refers to beta-2 microglobulin (Ref.Seq. (mRNA): NM_004048), RPL37A refers to 60S ribosomal protein L37a (Ref.Seq. (mRNA): NM_000998), GUSB refers to beta-glucuronidase (Ref.Seq. (mRNA): NM_000181), HPRT1 refers to hypoxanthine-phosphoribosyl-transferase 1 (Ref.Seq. (mRNA): NM_000194) and GAPDH refers to glycerinaldehyde-3-phosphate-dehydrogenase (Ref. Seq. (mRNA): NM_002046).

In some embodiments, the kit further comprises at least one control RNA sample.

In some embodiments, the primers provide an amplicon size of less than 120 bp.

In some embodiments, the HLA-A-specific primers have a length of 15 to 30 nucleotides and comprise at least 10 contiguous nucleotides of the sequences of SEQ ID NOs: 1, 2 or 3, and/or the HLA-B/C-specific primers have a length of 15 to 30 nucleotides and comprise at least 10 contiguous nucleotides of the sequences of SEQ ID NOs: 4, 5 or 6; and/or the HLA-G-specific primers have a length of 15 to 30 nucleotides and comprise at least 10 contiguous nucleotides of one of the sequences of SEQ ID NOs: 7 through 27; and/or the HLA-H-specific primers have a length of 15 to 30 nucleotides and comprise at least 10 contiguous nucleotides of one of the sequences of SEQ ID NOs: 28 through 33.

In some embodiments, the cancer indication is breast cancer, ovarian, lung cancer, bladder cancer, gastric cancer or colon cancer
In a further aspect, the invention relates to the use of the expression level of RNA transcript of HLA-E and/or the expression level of RNA transcript of HLA-F and/or the expression level of RNA transcript of HLA-G as prognostic biomarker(s) or as predictive biomarker(s) for cancer, in particular as predictive biomarker(s) indicating resistance towards chemotherapy or indicating resistance towards immune therapy.

The term "marker" or "biomarker" refers to a biological molecule, e.g., a nucleic acid, peptide, protein, hormone, etc., whose presence or concentration can be detected and correlated with a known condition, such as a disease state or a combination of these, e.g. by a mathematical algorithm.

The term "prognostic marker", as used herein, refers to a marker that provides information on the likely course of the respective disease (e.g.: bladder cancer) in a treated or untreated patient. In some embodiments, the prognosis comprises one or more of disease-specific survival (DSS), recurrence-free survival (RFS), progression-free survival (PFS) and distant recurrence-free survival. In some embodiments, the prognosis comprises DSS. The term "singular prognostic biomarker", as used herein, means that no additional prognostic marker is used/analyzed for the prognosis.

In some embodiments, the expression level of RNA transcript of HLA-E or the expression level of RNA transcript of HLA-F or the expression level of RNA transcript of HLA-G is used as a singular prognostic biomarker.

In some embodiments,
- an expression level of RNA transcript of HLA-E which is lower than a defined expression threshold of RNA transcript of HLA-G indicates a positive prognosis; and/or
- an expression level of RNA transcript of HLA-F which is lower than a defined expression threshold of RNA transcript of HLA-F indicates a negative prognosis; and/or
- an expression level of RNA transcript of HLA-F which is lower than a defined expression threshold of RNA transcript of HLA-G indicates a positive prognosis.

In some embodiments, the positive prognosis comprises an increased probability of one or more of prolonged disease-specific survival (DSS), recurrence-free survival (RFS), progression-free survival (PFS) and distant recurrence-free survival, preferably DSS.

In some embodiments,
- an expression level of RNA transcript of HLA-E which is higher than a defined expression threshold of RNA transcript of HLA-E indicates a negative prognosis; and/or
- an expression level of RNA transcript of HLA-F which is higher than a defined expression threshold of RNA transcript of HLA-F indicates a positive prognosis; and/or
- an expression level of RNA transcript of HLA-G which is higher than a defined expression threshold of RNA transcript of HLA-F indicates a negative prognosis.

In some embodiments, the negative prognosis comprises a reduced probability of one or more of prolonged disease-specific survival (DSS), recurrence-free survival (RFS), progression-free survival (PFS) and distant recurrence-free survival, preferably DSS.

In another aspect, the present invention relates to the use of a pair of primers as defined herein and/or a probe as defined herein for identifying a molecular subtype of a tumor in a bladder cancer patient, e.g., in a method as defined herein, wherein the pair of primers and/or probe is specific for a gene selected from the group consisting of HLA-E, HLA-F and HLA-G.

In some embodiments, the probe is a dual-label probe comprising a fluorescence reporter moiety and a fluorescence quencher moiety.

In yet another aspect, the present invention relates to the use of a pair of primers as defined herein and/or a probe as defined herein for manufacturing of a kit for identifying a molecular subtype of a tumor in a bladder cancer patient by means of reverse transcription (RT) quantitative PCR (RT-qPCR), wherein the pair of primers and/or probe is specific for a gene selected from the group consisting of HLA-E, HLA-F, HLA-G, HLA-H, or HLA-J.

In some embodiments, the probe is a dual-label probe comprising a fluorescence reporter moiety and a fluorescence quencher moiety.

In one aspect, the invention relates to an *in vitro* method of identifying a molecular subtype of a tumor in a patient having cancer, said method comprising determining the expression level of RNA transcripts and thereafter determining the HLA expression as described above.

The term "molecular subtype of a tumor" (or "molecular subtype of a cancer"), as used herein, refers to subtypes of a tumor/cancer that are characterized by distinct molecular profiles, e.g., gene expression profiles.

In some embodiments, said method comprises the determination of the expression level, in particular the expression level of RNA transcript, of one or more additional non-reference genes.

The term "non-reference gene", as used herein is meant to refer to a gene which has a variable level of expression on the RNA transcript/mRNA level in the system which is being examined, i.e. cancer, and thus can be used, e.g., for the subtyping of tumors/cancers and/or the assessment of cancer progression. In some embodiments, the non-reference gene is selected from tumor markers, e.g., those known from the prior art. Non-reference genes that can be used in accordance with the present invention may be bladder-specific or non-bladder-specific genes.

In some embodiments, said method does not comprise the determination of the expression level, in particular the expression level of RNA transcript, of more than three, two or one additional non-reference gene.

In some embodiments, said method does not comprise the determination of the expression level, in particular the expression level of RNA transcript, of any additional non-reference gene. In other words, no expression level, in particular no expression level of RNA transcript, of a gene other than HLA-E, HLA-F, HLA-G, HLA-H, or HLA-J and, optionally, at least one gene selected from HLA-A, HLA-B, HLA-C and HLA-D, and, optionally, one or more reference genes is determined.

In some embodiments, the expression levels of RNA transcript of a maximum of 7, preferably 6, more preferably 5, even more preferably 4 different non-reference genes are determined.

In some embodiments, said method does not comprise any other diagnostic steps, such as histological grading or determining the lymph nodal status. In some embodiments, said method does not comprise any steps involving immunohistochemistry (IHC).

In some embodiments of the present invention, the tumor is a solid tumor. In some embodiments, the tumor is a bladder or urethral tumor or is derived from a bladder or urethral tumor (e.g., by metastasis). The term "bladder", as used herein, refers to the urinary bladder.

In some embodiments, the sample of the tumor may be a tumor tissue sample isolated from the cancer patient (e.g., a biopsy or resection tissue of the tumor). In a preferred embodiment, the tumor tissue sample is a cryo-section of a tumor tissue sample or is a chemically fixed tumor tissue sample. In a more preferred embodiment, the tumor tissue sample is a formalin-fixed and paraffin-embedded (FFPE) tumor tissue sample. In some embodiments, the sample of the tumor is (total) RNA extracted from the tumor tissue sample. In a particularly preferred embodiment, the sample of the tumor is (total) RNA extracted from a FFPE tumor tissue sample. Those skilled in the art are able to perform RNA extraction procedures. For example, total RNA from a 5 to 10 µm curl of FFPE tumor tissue can be extracted using the High Pure RNA Paraffin Kit (Roche, Basel, Switzerland) or, the XTRAKT RNA Extraction Kit XL (Stratifyer Molecular Pathology, Cologne, Germany) or RNXtract^{®} Extraction Kit (BioNTech Diagnostics GmbH, Mainz, Germany). It is also possible to store the sample material to be used/tested in a freezer and to carry out the method of the present invention at an appropriate point in time after thawing the respective sample material. The sample may be obtained from the cancer patient prior to initiation of a therapeutic treatment, during the therapeutic treatment and/or after the therapeutic treatment, i.e. prior to, during or following the administration of cancer therapy.

In a further aspect, the disclosure relates to a method of stratifying a patient, e.g. of bladder cancer, for tumor treatment, said method comprising, as a first step, identifying a molecular subtype of a tumor in the cancer patient using the *in vitro* method as defined above and, as a second step, selecting a tumor treatment regimen based on the molecular subtype identified by the *in vitro* method.

In some embodiments, said method of stratifying a bladder cancer patient for tumor treatment does not comprise any other diagnostic steps, such as histological grading or determining the lymph nodal status, besides the step of identifying the molecular subtype of the tumor in the cancer patient using the *in vitro* method as defined above. In some embodiments, said method does not comprise any steps involving immunohistochemistry (IHC).

In some embodiments, the molecular subtype is selected from the group consisting of HER2-positive, triple-negative (also referred to as "basal-like"), luminal A and luminal B. The term "basal-like" refers to the fact that such tumors have some similarity in gene expression to that of basal epithelial cells. The term "luminal" derives from the similarity in gene expression between the tumors and the luminal epithelium.

In some embodiments, the expression levels of RNA transcript of HER2, ESR1 and Ki67 are determined, and the molecular subtype is selected from the group comprising, preferably consisting of, HER2+, HER2-/ESR1+, HER2-/ESR1-/Ki67+ and HER2-/ESR1-/Ki67-. In some embodiments, said molecular subtype relates to MIBC. The molecular subtypes may differ markedly in clinical outcome and response to therapy.

In some embodiments,
- the molecular subtype is HER2-positive, and the tumor treatment regimen comprises transurethral resection and/or BCG-instillation and/or chemotherapy and/or anti-HER2 therapy and/or administration of antibodies targeting immune check points and/or cystectomy followed by administration of anti-HER2 therapy and/or chemotherapy;
- the molecular subtype is triple-negative, and the tumor treatment regimen comprises transurethral resection and/or BCG-instillation and/or chemotherapy, in particular neoadjuvant chemotherapy, and/or administration of antibodies targeting immune check points and/or cystectomy;
- the molecular subtype is luminal A, and the tumor treatment regimen comprises transurethral resection and/or BCG-instillation and/or cystectomy and/or chemotherapy, in particular adjuvant chemotherapy, and/or (adjuvant) endocrine therapy; and/or
- the molecular subtype is luminal B, and the tumor treatment regimen comprises transurethral resection and/or BCG-instillation and/or endocrine therapy and/or chemotherapy, in particular adjuvant chemotherapy or chemotherapy in the perioperative situation, and/or cystectomy.

In some embodiments,
- the molecular subtype is luminal A, the bladder cancer is NMIBC, and the tumor treatment regimen comprises transurethral resection (TUR) and/or Bacillus Calmette-Guerin (BCG)-instillation, preferably TUR and BCG-instillation;
- the molecular subtype is luminal B, the bladder cancer is NMIBC, and the tumor treatment regimen comprises adjuvant chemotherapy with or without adjuvant endocrine therapy;
- the molecular subtype is HER2-positive, the bladder cancer is NMIBC, and the tumor treatment regimen comprises (neo)adjuvant chemotherapy with or without (neo)adjuvant anti-HER2 therapy;
- the molecular subtype is triple-negative, the bladder cancer is NMIBC, and the tumor treatment regimen comprises neoadjuvant chemotherapy;
- the molecular subtype is luminal A, the bladder cancer is MIBC, and the tumor treatment regimen comprises (i) cystectomy and (ii) adjuvant chemotherapy and/or adjuvant endocrine therapy, preferably adjuvant chemotherapy or adjuvant endocrine therapy;
- the molecular subtype is luminal B, the bladder cancer is MIBC, and the tumor treatment regimen comprises (i) cystectomy and (ii) adjuvant chemotherapy and/or adjuvant endocrine therapy, preferably adjuvant chemotherapy and adjuvant endocrine therapy;
- the molecular subtype is HER2-positive, the bladder cancer is MIBC, and the tumor treatment regimen comprises (i) cystectomy and (ii) adjuvant chemotherapy and/or adjuvant anti-HER2 therapy, preferably adjuvant chemotherapy and adjuvant anti-HER2 therapy; and/or
- the molecular subtype is triple-negative, the bladder cancer is MIBC, and the tumor treatment regimen comprises neoadjuvant chemotherapy with or without subsequent cystectomy.

In some embodiments, the molecular subtype is HER2-positive, the cancer is preferably NMIBC, and the tumor treatment regimen comprises anti-HER2 therapy in combination with endocrine therapy, hormonal therapy and/or chemotherapy, all of which can be in the form of adjuvant or neoadjuvant therapy.

In another embodiment, the molecular subtype is HER2-positive, the cancer is preferably NMIBC, and the tumor treatment regimen comprises instillation therapy, e.g., BCG-instillation.

In some embodiments, the molecular subtype is luminal B, the bladder cancer is preferably NMIBC, and the tumor treatment regimen comprises instillation therapy (e.g., BCG-instillation) and/or neoadjuvant/adjuvant chemotherapy, preferably in combination with endocrine therapy, e.g., with tamoxifen (Nolvadex^{®}), fulvestrant (Faslodex^{®}) or aromatase inhibitors.

The meaning of the term "anti-HER2 therapy" is known to a person skilled in the art. In some embodiments, anti-HER2 therapy comprises the administration of anti-HER2 antibodies, in particular monoclonal anti-HER2 antibodies. Monoclonal anti-HER2 antibodies include trastuzumab (Herceptin^{®}) and pertuzumab (Perjeta^{®}), which may be administered alone or in combination. Trastuzumab is effective only in cancers where HER2 is over-expressed. Other monoclonal antibodies, such as ertumaxomab (Rexomun^{®}), are presently undergoing clinical trials. The anti-HER2 antibodies can further be modified to comprise a therapeutic moiety/agent, such as a cytotoxic agent, a drug (e.g., an immunosuppressant), a chemotherapeutic agent or a radionuclide, or a radioisotope. Thus, if the tumor treatment regimen comprises (a combination of) anti-HER2 therapy and chemotherapy, an anti-HER2 antibody conjugated to a chemotherapeutic agent may be used. A cytotoxin or cytotoxic agent includes any agent that is detrimental to and, in particular, kills cells. Examples include mertansine or emtansine (DM1), taxol, cytochalasin B, gramicidin D, ethidium bromide, emetine, mitomycin, etoposide, tenoposide, vincristine, vinblastine, colchicin, doxorubicin, daunorubicin, dihydroxy anthracin, dione, mitoxantrone, mithramycin, actinomycin D, amanitin, 1-dehydrotestosterone, glucocorticoids, procaine, tetracaine, lidocaine, propranolol, and puromycin and analogs or homologs thereof. In some embodiments, the antibody conjugate is trastuzumab (T)-DM1, e.g., trastuzumab emtansine. Other suitable therapeutic agents for forming antibody conjugates include, but are not limited to, antimetabolites (e.g., methotrexate, 6-mercaptopurine, 6-thioguanine, cytarabine, fludarabin, 5-fluorouracil decarbazine), alkylating agents (e.g., mechlorethamine, thioepachlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In some embodiments, the therapeutic agent is GM-CSF. In another preferred embodiment, the therapeutic agent is doxorubicin, cisplatin, bleomycin, sulfate, carmustine, chlorambucil, cyclophosphamide or ricin A. Further therapeutic moieties include therapeutic moieties acting on mRNA and/or protein synthesis. Several inhibitors of transcription are known. For instance, actinomycin D, which is both a transcriptional inhibitor and a DNA damage agent, intercalates within the DNA and thus inhibits the initiation stage of transcription. Flavopiridol targets the elongation stage of transcription. α-Arnanitin binds directly to RNA polymerase II, which leads to the inhibition of both initiation and elongation stages. Anti-HER2 antibodies also can be conjugated to a radioisotope, e.g., iodine-131, yttrium-90 or indium-111, to generate cytotoxic radiopharmaceuticals. An alternative to the administration of anti-HER2 antibodies is the administration of small compounds targeting HER2, such as lapatinib (Tykerb^{®} or Tyverb^{®}), afatinib or neratinib. Anti-HER2 therapy may also be supplemented with endocrine therapy (also referred to as anti-hormonal treatment), hormonal therapy, e.g., with progestin, and/or chemotherapy.

Chemotherapy comprises the administration of chemotherapeutic agents, such as cytostatic compounds or cytotoxic compounds. Traditional chemotherapeutic agents act by killing cells that divide rapidly, one of the main properties of most cancer cells. The term "chemotherapeutic agent" includes taxanes, platinum compounds, nucleoside analogs, camptothecin analogs, anthracyclines and anthracycline analogs, etoposide, bleomycin, vinorelbine, cyclophosphamide, antimetabolites, anti-mitotics, and alkylating agents, including the agents disclosed above in connection with antibody conjugates, and combinations thereof. In some embodiments, the chemotherapy is platinum-based, i.e. comprises the administration of platinum-based compounds, e.g., cisplatin. A reference to a chemotherapeutic agent may include any prodrug such as ester, salt or derivative such as a conjugate of said agent. Examples are conjugates of said agent with a carrier substance, e.g., protein-bound paclitaxel such as albumin-bound paclitaxel. Preferably, salts of said agent are pharmaceutically acceptable. Chemotherapeutic agents are often given in combinations, usually for 3-6 months. One of the most common treatments is cyclophosphamide plus doxorubicin (adriamycin; belonging to the group of anthracyclines and anthracycline analogs), known as AC. Sometimes, a taxane drug, such as docetaxel, is added, and the regime is then known as CAT; taxane attacks the microtubules in cancer cells. Another common treatment, which produces equivalent results, is cyclophosphamide, methotrexate, which is an antimetabolite, and fluorouracil, which is a nucleoside analog (CMF). Another standard chemotherapeutic treatment comprises fluorouracil, epirubicin and cyclophosphamide (FEC), which may be supplemented with a taxane, such as docetaxel, or with vinorelbine.

In some embodiments, the molecular subtype is luminal B, and the tumor treatment regimen comprises administration of chemotherapeutic agents. In some embodiments, the molecular subtype is luminal B, and the tumor treatment regimen comprises administration of a taxane, preferably docetaxel. In some embodiments, the taxane is administered in combination with platinum-based chemotherapy.

Endocrine therapy (also referred to as anti-hormonal treatment) targets cancers that require estrogen to continue growing by administration of drugs that either block/down-regulate estrogen and/or progesterone receptors, e.g., tamoxifen (Nolvadex^{®}) or fulvestrant (Faslodex^{®}), or alternatively block the production of estrogen with an aromatase inhibitor, e.g., anastrozole (Arimidex^{®}) or letrozole (Femara^{®}). Aromatase inhibitors, however, are only suitable for postmenopausal patients. This is because the active aromatase in postmenopausal women is different from the prevalent form in premenopausal women, and therefore these agents are ineffective in inhibiting the predominant aromatase of premenopausal women.

In another aspect, the disclosure may be used for treatment of cancer, the method comprising, as a first step, stratifying a bladder cancer patient for tumor treatment using the method as defined above and, as a second step, providing the selected tumor treatment regimen to the bladder cancer patient. The tumor treatment regimen is selected based on the molecular subtype identified by the *in vitro* method as defined above.

The first step and the second step of said method may be performed separately from each other, in terms of time and/or location. The first step may, for example, result in the issuance of treatment guidelines, which are used for performing the second step at a different time and/or location. The first step may also be immediately followed by the second step.

In some embodiments, said method comprises using quantitative results obtained by the *in vitro* method as defined above for direct decision-making in favor of or against adjuvant/neoadjuvant chemotherapy.

In another aspect, the present disclosure may be used of the treatment of cancer, wherein the bladder cancer is characterized by a molecular subtype as defined herein, and wherein the method comprises providing a tumor treatment regimen that is selected based on the molecular subtype.

In another aspect, the disclosure relates to a method of producing a therapeutic agent, the method comprising determining an individual HLA pattern using a method as described above and producing soluble HLA domains or antibodies based on the determined individual HLA pattern. It also relates to a therapeutic agent produced according to above for use in the treatment of cancer.

### BRIEF DESCRIPTION OF THE FIGURES

**Figure 1** depicts sequence alignment of HLA-A1, -A2, -B, -E, -F1, -F2, -F3, -J, -G and HLA-H at the potential translation initiation site, according to Example 2.
**Figure 2** depicts sequence alignment of HLA-A1, -A2, -B, -E, -F1, -F2, -F3, -J, -G and HLA-H at the exon 4 to exon 5 junction.
**Figure 3** depicts sequence alignment of HLA-A1, -A2, -B, -E, -F1, -F2, -F3, -J, -G and HLA-H at the exon 8.
**Figure 4** depicts data distribution of luminal and basal subtype markers, check point target genes and FGFR1 to 4 gene expression as determined by RT-qPCR from FFPE tissues from muscle invasive bladder cancer patients.
**Figure 5** depicts intergene spearman correlation of luminal and basal subtype markers, check point target genes and FGFR1 to 4 gene mRNA expression as determined by RT-qPCR from XX tissues from muscle invasive bladder cancer patients.
**Figure 6** depicts intergene spearman correlation of HLA gene mRNA expression as determined by RT-qPCR from FFPE tissues from muscle invasive bladder cancer patients.
**Figure 7** depicts a Kaplan Meier Plot displaying disease specific survival (DSS) probability from FFPE tissues of muscle invasive bladder cancer patients based on stratification by combining HLA-A exon 8, HLA-G exon 8 and HLA-G exon 5 mRNA expression.
**Figure 8** depicts a Kaplan Meier Plot displaying disease specific survival (DSS) probability from FFPE tissues of muscle invasive bladder cancer patients based on stratification by intergenic combination of HLA-A exon 8 and HLA-G exon 8 mRNA expression
**Figure 9** depicts Kaplan Meier Plot displaying disease specific survival (DSS) probability from FFPE tissues of muscle invasive bladder cancer patients based on stratification by intragenic combination of HLA-G exon 8 and exon 5 mRNA expression.
**Figure 10** depicts a Kaplan Meier Plot displaying disease specific survival (DSS) probability from FFPE tissues of muscle invasive bladder cancer patients (n=61) based on stratification by single gene determination of only HLA-G exon 8 and exon 5 mRNA expression.
**Figure 11** depicts a data distribution of relative mRNA expression (40-DCT) of HLA-F isoforms and anti-sense HLA-F expression as determined by RT-qPCR.
**Figure 12** depicts a data distribution of relative mRNA expression (40-DCT) of ESR1, HLA-F3 and HLA-F AS1 expression as determined by RT-qPCR.
**Figure 13** depicts a partition test for HLA-F3 mRNA expression in pre-treatment biopsy samples of neoadjuvantly treated ovarian cancer patients determined by RT-qPCR to predict progression free survival.
**Figure 14** depicts a Kaplan Meier Plot displaying progression free survival (PFS) probability based on stratification by single gene determination of only HLA-F3 as quantified by RT-qPCR assay from fresh tissues of advanced ovarian cancer patients (n=27).
**Figure 15** depicts a Kaplan Meier Plot displaying overall survival (OS) probability based on stratification by single gene determination of only HLA-F3 as quantified by RT-qPCR assay from fresh tissues of advanced ovarian cancer patients (n=27).
**Figure 16** depicts a multivariate analysis for OS using cox proportional hazards models including Grade, FIGO stage, Primary site and HLA-F3 mRNA expression.
**Figure 17** depicts a Partition test for ESR1 and HLA-F3 mRNA expression in pre-treatment biopsy samples of neoadjuvantly treated ovarian cancer patients determined by RT-qPCR to predict progression free survival.
**Figure 18** depicts a Kaplan Meier Plot displaying progression free survival (PFS) probability based on stratification ESR1 and HLA-F3 mRNA expression as quantified by RT-qPCR assay from fresh tissues of advanced ovarian cancer patients (n=27).
**Figure 19** depicts a Kaplan Meier Plot displaying overall survival (OS) probability based on stratification ESR1 and HLA-F3 mRNA expression as quantified by RT-qPCR assay from fresh tissues of advanced ovarian cancer patients (n=27).
**Figure 20** depicts a multivariate analysis for PFS using cox proportional hazards models including Grade, FIGO stage, Primary site and the combination of ESR1 and HLA-F3 mRNA expression.
**Figure 21** depicts a multivariate analysis for OS using cox proportional hazards models including Grade, FIGO stage, Primary site and the combination of ESR1 and HLA-F3 mRNA expression.
**Figure 22** depicts a Kaplan Meier Plot displaying overall survival (OS) probability based on stratification HLA-F3 and HLA-F AS1 mRNA expression as quantified by RT-qPCR assay from fresh tissues of advanced ovarian cancer patients (n=27).
**Figure 23** depicts a Kaplan Meier Plot displaying overall survival (OS) probability based on stratification HLA-F3 and HLA-F AS1 mRNA expression as quantified by RT-qPCR assay from fresh tissues of advanced ovarian cancer patients (n=27).
**Figure 24** depicts a multivariate analysis for PFS using cox proportional hazards models including Grade, FIGO stage, Primary site and the combination of HLA-F3 and HLA-F AS1.
**Figure 25** depicts a multivariate analysis for OS using cox proportional hazards models including Grade, FIGO stage, Primary site and the combination of HLA-F3 and HLA-F AS1.
**Figure 26** depicts a consort diagram of advanced or metastatic urothelial cancer cohort.
**Figure 27** depicts a Kaplan Meier Plot displaying disease specific survival (DSS) probability from muscle invasive bladder cancer patients having locally advanced or metastatic UBC (n=55) based on stratification by HLA-F1/F2 expression as quantified by RT-qPCR assay.
**Figure 28** depicts a Kaplan Meier Plot displaying disease specific survival (DSS) probability from muscle invasive bladder cancer patients having locally advanced or metastatic UBC (n=55) based on stratification by HLA-F 1/F2 and HLA-G Exon 8 mRNA expression as quantified by RT-qPCR assay.
**Figure 29** depicts a Kaplan Meier Plot displaying disease specific survival (DSS) probability from muscle invasive bladder cancer patients having locally advanced or metastatic UBC (n=55) based on stratification by HLA-F1/F2 and HLA-B/C Exon 8 mRNA expression as quantified by RT-qPCR assay.
**Figure 30** depicts a Kaplan Meier Plot displaying disease specific survival (DSS) probability from muscle invasive bladder cancer patients having locally advanced or metastatic UBC (n=55) based on stratification by HLA-B/C Exon 8 mRNA expression as quantified by RT-qPCR assay.

### DETAILED DESCRIPTION OF THE INVENTION

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

In the following, certain elements of the present invention will be described. These elements may be listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise. For example, as will be clear to a person skilled in the art, the specific embodiments disclosed herein relating to the expression levels of RNA transcript of particular genes (being higher or lower than defined expression thresholds of RNA transcript of the particular genes) and the molecular subtypes based thereon can be combined so as to allow for the identification of a molecular subtype of a given tumor.

Before proceeding with the description of embodiments and examples, some general remarks on terminology follow: Preferably, the terms used herein are defined as described in "A multilingual glossary of biotechnological terms (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 3rd Edition, J. Sambrook et al. eds., Cold Spring Harbor Laboratory Press, Cold Spring Harbor 2000).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

The term "classification of a sample" of a patient, as used herein, relates to the association of said sample with at least one of at least two categories. These categories may be for example "high risk" and "low risk", high, intermediate and low risk, wherein risk is the probability of a certain event occurring in a certain time period, e.g. occurrence of metastasis, disease free survival, and the like. It can further mean a category of favourable or unfavourable clinical outcome of disease, responsiveness or non-responsiveness to a given treatment or the like. Classification may be performed by use of an algorithm, in particular a discriminant function. A simple example of an algorithm is classification according to a first quantitative parameter, e.g. expression level of a gene of interest, being above or below a certain threshold value. Classification of a sample of a patient may be used to predict an outcome of disease. Instead of using the expression level of a single gene of interest, a combined score of several genes of interest may be used. Further, additional data may be used in combination with the first quantitative parameter. Such additional data may be clinical data from the patient, such as sex, age, weight of the patient, tumor grading or stage etc.

The term "metastasis" is meant to refer to the spread of cancer cells from their original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential.

A "discriminant function" is a function of a set of variables used to classify an object or event. A discriminant function thus allows classification of a patient, sample or event into a category or a plurality of categories according to data or parameters available from said patient, sample or event. Such classification is a standard instrument of statistical analysis well known to the skilled person. E.g. a patient may be classified as "high risk" or "low risk", "high probability of metastasis" or "low probability of metastasis", "in need of treatment" or "not in need of treatment" according to data obtained from said patient, sample or event. Classification is not limited to "high vs. low", but may be performed into a plurality of categories, grading or the like. Examples for discriminant functions which allow a classification include, but are not limited to discriminant functions defined by support vector machines (SVM), k-nearest neighbors (kNN), (naive) Bayes models, or piecewise defined functions such as, for example, in subgroup discovery, in decision trees, in logical analysis of data (LAD) an the like.

The term "prediction" as used herein relates to the likelihood that a patient will respond either favourably or unfavourably to a given therapy. Especially, the term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assessment of the malignancy of a tumor, or to the expected survival rate (DFS, disease free survival) of a patient, if the tumor remains untreated.

The term "response marker" relates to a marker which can be used to predict the clinical response of a patient towards a given treatment. Response includes direct observation of tumor shrinkage upon neoadjuvant or palliative treatment as evident by e.g. CT-Scans and/or serum biomarkers as well as effects on Disease Free Survival (DFS), Overall Survival (OAS), Metastasis Specific Survival (MSS), Disease Specific Survival and related assessments.

The term "clinical response" of a patient, as used herein, relates to the effectiveness of a certain therapy in a patient, meaning an improvement in any measure of patient status, including those measures ordinarily used in the art, such as overall survival, progression free survival, recurrence-free survival, and distant recurrence-free survival. Recurrence-free survival (RFS) refers to the time (in years) from surgery to the first local, regional, or distant recurrence. Distant recurrence-free survival (DFRS) refers to the time (in years) from surgery and/or initial diagnosis to the first anatomically distant recurrence. The calculation of these measures in practice may vary from study to study depending on the definition of events to be either censored or not considered.

The term "neoplastic disease" refers to a cancerous tissue this includes carcinomas, e.g., carcinoma in situ, invasive carcinoma, metastatic carcinoma, and pre-malignant conditions, neomorphic changes independent of their histological origin. The term "adenocarcinoma" refers to a malignant tumor originating in glandular tissue.

The terms "cancer" and "cancerous" refer to or describe the physiological condition in mammals that is typically characterized by unregulated cell growth. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies and transformations specially associated with gynecologic cancer are included. The terms "neoplastic disease" or "cancer" are not limited to any tissue or cell type they also include primary, secondary or metastatic lesions of cancer patients, and also comprise lymph nodes affected by cancer cells or minimal residual disease cells either locally deposited or freely floating throughout the patient's body.

As used herein, the term "cancer" includes a disease characterized by aberrantly regulated cellular growth, proliferation, differentiation, adhesion, and/or migration. The term cancer as used herein also comprises cancer metastases. The terms "tumor" and "cancer" may be used interchangeably herein.

The term "tumor" as used herein, refers to all neoplastic cell growth and proliferation, whether malignant or benign, and all pre-cancerous and cancerous cells and tissues.

As used herein, the term "lung cancers" refers to cancer or malignancies which are diagnosed in the lung and is meant to include all cancers, neoplastic growths and cancerous transformations of lung tissue. Examples of lung cancers include, but are not limited to: small cell lung carcinoma (SCLC), and non-small cell lung carcinoma (NSCLC), in particular squamous cell lung carcinoma, adenocarcinoma, bronchioloalveolar carcinoma, large cell lung carcinoma, and others, such as pleuropulmonary blastoma and carcinoid tumors.

The term "neoplastic cells" refer to abnormal cells that grow by increased cellular proliferation, altered cell division symmetry or decreased cell death mechanisms more rapidly than normal. As such, neoplastic cells of the invention may be cells of a benign neoplasm or may be cells of a malignant neoplasm.

Furthermore, the term "characterizing the state" of a neoplastic disease or cancer is related to, but not limited to, measurements and assessment of one or more of the following conditions: Type of tumor, histomorphological appearance, dependence on external signal (e.g. hormones, growth factors), invasiveness, motility, state by TNM Classification of Malignant Tumors (TNM), a cancer staging system developed and maintained by the International Union Against Cancer, or similar, aggressivity, malignancy, metastatic potential, and responsiveness to a given therapy.

The term "therapy modality", "therapy mode", "regimen" or "chemo regimen" as well as "therapy regimen" refers to a timely sequential or simultaneous administration of anti-tumor, and/or anti vascular, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, timeframe of application and frequency of administration within a defined therapy window. Currently various combinations of various drugs and/or physical methods, and various schedules are under investigation.

The term "endocrine treatment" refers to various treatment modalities known as hormonal therapy or anti hormonal therapy that produce the desired therapeutic effect by means of change of hormone/hormones level. The treatment may include administration of hormones or hormone analogs, synthetic hormones or other drugs to the patient, or decreasing the level of hormones in the body by using hormone antagonists, hormone receptor antagonists or hormone ablation therapy either by surgical resection of ovaries or by chemical suppression of hormone synthesis. Endocrine therapy shall be taken to include hormonal therapies such as selective estrogen reuptake inhibitors, selective estrogen receptor downregulators, aromatase inhibitors and ovarian ablation. Said endocrine treatment may include administration of hormones or hormone analogs, synthetic hormones or other drugs to the patient, e.g. tamoxifen, raloxifen and/or gosereline (tradename Zoladex^{®}). In a preferred embodiment the said endocrine treatment comprises the administration of tamoxifen or of tamoxifen and gosereline. Further, said endocrine treatment may comprise the administration of an anti estrogen drug selected from the group comprising anastrozole, letrozole, exemestane, fulvestrant, toremifene and megasterol acetate. Said endocrine treatment may also comprise the administration of estrogen, progestin and/or gestagen.

The term "determining the expression level of a gene on a non protein basis" relates to methods which are not focussed on the secondary gene translation products, i.e. proteins, but on other levels of the gene expression, based on RNA and DNA analysis. In some embodiments of this invention the analysis uses mRNA including its precursor forms. An exemplary determinable property is the amount of the HLA mRNA, i.e. HLA-A, HLA-B, HLA-C, HLA-D, HLA-E, HLA-F, HLA-G, HLA-H, HLA-J or parts thereof.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, fat cells, endothelial cells of blood vessels as well as cells of the immune system, e.g. lymphocytes, macrophages, killer cells.

The term "pattern of RNA expression" refers to a determined level of RNA expression compared either to a reference RNA or to a computed average expression value. A pattern is not limited to the comparison of two RNAs but is more related to multiple comparisons of RNAs to reference RNAs or samples. A certain "pattern of expression levels" may also result and be determined by comparison and measurement of several RNAs and display the relative abundance of these transcripts to each other.

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

The terms "modulated" or "modulation" or "regulated" or "regulation" and "differentially regulated" as used herein refer to both upregulation, i.e., activation or stimulation, e.g., by agonizing or potentiating, and down regulation, i.e., inhibition or suppression, e.g., by antagonizing, decreasing or inhibiting.

The phrase "response", "therapeutic success", or "response to therapy" refers in the neoadjuvant, adjuvant and palliative chemotherapeutic setting to the observation of a defined tumor free or recurrence free or progression free survival time (e.g. 2 years, 4 years, 5 years, 10 years). This time period of disease free -, recurrence free - or progression free survival may vary among the different tumor entities but is sufficiently longer than the average time period in which most of the recurrences appear. In a neoadjuvant and palliative therapy modality, response may additionally be monitored by measurement of tumor shrinkage and regression due to apoptosis and necrosis of the tumor mass or reduced blood supply due to altered angiogenic events.

The term "recurrence" or " recurrent disease" includes distant metastasis that can appear even many years after the initial diagnosis and therapy of a tumor, or local events such as infiltration of tumor cells into regional lymph nodes, or occurrence of tumor cells at the same site and organ of origin within an appropriate time.

"Prediction of recurrence" or "prediction of therapeutic success" does refer to the methods described in this invention, wherein a tumor specimen is analyzed for e.g. its gene expression, genomic status and/or histopathological parameters (such as TNM and Grade) and/or imaging data and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prolonged disease free post therapy time.

The term "marker gene" as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and gynecological cancer in particular. A marker gene may also have the characteristics of a target gene.

"Target gene", as used herein, refers to a differentially expressed gene involved in cancer, e.g. lung cancer, in a manner in which modulation of the level of the target gene expression or of the target gene product activity may act to ameliorate symptoms of malignant neoplasia. A target gene may also have the characteristics of a marker gene.

The term "receptor", as used herein, relates to a protein on the cell membrane or within the cytoplasm or cell nucleus that binds to a specific molecule (a ligand), such as a neurotransmitter, hormone, or other substance, especially a hormone as estrogen, and initiates the cellular response. Ligand-induced changes in the behavior of receptor proteins result in physiological changes that constitute the biological actions of the ligands.

The term "signalling pathway" is related to any intra- or intercellular process by which cells converts one kind of signal or stimulus into another, most often involving ordered sequences of biochemical reactions out- and inside the cell, that are carried out by enzymes and linked through hormones and growth factors (intercellular), as well as second messengers (intracellular), the latter resulting in what is thought of as a "second messenger pathway". In many signalling pathways, the number of proteins and other molecules participating in these events increases as the process emanates from the initial stimulus, resulting in a "signal cascade" and often results in a relatively small stimulus eliciting a large response.

The term "small molecule", as used herein, is meant to refer to a compound which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

When used in reference to a single-stranded nucleic acid sequence, the term "substantially homologous" refers to any probe that can hybridize (i.e., it is the complement of) the single-stranded nucleic acid sequence under conditions of low stringency as described above.

As used herein, the term "hybridization" is used in reference to the pairing of complementary nucleic acids.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described below. When it comes to the determination of expression levels, hybridization based methods may for example be used to determine the amount of mRNA for a given gene.

By "array" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents an independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides are preferably single-stranded DNA probe oligonucleotides. Moreover, in context of applicable detection methodologies, the term "oligonucleotide" also refers to nucleotide analogues such as PNAs and morpholinos.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). The term "PCR based method" comprises both end-point PCR applications as well as kinetic/real time PCR techniques applying special fluorophors or intercalating dyes which emit fluorescent signals as a function of amplified target and allow monitoring and quantification of the target. Quantification methods could be either absolute by external standard curves or relative to a comparative internal standard.

The term "method based on the electrochemical detection of molecules" relates to methods which make use of an electrode system to which molecules, particularly biomolecules like proteins, nucleic acids, antigens, antibodies and the like, bind under creation of a detectable signal. Such methods are for example disclosed in WO 02/42759, WO 02/41992 and WO 02/097413

. These detectors comprise a substrate with a planar surface which is formed, for example, by the crystallographic surface of a silicon chip, and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules. The probe molecules are for example immobilized by a Thiol-Gold-binding. For this purpose, the probe is modified at its 5'- or 3'-end with a thiol group which binds to the electrode comprising a gold surface. These target nucleic acid molecules may carry, for example, an enzyme label, like horseradish peroxidase (HRP) or alkaline phosphatase. After the target molecules have bound to the probes, a substrate is then added (e.g., α-naphthyl phosphate or 3,3'5,5'-tetramethylbenzidine which is converted by said enzyme, particularly in a redox-reaction. The product of said reaction, or a current generated in said reaction due to an exchange of electrons, can then be detected with help of the electrical detector in a site specific manner.

The term "nucleic acid molecule" is intended to indicate any single- or double stranded nucleic acid and/or analogous molecules comprising DNA, cDNA and/or genomic DNA, RNA, preferably mRNA, peptide nucleic acid (PNA), locked nucleic acid (LNA) and/or Morpholino.

The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5° C. lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30° C. for short probes (e.g. 10 to 50 nucleotides) and at least about 60° C. for longer probes. Stringent conditions may also be achieved with the addition of destabilizing agents, such as formamide and the like.

The term "fragment of the nucleic acid molecule" is intended to indicate a nucleic acid comprising a subset of a nucleic acid molecule according to one of the claimed sequences. The same is applicable to the term "fraction of the nucleic acid molecule".

The term "variant of the nucleic acid molecule" refers herein to a nucleic acid molecule which is substantially similar in structure and biological activity to a nucleic acid molecule according to one of the claimed sequences.

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same binding properties as the latter.

The term "derivative" as used herein, refers to a nucleic acid molecule that has similar binding characteristics to a target nucleic acid sequence as a nucleic acid molecule according to one of the claimed sequences
The term "hybridizing counterparts" as used herein, refers to a nucleic acid molecule that is capable of hybridizing to a nucleic acid molecules under stringent conditions.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

The term "clinical outcome" is defined as the clinical result of a disease, in particular following a treatment, e.g., reduction or amelioration of symptoms. In some embodiments, poor clinical outcome comprises a relative reduction in or more of disease-specific survival (DSS), recurrence-free survival (RFS), progression-free survival (PFS) and distant recurrence-free survival. The term "recurrence" with respect to cancer includes re-occurrence of tumor cells at the same site and organ of the origin disease, metastasis that can appear even many years after the initial diagnosis and therapy of cancer, or local events such as infiltration of tumor cells into regional lymph nodes. "Distant recurrence" refers to a scenario, where the cancer cells have spread (metastasized) to a distant part (i.e., another organ) of the body beyond the regional lymph nodes. Recurrence-free survival is generally defined as the time from randomization to the first of recurrence, relapse, second cancer, or death. Progression-free survival is the time that passes from a certain date (generally the first day of treatment, or the day in which a patient is enrolled in a clinical trial) and the date on which disease "progresses" or the date on which the patient dies, from any cause. The terms "DSS" and "CSS" (for "cancer-specific survival") may be used interchangeably herein.

The term "(therapeutic) treatment", in particular in connection with the treatment of cancer, as used herein, relates to any treatment which improves the health status and/or prolongs (increases) the lifespan of a patient. Said treatment may eliminate cancer, reduce the size or the number of tumors in a patient, arrest or slow the development of cancer in a patient, inhibit or slow the development of new cancer in a patient, decrease the frequency or severity of symptoms in a patient, and/or decrease recurrences in a patient who currently has or who previously has had cancer. In some embodiments, the terms "treatment" and "therapeutic treatment" are meant to refer to one or more of surgical removal of the primary tumor, chemotherapy, anti-hormonal therapy, radiation therapy and immunotherapy/targeted therapy.

Adjuvant therapy is a treatment that is given in addition to the primary, main or initial treatment. The surgeries and complex treatment regimens used in cancer therapy have led the term to be used mainly to describe adjuvant cancer treatments. An example of adjuvant therapy is the additional treatment (e.g., chemotherapy) usually given after surgery (post-surgically), where all detectable disease has been removed, but where there remains a statistical risk of relapse due to occult disease. Neoadjuvant therapy is treatment given before the primary, main or initial treatment (e.g., pre-surgical chemotherapy).

The term "defined expression threshold of RNA transcript", as used herein, may refer to the mean cut-off value (in short: cut-off) calculated from a number of samples, said number of samples being obtained from a number of subjects, in particular, subjects having cancer. To obtain the threshold, the number of subjects may include subjects having tumors of different molecular subtypes, e.g., subjects having HER2-positive tumors and/or subjects having triple-negative tumors and/or subjects having luminal A tumors and/or subjects having luminal B tumors. The threshold may represent an amount or concentration of the RNA transcript. In some embodiments, the threshold is given as CT (cycle threshold; also referred to as quantification cycle, Cq) value (see below). In some embodiments, the (relative) expression level and expression threshold are expressed as 40-ΔCT or 40-ΔΔCT values (see below).

The term "subject", as used herein, relates to any organism such as vertebrate, particularly any mammal, including both a human and another mammal, e.g. an animal such as a rodent, a rabbit, or a monkey. The rodent may be a mouse, rat, hamster, guinea pig, or chinchilla. Preferably, the subject is a human. In some embodiments, a subject is a subject with or suspected of having a disease, in particular cancer, also designated "patient" herein. For the determination of the mean cut-off value, at least two subjects, preferably at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 subjects, are tested.

Returning to the description of preferred embodiments In some embodiments, the cut-off/threshold is defined based on one or more previous clinical studies. Moreover, additional clinical studies may be conducted for the establishment and validation of the cut-off/threshold. The cut-off/threshold may be determined/defined by techniques known in the art. Various clinical studies have already been conducted with the gene markers used in accordance with the present invention. A concordance study in a training-testing setting may be sufficient for the definition and validation of a clinical cut-off/threshold for dichotomization of quantitative results in "expression-positive" or "expression-negative".

In some embodiments, the cut-off/threshold is determined/defined on the basis of clinicopathological parameters, such as IHC-ISH, and/or the data for overall survival (OS), disease-specific survival (DSS), and progression-free survival (PFS), in training cohorts by partitioning tests (e.g., SAS Software JMP^{®} 9.0.0).

In some embodiments, the expression level of RNA transcript is determined by reverse transcription (RT) quantitative PCR (RT-qPCR). As RNA cannot be directly amplified in PCR, it must be reverse transcribed into cDNA using the enzyme reverse transcriptase. For this purpose, a one-step RT-qPCR can be utilized, which combines the reactions of reverse transcription with DNA amplification by PCR in the same reaction. In one-step RT-qPCR, the RNA template is mixed in a reaction mix containing reverse transcriptase, DNA polymerase, primers and probes, dNTPs, salts and detergents. In a first PCR step, the target RNA is reverse transcribed by reverse transcriptase using the target-specific reverse primers. Afterwards, the cDNA is amplified using the primers/probes and DNA polymerase.

For instance, fluorescence-based quantitative real-time PCR may be used. The fluorescence-based quantitative real-time PCR comprises the use of a fluorescently labeled probe. Preferably, the fluorescently labeled probe consists of an oligonucleotide labeled with both a fluorescent reporter dye and a quencher dye (= dual-label probe). Suitable fluorescent reporter and quencher dyes/moieties are known to a person skilled in the art and include, but are not limited to the reporter dyes/moieties 6-FAM^{™}, JOE^{™}, Cy5^{®}, Cy3^{®} and the quencher dyes/moieties dabcyl, TAMRA^{™}, BHQ^{™}-1, -2 or -3. Amplification of the probe-specific product causes cleavage of the probe (= amplification-mediated probe displacement), thereby generating an increase in reporter fluorescence. The increase of fluorescence in the reaction is directly proportional to the increase of target amplificates. By using the LightCycler 480 II system (Roche) or the Versant kPCR system (Siemens) or the Mx3005P system (Agilent Technologies) or equivalent real-time instruments for detection of fluorescence originating from the probe, one can measure the increase in fluorescence in real-time. Analysis output is a CT value of each target. The CT (cycle threshold; also referred to as quantification cycle, Cq) value is determined by the number of PCR amplification cycles, after which the fluorescence signal of the probe exceeds a certain background signal, wherein the CT value is a measure for the amount of target molecules in the sample before the PCR amplification. Preferably, CT-values are further analyzed with appropriate software (e.g., Microsoft Excel^{™}) or statistical software packages (e.g., SAS JMP^{®} 9.0.0, GraphPad Prism4, Genedata Expressionist^{™}). The CT value can either be converted to an absolute target molecule amount (e.g., ng/µl or molecules/µl) based on the CT results of a standard curve with known target concentrations. Alternatively, the target amount can be reported as x-fold decreased or increased amount based on a reference (= ΔCT). Low ΔCT values (small difference) indicate higher amounts of target relative to the reference compared to high ΔCT (big difference). It is suitable to re-calculate the ΔCT by subtracting it from a fixed value (such as the number of PCR cycles, e.g. 40). The result is a value with direct correlation to target amount (high value = high amount) and expressed as 40-ΔCT values, wherein one integer refers to a doubling of the target amount (e.g., a value of 34 indicates an amount which is twice as much as that with a value of 33). Depending on the desired reproducibility and precision of the system, it is possible to panel multiple reference assays or to re-calculate/normalize the ΔCT of the sample with the ΔCT of a calibrator (1 point calibration; Pfaffl (2001), Nucleic Acid Res., 29(9):e45). By using different fluorophores for specific probes it is also possible to multiplex different target assays in the same reaction. During PCR, each target in the multiplex is amplified in parallel, but separately detected utilizing the different fluorescent emission.

In some embodiments, the 40-ΔCT value is calculated as follows: 40 - [CT of the respective biomarker (e.g., HLA-E, HLA-F or HLA-G) of a patient sample - CT of a reference gene (e.g., CALM2) of a patient sample] (= calculation method 1). If more than one reference gene is used, the 40-ΔCT value is calculated as follows: 40 - (CT of the respective biomarker of a patient sample - mean CT of selected reference genes of a patient sample) (= calculation method 2). Alternatively, a 40-ΔΔCT value can be used, wherein the 40-ΔΔCT can be calculated as follows: ΔΔCT = 40 - [(CT biomarker of a patient sample - CT biomarker of a reference sample) - (CT reference gene of patient sample - CT reference gene of a reference sample)] (= calculation method 3); e.g., 40-ΔΔCT = 40 - [(CT HLA-G patient sample - CT HLA-G reference sample) - (CT CALM2 of a patient sample - CT CALM2 of a reference sample)]. In some embodiments, CALM2 is used as reference gene.

For instance, the relative expression level of the biomarkers is given as a 40-ΔΔCT value, which is calculated as follows: 40 - [(CT biomarker of a patient sample - CT reference gene of the patient sample) - (CT biomarker of a control sample - CT reference gene of the control sample)] (= calculation method 4); e.g., 40-ΔΔCT = 40 - [(CT HLA-G patient sample - CT Mean CombRef patient sample) - (CT HLA-G control sample - CT Mean CombRef control sample)]. In some embodiments, the CT is the median CT. The CT of the reference gene can be the CT of a single reference gene or the mean CT of two or more reference genes (referred to as Mean CombRef). Preferably, the same control sample (also referred to as calibrator) is used in all analyses and leads to the same RT-qPCR or qPCR results. In some embodiments, the control sample is a cell line RNA, an *in vitro* transcribed artificial RNA or an equimolar mixture of DNA oligonucleotides, representing the biomarker mRNA or cDNA or the biomarker amplicon or a part of the biomarker amplicon with a constant ratio. In some embodiments, CALM2 and/or B2M are used as reference genes and a positive control (e.g., *in vitro* transcribed artificial RNA) is used as control sample (calibrator).

In one another exemplary embodiment, the mean cut-off value is given as a 40-ΔΔCT value according to calculation method 4, wherein the mean cut-off value for HLA-G is a 40-ΔΔCT value of 40.10.

In some embodiments, the steps of the method (e.g. steps (a), (b), (c) and (d) ) are performed in random order. In a preferred embodiment, step (a) is performed first, i.e., before steps (b), (c) and (d). In some embodiments, step (d) is performed after steps (a), (b) and (c). In some embodiments, step (a) is performed before step (b), step (b) is performed before step (c), and step (c) is performed before step (d).

The probes as defined above are preferably labeled, e.g., with a label selected from a fluorescent label, a fluorescence quenching label, a luminescent label, a radioactive label, an enzymatic label and combinations thereof. Preferably, the probes as defined above are dual-label probes comprising a fluorescence reporter moiety and a fluorescence quencher moiety.

Novelties of the present invention include not only the mRNA-based determination of HLA-based cancer biomarkers in bladder cancer, but also the algorithmic inclusion of the subtypes.

All these new aspects of the present invention contribute to the prognostic value of the RT-qPCR-based approach and kit of the present invention. In fact, these novelties provide a more accurate and meaningful HLA typing in molecular subtypes of patients particularly in the advanced stage, which ultimately provides a prognostic tool for more individualized treatment decisions in cancer.

The present invention is further illustrated by the following examples which are not be construed as limiting the scope of the invention.

### EXAMPLES

### Example 1: Determination of mRNA expression levels by reverse transcription (RT) quantitative PCR (RT-qPCR)

RNA was isolated from formalin-fixed paraffin-embedded tissues (= FFPE tissues). More particularly, total RNA from a 5 to 10 µm curl of FFPE tumor tissue was extracted using the RNXtract^{®} Extraction Kit (BioNTech Diagnostics GmbH, Mainz, Germany) and qualified by real-time fluorescence RT-qPCR of a fragment of the reference gene CALM2. In general, 2,5 µl RNA of each qualified extraction (approx. 50-100 ng) were assayed by RT-qPCR as described below.

For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant splice variants. All primer pairs were checked for specificity by conventional PCR reactions.

TaqMan^{®} validation experiments were performed showing that the efficiencies of the target and the control amplifications were approximately equal, which is a prerequisite for the relative quantification of gene expression by the comparative ΔCT method. To perform the expression analysis of genes of interest within a biological sample, 4x duplex assay-mixtures were prepared by mixing the respective primers/probes of two specific assays. For separate detection of CT values, the assay probes were modified with different fluorescent probes. Each 4x assay-mix contained 2 µM of unmodified forward and reverse primers and 1,2 µM of probe. For each reaction, 2,5 µl total RNA extracted from FFPE sections (see above) were mixed with 2,5 µl assay-mix, 2,5 µl enzyme-mix and 2,5 µl water in one well of a 96-well-optical reaction plate. Measurements of the PCR reaction were done according to the instructions of the manufacturer with a Versant kPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles). Prior to the measurement of so far unclassified biological samples, control experiments with, e.g., cell lines, healthy control samples, samples of defined molecular tumor subtypes can be used for standardization of the experimental conditions.

### Example 2: Comparison of classical and non-classical HLA Genes by DNA sequence

Genome Analysis and Sequence alignment were done by accessing UCSC genome browser (https://genome.ucsc.edu/cgi-bin/hgGateway) and downloading the genomic sequences of HLA-A1 (NM_002116.7), HLA-A2 (NM_001242758.1), HLA-G (NM_0021275), HLA-F1 (NM_001098479.1), HLA-F2 (NM_018950.2), HLA-F3 (NM_001098478.1), HLA-J (NR_024240.1) and the putative sequence of HLA-H (NR_001434.4). The initial alignment analysis focused on the potential translation initiation region and the potential transition from extracellular alpha domains into the transmembrane region. HLA-H is thought to be a pseudogene due to single-base-pair deletion in exon 4 causing a frame shift, resulting in a premature stop codon in exon 4 (Chorney et al., 1990. Transcription analysis, physical mapping, and molecular characterization of a non-classical human leukocyte antigen class I gene. Mol. Cell. Biol. 10:243-253 and Zemmour et al., 1990. HLA-AR, an inactivated antigen-presenting locus related to HLA-A. J. Immunol. 144:3619-3629). Such definition of pseudogenes as being potentially defined by the loss of function in their protein coding ability due to mutations. However, sequence analysis revealed that the surrounding nucleotides of the ATG at 5' and 3' end are in line with the necessities of a Kozak Sequence.

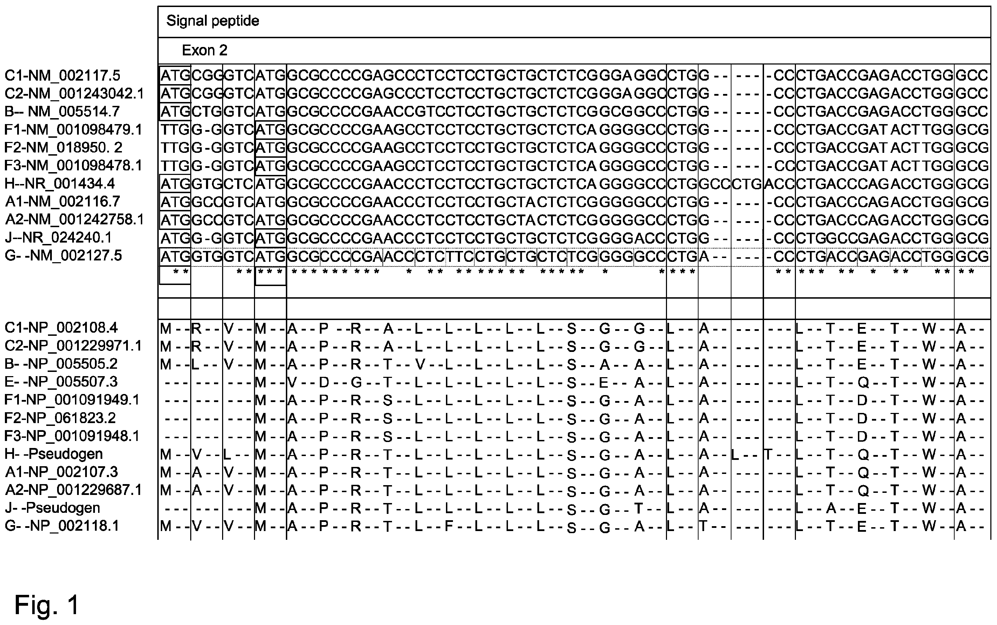

**Figure 1** depicts the sequence alignment of HLA-A 1, -A2, -B, -E, -F1, -F2, -F3, -J, -G and HLA-H at the potential translation initiation site. Potential start codons are highlighted by black frame.

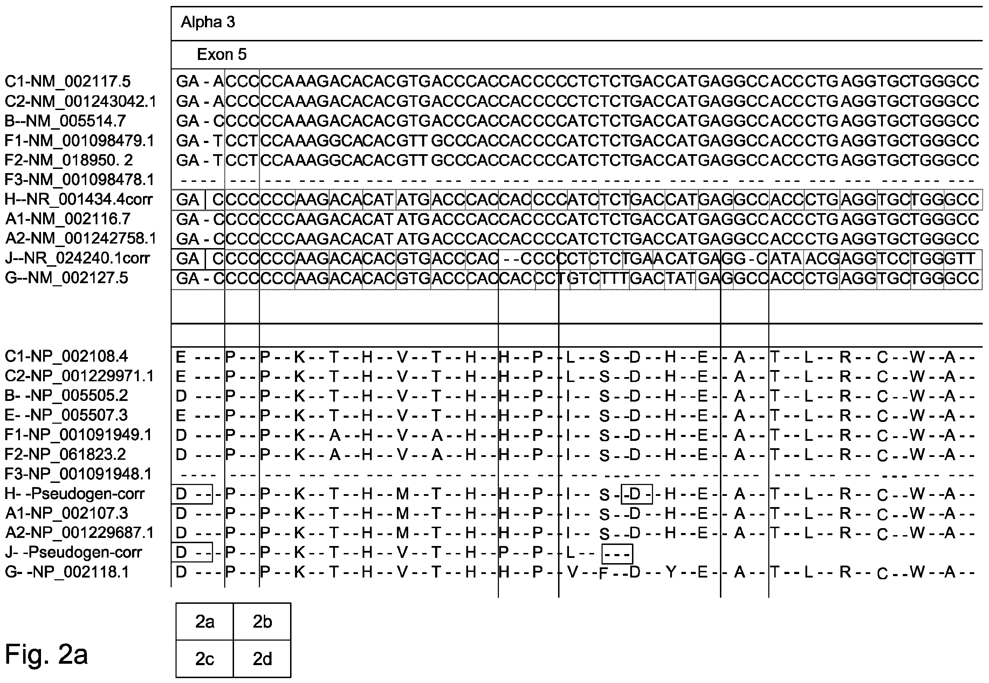

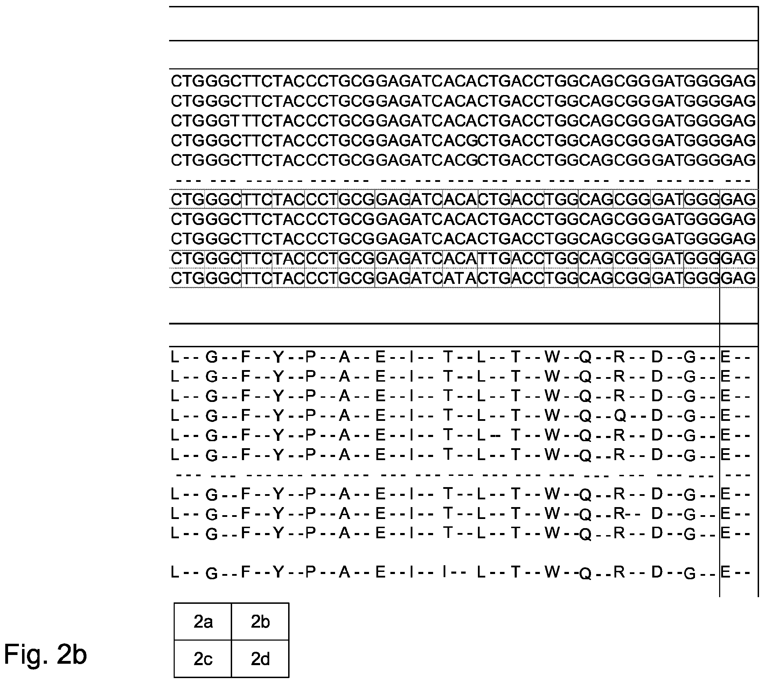

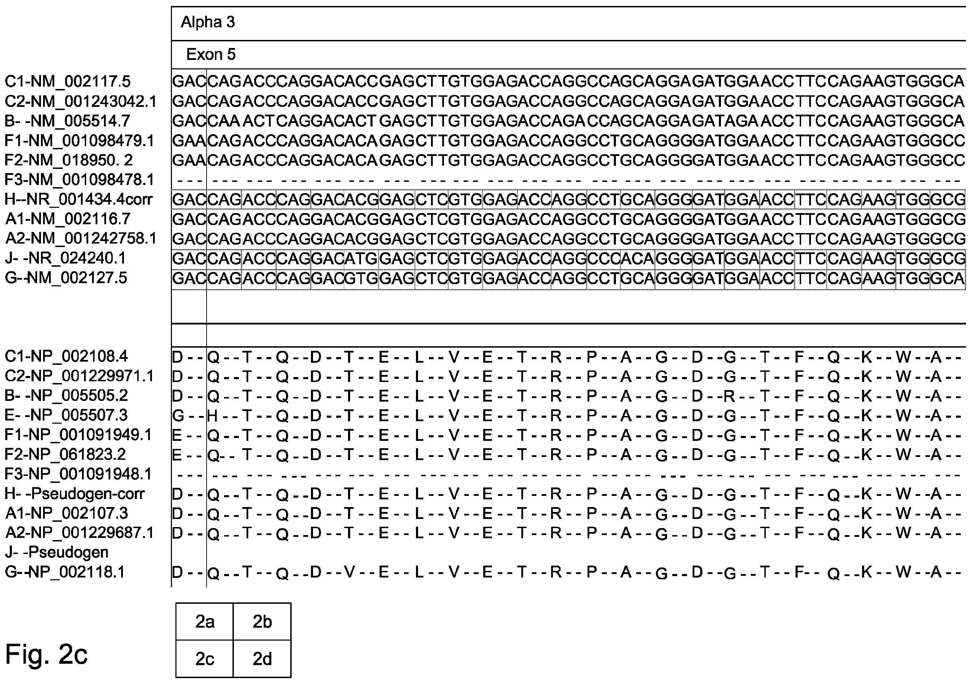

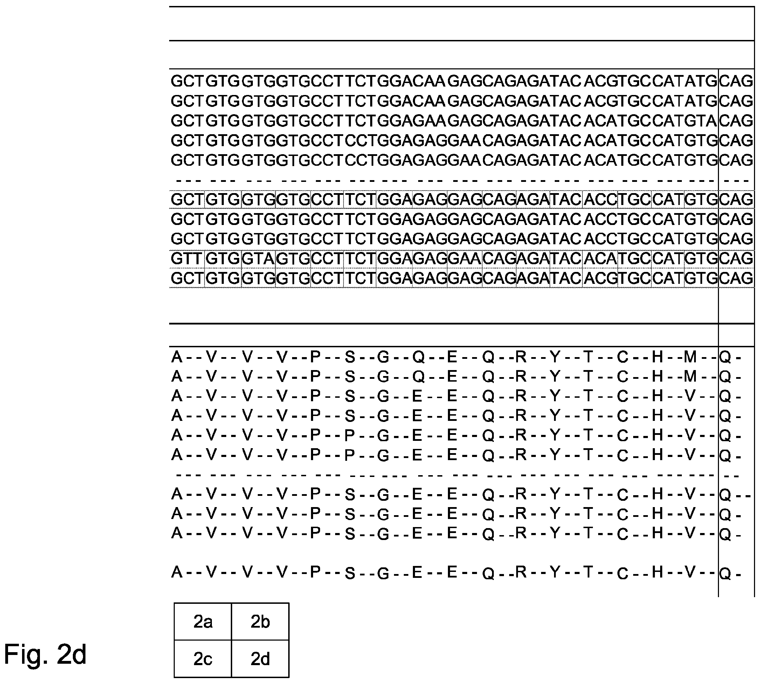

**Figure 2** depicts sequence alignment of HLA-A1, -A2, -B, -E, -F1, -F2, -F3, -J, -G and HLA-H at the exon 4 to exon 5 junction. The sequence with the premature stop codon is depicted by a yellow background.

As previously described, HLA-H had been defined in the literature as a pseudogene due to premature stop codon in exon 4. They identified a sequence GAC-CAG-ACC-CA- -CAC (single nucleotide deletion highlighted in red), which causes the in-frame shift. Comparing the sequence from Chorney et al (depicted by a yellow background in **Figure 2****),** investigators could not observe the single base pair deletion (depicted by a red background in **Figure 2****).** This observation leads to the assumption that HLA-H is also a full length protein and therefore not a pseudogene. Furthermore, the investigators identified the sequence in Exon 5, which encodes the alpha 3 domain. Even though, single base pair deletion would case a premature stop codon at the end of exon 5, this would mean that HLA-H lacks the transmembrane and cytoplasmatic domain. For HLA-G, it is known that transcript variant with a premature stop codon in intron 5 causes the translation of the soluble isoform HLA-G5. HLA-H would therefore be a soluble relative of the soluble HLA-G5. The soluble HLA-G forms are active proteins, causing immune cell inhibition through the interaction with various receptors such as the leukocyte immunoglobulin like receptor 1 and 2 (LILRB1 and LILRB2), the Killer Cell Immunoglobulin-like Receptor 2DL4 (KIR2DL4) and CD8 (Rajagopalan, S. and E. Long, KIR2DL4 (CD158d): An activation receptor for HLA-G. Frontiers in Immunology, 2012. 3(258) and Carosella, et al., Beyond the increasing complexity of the immunomodulatory HLA-G molecule. Blood, 2008. 111(10): 4862-70).

The literature named a second single nucleotide deletion in exon 7 at the end of sequence ctc-acg-gcg-tg-. Investigators identified this sequence in Exon 8, which encodes the untranslated region and is not relevant for protein translation.

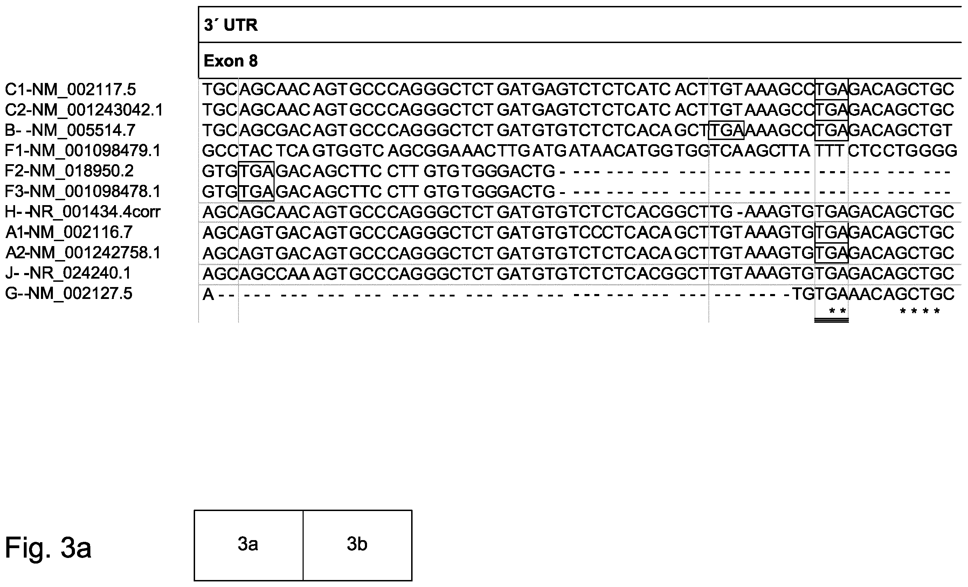

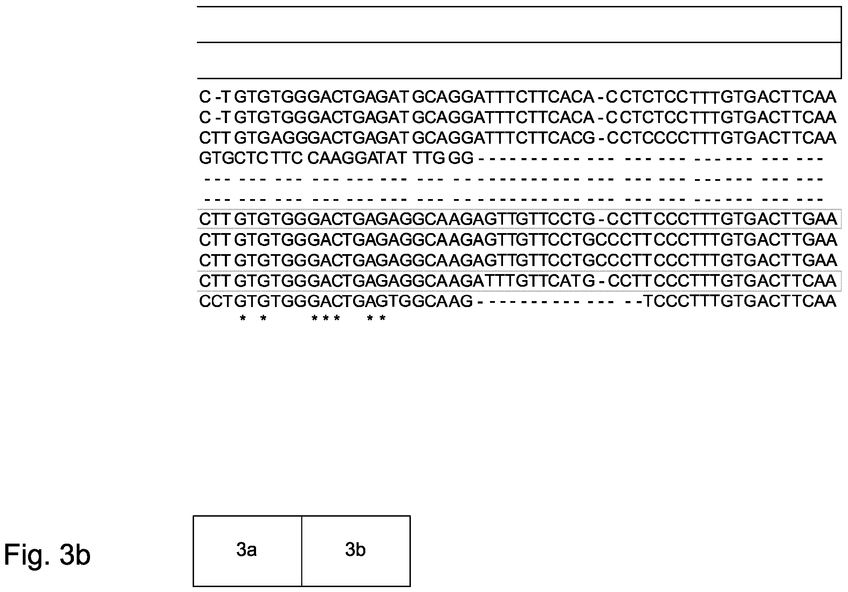

**Figure 3** depicts sequence alignment of HLA-A1, -A2, -B, -E, -F1, -F2, -F3, -J, -G and HLA-H at the exon 8. The sequence with the premature stop codon is depicted by a yellow background.

Investigators also identified the sequence GAC-CAG-ACC-CA- with the assumed single base pair deletion from Chorney et al also in Exon5 of HLA-J, the second pseudogene from the sequence alignments. Sequence comparison between HLA-H and HLA-J revealed that these two pseudogenes share 69% sequence homology for RNA and only 20% between the amino acid sequences **(Table 3).**

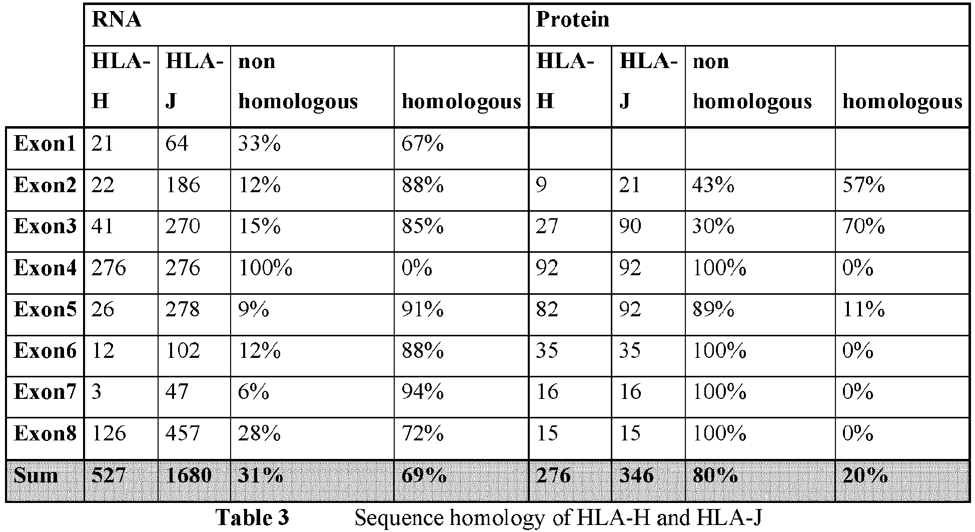

**Table 4** summarizes the homologies of between HLA-H and HLA class I genes (HLA-A, B, C), non classical HLA class I genes (HLA-E, F and G) and further pseudogenes (HLA-J, L, V, Y). HLA-H RNA is 77.4% homologous to HLA class I genes (HLA-A, B, C), non classical HLA class I genes (HLA-E, F and G) and 22,6% non homologous. Considering protein sequences, HLA-H is 27.3 % non homologous to classical and non-classical HLA class I genes (HLA-A1, A2, B, C, E, F1, F2, F3, G) and 58.8% non homologous to HLA-J.

**Table 4 Sequence homology and non homology of HLA-H mRNA and protein sequences with HLA class I genes and pseudogenes in percent**

| | HLA-H Protein | | HLA-H mRNA | |
|---|---|---|---|---|
| | Homologous [%] | non homologous [%] | Homologous [%] | non homologous [%] |
| HLA-A1 | 77,0 | 23,0 | 87,3 | 12,7 |
| HLA-A2 | 75,2 | 24,8 | 83,2 | 16,8 |
| HLA-B | 79,7 | 20,3 | 81,9 | 18,1 |
| HLA-C | 76,6 | 23,4 | 81,9 | 18,1 |
| HLA-E | 66,2 | 33,8 | 71,3 | 28,7 |
| HLA-F1 | 69,9 | 30,1 | 69,7 | 30,3 |
| HLA-F2 | 69,9 | 30,1 | 76,0 | 24,0 |
| HLA-F3 | 68,0 | 32,0 | 69,6 | 30,4 |
| HLA-G | 72,1 | 27,9 | 76,0 | 24,0 |
| HLA-J Frame3 | 41,2 | 58,8 | 65,1 | 34,9 |
| HLA-L Frame1 | 17,6 | 82,4 | 59,3 | 40,7 |
| HLA-V Frame1 | 69,2 | 30,8 | 61,0 | 39,0 |
| HLA-Y (DEL) | 80,2 | 19,8 | 91,9 | 8,1 |

### Example 3: Determination of HLA mRNA expression levels by reverse transcription (RT) quantitative PCR (RT-qPCR) in a immunetherapy treated urothelial cancer cohort

Seventy-two newly diagnosed patients with histologically confirmed urothelial cancer, including bladder cancer and upper urothelial tract carcinoma were enrolled in the study between 2016 and 2018. The initial study population of 72 patients was restricted to 61, after excluding six patients whose biopsy samples were not adequate and five patients due to lymph node metastasis. Within the urothelial cancer (UC) cohort, 49 patients suffered from urothelial bladder cancer (UBC) and 12 patients from carcinomas of the upper urothelial tract. Nivolumab, Pemprolizumab and Atezolizumab were given as 1^{st}, 2^{nd} and 3^{rd} line mono-treatment according to approved instructions.

For survival analysis, disease specific survival (DSS) was used for Kaplan meier survival estimates and cox regression analysis. Complete survival data were available from 61 patients. At time of data closure, the median DSS was at 4.32 months.

Gene specific TaqMan-based Primer/Probe sets for the assessment of the expression of HLA genes were used. For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant or selected splice variants as appropriate All primer pairs were checked for specificity by conventional PCR reactions. After further optimization of the primers/probes, the primers and probes listed in **Table 5** gave the best results. These primers/probes are superior to primers/probes known from the prior art, e.g., in terms of specificity and amplification efficiency. To standardize the amount of sample RNA, the CALM2 was selected as reference gene, since they were not differentially regulated in the samples analyzed. Paired samples having low RNA content (i.e. Raw CT values for CALM2 of less than 22) for pretreatment biopsy or post treatment resectate were excluded.

**Table 5. Used primers and probes for HLA-H mRNA quantitation**

| Gene | Forward Primer | | Reverse Primer |
|---|---|---|---|
| HLA-A (MP779) | GTAACTTCTTCCTTCCCTATTAAAATTAGA (SEQ ID NO. 1) | TTTACTTTCTCAAATTCTTGCCATGAGAGGTTGATG (SEQ ID NO. 2) | TGGACTCTGGAAGGTTCTCATG (SEQ ID NO. 3) |
| HLA-B/C (MP783) | CCATCTCTGTCTCAAATTCATGGT (SEQ ID NO. 4) | CACTGAGCTGCAACTTCTTACTTCCCTAATGA (SEQ ID NO. 5) | CAGGTCTTTATTTGCTCTCTCAACTTC (SEQ ID NO. 6) |
| HLA-G-Ex3 (MP728) | GGCCGGAGTATTGGGAAGA (SEQ ID NO. 7) | CAAGGCCCACGCACAGACTGACA (SEQ ID NO. 8) | GCAGGGTCTGCAGGTTCATT (SEQ ID NO. 9) |
| HLA-G Ex4 (MP730) | CTGCGGCTCAGATCTCCAA (SEQ ID NO. 10) | CGCAAGTGTGAGGCGGCCAAT (SEQ ID NO. 11) | CAGGTAGGCTCTCCTTTGTTCAG (SEQ ID NO. 12) |
| HLA-G Ex5 (MP743) | CACCACCCTGTCTTTGACTATGAG (SEQ ID NO. 13) | ACCCTGAGGTGCTGGGCCCTG (SEQ ID NO. 14) | AGTATGATCTCCGCAGGGTAGAAG (SEQ ID NO. 15) |
| HLA-G Ex6 (MP744) | CATCCCCATCATGGGTATCG (SEQ ID NO. 16) | TGCTGGCCTGGTTGTCCTTGCA (SEQ ID NO. 17) | CCGCAGCTCCAGTGACTACA (SEQ ID NO. 18) |
| HLA-G Ex8 (MP747) | GACCCTCTTCCTCATGCTGAAC (SEQ ID NO. 19) | CATTCCTTCCCCAATCACCTTTCCTGTT (SEQ ID NO. 20) | CATCCCAGCCCCTTTTCTG (SEQ ID NO. 21) |
| HLA-G Ex3-5'(MP757) | TTCATCGCCATGGGCTACG (SEQ ID NO. 22) | CGACACGCAGTTCGTGCGGTTC (SEQ ID NO. 23) | ATCCTCGGACACGCCGAGT (SEQ ID NO. 24) |
| HLA-G Ex2/3 (MP779) | CCGAACCCTCTTCCTGCTGC (SEQ ID NO. 25) | CGAGACCTGGGCGGGCTCCC (SEQ ID NO. 26) | GCGCTGAAATACCTCATGGA (SEQ ID NO. 27) |
| HLA-H Ex 2/3 (MP802) | GAGAGAACCTGCGGATCGC (SEQ ID NO. 28) | AGCGAGGGCGGTTCTCACACCATG (SEQ ID NO. 29) | CCACGTCGCAGCCATACAT (SEQ ID NO. 30) |
| HLA-H (MP803) | TGGCCCTGACCCTGACCC (SEQ ID NO. 31) | AGACCTGGGCGCGCTCCCAC (SEQ ID NO. 32) | CGGGCCGGGACATGGT (SEQ ID NO. 33) |
| CALM2 | GAGCGAGCTGAGTGGTTGTG (SEQ ID NO. 34) | TCGCGTCTCGGAAACCGGTAGC (SEQ ID NO. 35) | AGTCAGTTGGTCAGCCATGCT (SEQ ID NO. 36) |

TaqMan^{®} validation experiments were performed showing that the efficiencies of the target and the control amplifications were approximately equal, which is preferable for the relative quantification of gene expression by the comparative ΔCT method. To perform the expression analysis of genes of interest within a biological sample, 4x duplex assay-mixtures were prepared by mixing the respective primers/probes of two specific assays. For separate detection of CT values, the assay probes were modified with different fluorescent probes. Each 4x assay-mix contained 2 µM of unmodified forward and reverse primers and 1,2 µM of probe. For each reaction, 2,5 µl total RNA extracted from FFPE sections (see above) were mixed with 2,5 µl assay-mix, 2,5 µl enzyme-mix and 2,5 µl water in one well of a 96-well-optical reaction plate. Measurements of the PCR reaction were done according to the instructions of the manufacturer with a Versant kPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles).

The determination of luminal and basal subtypes in the UC cohort by RT-qPCR revealed a broad dynamic range of KRT5 and KRT20 mRNA ranging from 40-DCT values of 19 to 48 in similar ranges. The dynamic range for PD-1 and PD-L1 mRNA expression is ranging from 19 to 41 for both mRNA analyses. The dynamic range for the FGFR genes is rather individual within the FGFR family. The dynamic range for FGFR1 is ranging from 40-DCT values of 29 to 37, for FGFR2 from 19 to 39 40-DCT values, FGFR3 from 19 to 43 and for FGFR4 from 19 to 36 40-DCT values.

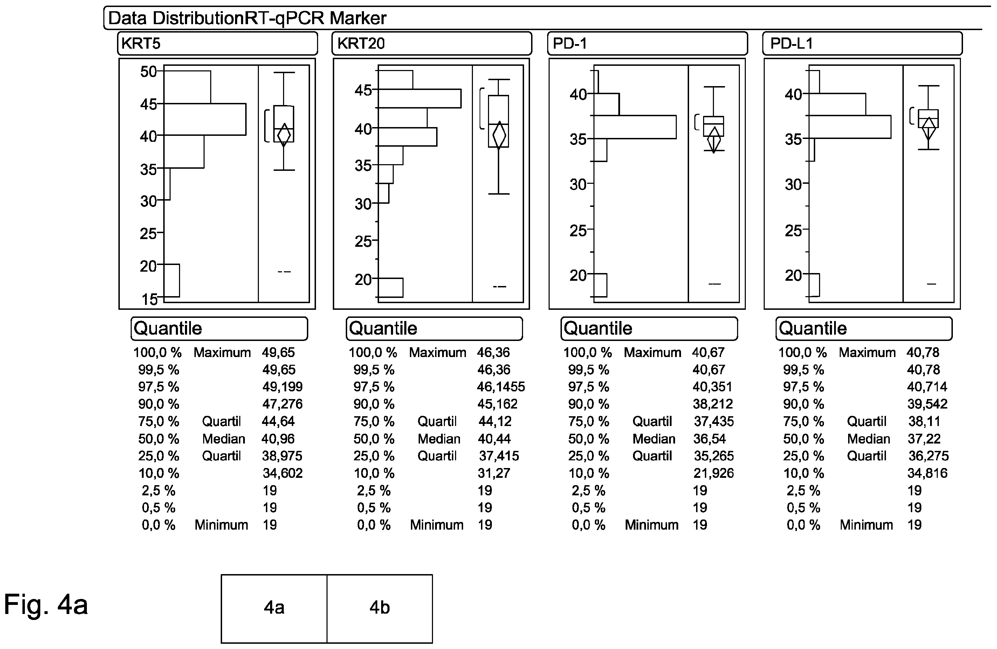

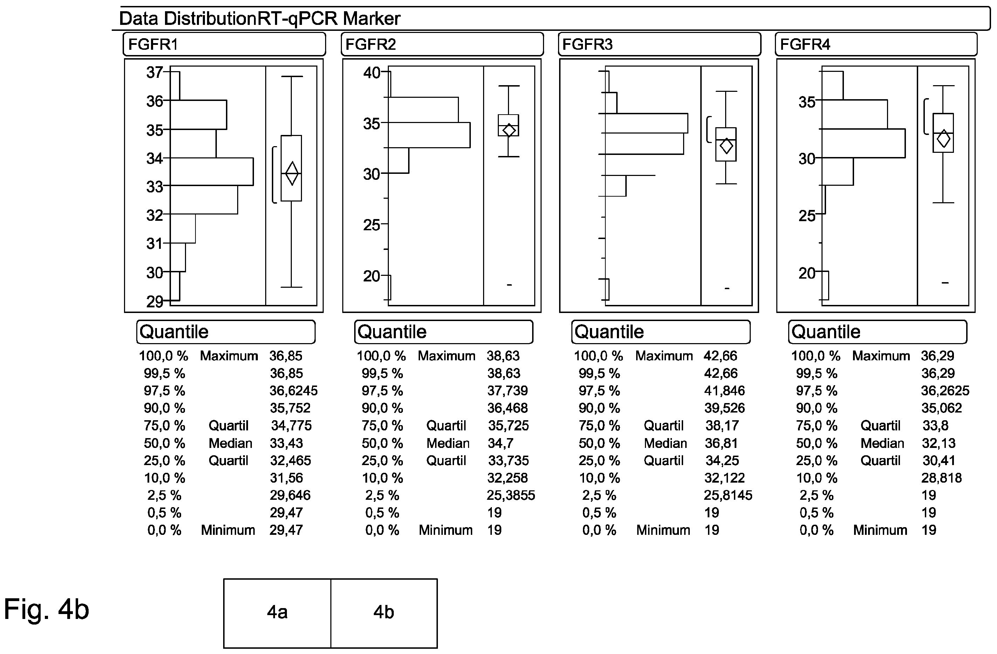

**Figure 4** depicts data distribution of luminal and basal subtype markers, check point target genes and FGFR1 to 4 gene expression as determined by RT-qPCR from FFPE tissues from muscle invasive bladder cancer patients (n=61).

The spearman correlation revealed a high significant co-expression of FGFR receptors 2 (p=0.0008) and 3 (p=0.0066) within the luminal urothelial cancer cell type (KRT20). For the basal like urothelial cancer cases no significant upregulation of any FGFR gene could be observed. In addition, FGFR2 and FGFR3 expression is significantly associated with low PD-1 (p=0.02554) and PD-L1 (p=0.0074) mRNA expression. However, the check point markers PD-1 (p=0.0004) and PD-L1 (p=0.0452) showed high significant expression in the basal like urothelial cancer subtype (KRT5). Both, high PD-1 and PD-L1 mRNA expression are associated with infiltration of immune cells into the tumor tissue as has been previously described (Eckstein et al., Oncotarget 2018).

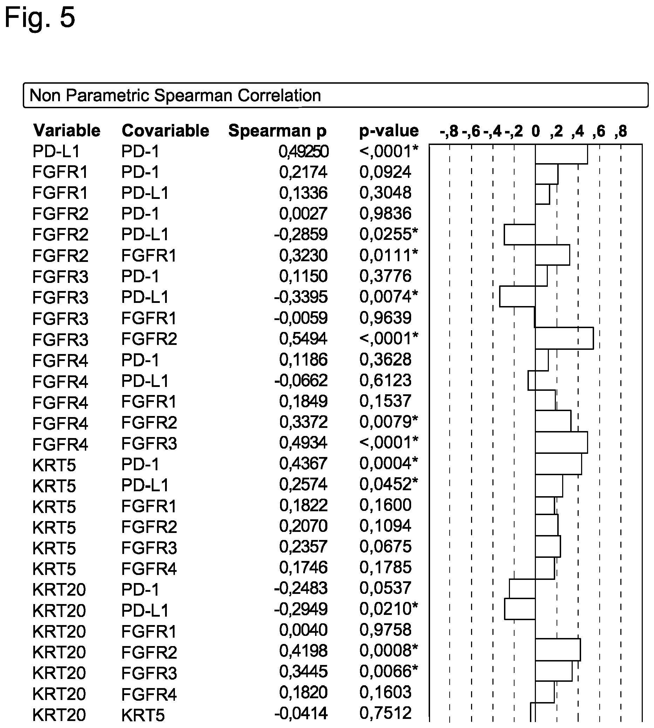

**Figure 5** depicts intergene spearman correlation of luminal and basal subtype markers, check point target genes and FGFR1 to 4 gene mRNA expression as determined by RT-qPCR from tissues from muscle invasive bladder cancer patients (n=61).

In addition to the mRNA expression analysis of luminal and basal markers, PD-1, PD-L1 and the FGFR family, the expression profile of classical and non classical HLAs have been carried out.

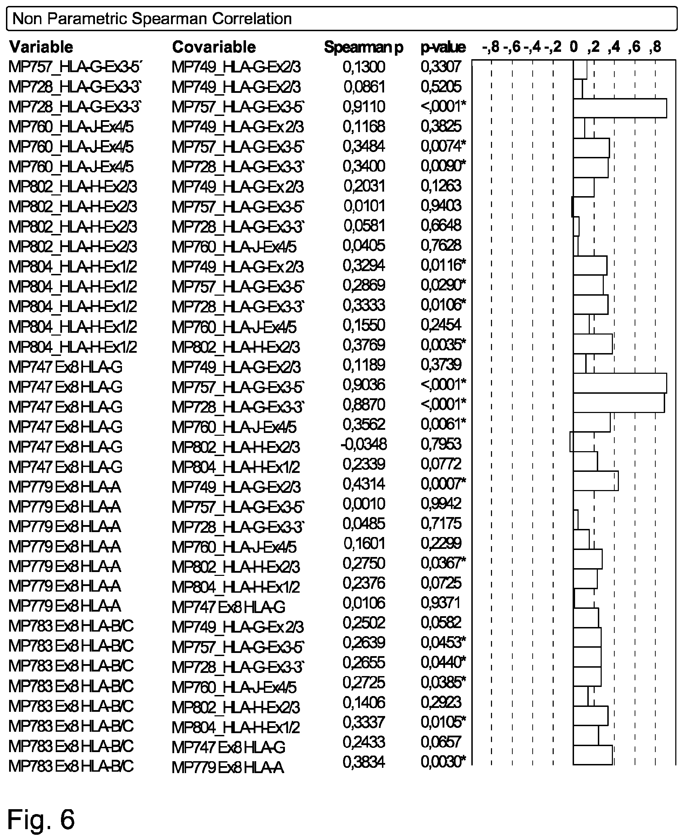

**Figure 6** depicts intergene spearman correlation of HLA gene mRNA expression as determined by RT-qPCR from FFPE tissues from muscle invasive bladder cancer patients (n=61).

As shown in **Figure 6****,** the intergene correlation of diverse HLA genes displays a complex pattern. As an example, HLA-J expression is only moderately correlated with non-classical HLA-G or classical HLA-A or HLA-B/C gene expression with Spearman correlation coefficients in the range of 0.34, 0,16 and 0,27, respectively. Similarly, the other pseudogene HLA-H as exemplified by the HLA-H Ex1/2 assay only marginally or moderately correlates with classical and non-classical HLAs such as HLA-G, HLA-A, HLA-B/C, HLA-J (r=0.23993, r=0.2376, r=0.3373, r=0,1550). Importantly there are substantial differences of the correlation coefficients of one HLA such as HLA-H ex1/2 vs HLA-H ex2/3 when comparing with other HLA gene fragments. This implies differential splicing events resulting in an intra- and intergenic interplay of clinical relevance.

As one example of the intergenic and intragenic interplay of HLA gene expression affecting the disease specific survival of cancer patients undergoing immune therapy, we have analyzed the mRNA expression of HLA-G in combination with HLA-A. Both genes were determined by highly specific Assays determining unique regions in the comparably heterologous parts of HLA gene after the translational stop in exon 7.

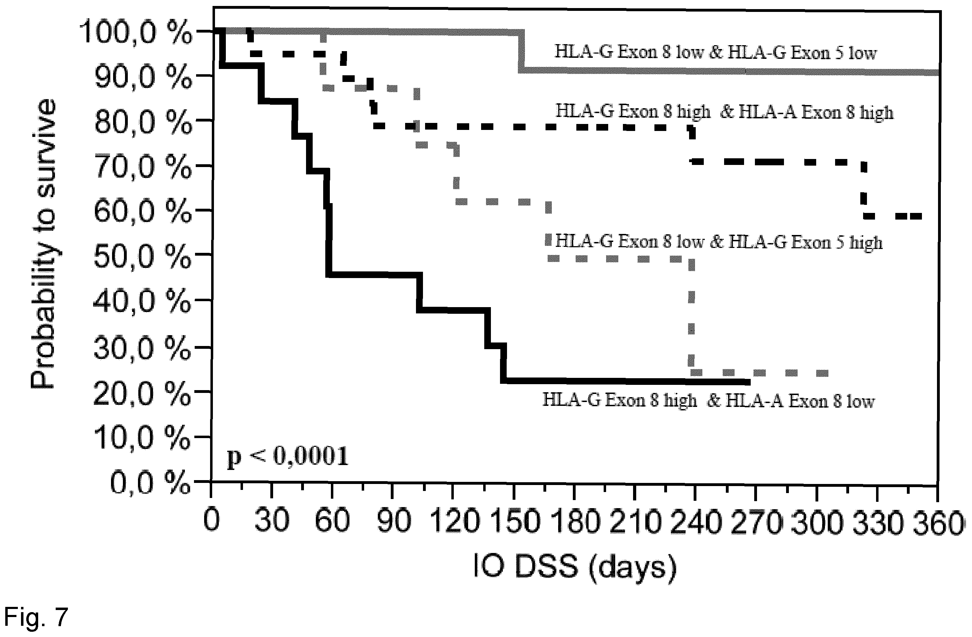

**Figure 7** depicts a Kaplan Meier Plot displaying disease specific survival (DSS) probability from FFPE tissues of muscle invasive bladder cancer patients (n=61) based on stratification by combining HLA-A exon 8, HLA-G exon 8 and HLA-G exon 5 mRNA expression as quantified by RT-qPCR assay. Relative mRNA expression is determined by the 40-DCT method using CALM2 as reference gene. Patients, whose tumor exhibited low HLA-G Exon 8 mRNA (<28,43) and low HLA-G exon 5 mRNA (<37,11) expression exhibited best survival (grey, solid line). Patients, whose tumor exhibited low HLA-G Exon 8 mRNA (<28,43) but high HLA-G exon 5 mRNA (>37,11) expression, exhibited second worst survival (grey, dotted line). Patients, whose tumor exhibited high HLA-G Exon 8 mRNA (>28,43) and high HLA-A exon 8 mRNA (>35,26) expression, exhibited second best survival (black, dotted line). Patients, whose tumor exhibited high HLA-G Exon 8 mRNA (>28,43) but low HLA-A exon 8 mRNA (<35,26) expression, exhibited worst survival (black, solid line).

As displayed in **Figure 7** urothelial cancer patients being treated with immune oncology drugs after failure of preceding chemotherapy do have worst survival when HLA-G Exon 8 is expressed, but HLA-A Exon 8 is not expressed (compare blue curve with golden curve). These data indicate that the presence of classical HLA's may compensate the otherwise fatal expression of non-classical HLA's when patients are being treated with immune-oncology ("IO") treatment. This also shows that immune modulatory drugs targeting the check point inhibition (such as anti-PD1 / anti PDL1) seem to be more efficient in tumors with at least partially intact classical HLA function. Moreover, these data demonstrate the superiority of combined determination of more than one HLA gene to specify the prognostic value of individual HLA regions, which is prerequisite to maximize effectiveness of immune therapies and to reduce risks of potential hazards associated with combinations of chemotherapy and immune therapies.

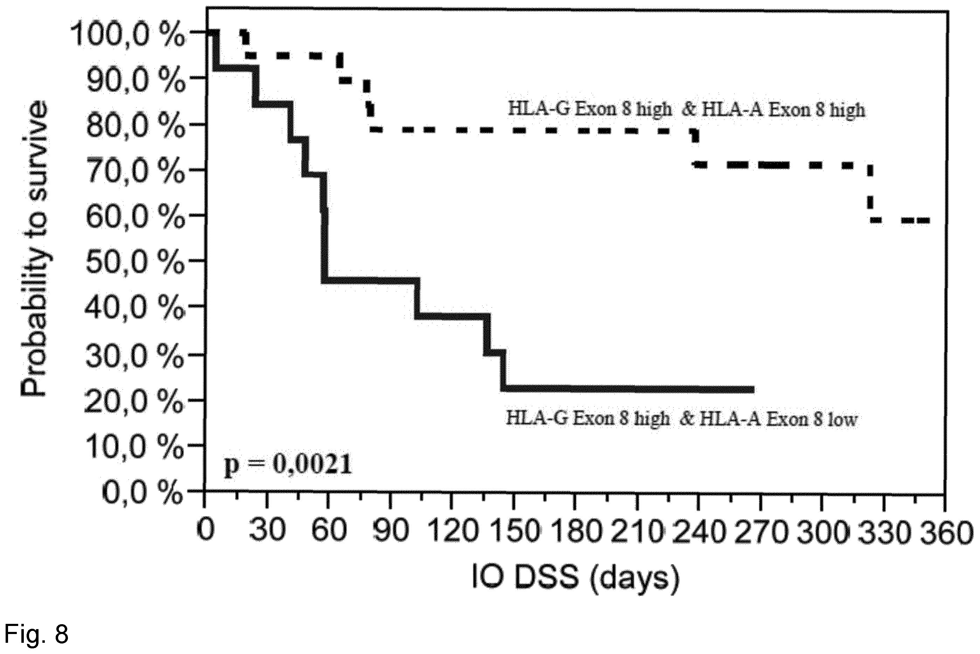

**Figure 8** depicts a Kaplan Meier Plot displaying disease specific survival (DSS) probability from FFPE tissues of muscle invasive bladder cancer patients (n=61) based on stratification by intergenic combination of HLA-A exon 8 and HLA-G exon 8 mRNA expression as quantified by RT-qPCR assay. Relative mRNA expression is determined by the 40-DCT method using CALM2 as reference gene. Patients, whose tumor exhibited high HLA-G Exon 8 mRNA (>28,43) and high HLA-A exon 8 mRNA (>35,26) expression, exhibited second best survival (black, dotted line). Patients, whose tumor exhibited high HLA-G Exon 8 mRNA (>28,43) but low HLA-A exon 8 mRNA (<35,26) expression, exhibited worst survival (black, solid line).

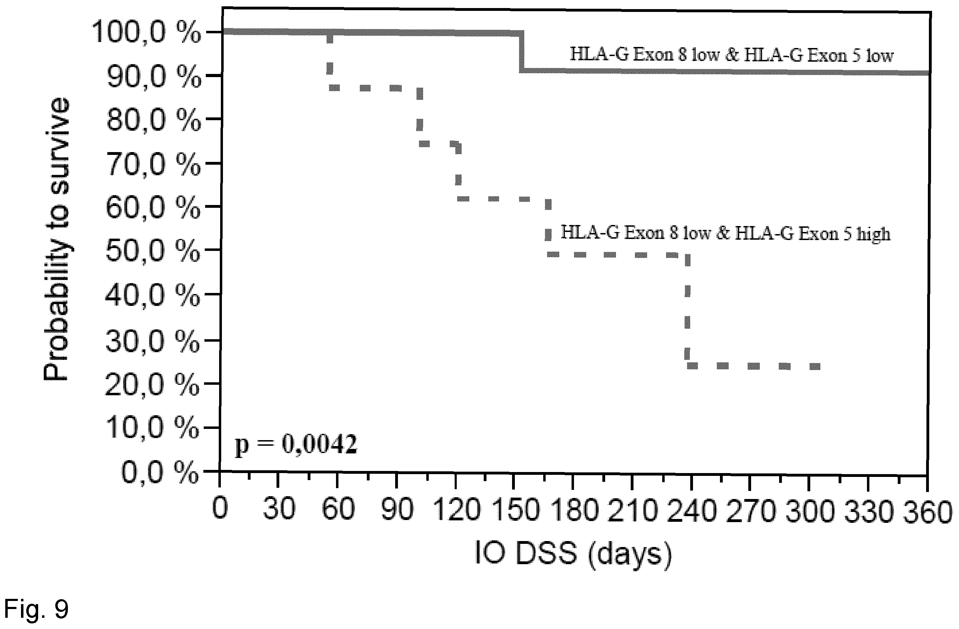

**Figure 9** depicts Kaplan Meier Plot displaying disease specific survival (DSS) probability from FFPE tissues of muscle invasive bladder cancer patients (n=61) based on stratification by intragenic combination of HLA-G exon 8 and exon 5 mRNA expression as quantified by RT-qPCR assay. Relative mRNA expression is determined by the 40-DCT method using CALM2 as reference gene. Patients, whose tumor exhibited low HLA-G Exon 8 mRNA (<28,43) and low HLA-G exon 5 mRNA (<37,11) expression, exhibited best survival (grey, solid line). Patients, whose tumor exhibited low HLA-G Exon 8 mRNA (<28,43) but high HLA-G exon 5 mRNA (>37,11) expression, exhibited worse survival (grey, dotted line).

As an example of the intragenic interplay of HLA gene expression affecting the disease specific survival of cancer patients undergoing immune-therapy, we have analyzed the HLA-G expression of HLA-G Ex8 and HLA-G Ex5 by RT-qPCR. By determining the HLA-G Ex8 the untranslated exon at the 3'end of the gene (after the C-terminal, cytoplasmic protein end is quantified. This enables the unique determination of a multitude of HLA-G splice variants that might include or exclude e.g. diverse extracellular alpha domains and/or the transmembrane region as well as cytoplasmic parts. This kind of HLA-G determination is not possible by antibodies on protein level and represents a highly specific HLA-G assessment. When setting the multitude of HLA-G splice variant expression in relation to the expression of HLA-G Ex5, resembling the alpha 3 domain, it becomes apparent, that by quantifying the combination of two HLA-G mRNA fragments one can distinguish different prognostic subgroup of patients having superior or inferior disease specific survival, when the time from initiation of immune-oncology treatment ("IO") treatment to death is being taken into account. Patients having low expression of HLA-G exon 8 containing splice variants but simultaneously higher levels of HLA-G exon 5 containing fragments do have higher risk of disease specific death despite having started IO treatment in the advanced chemotherapy refractory setting.

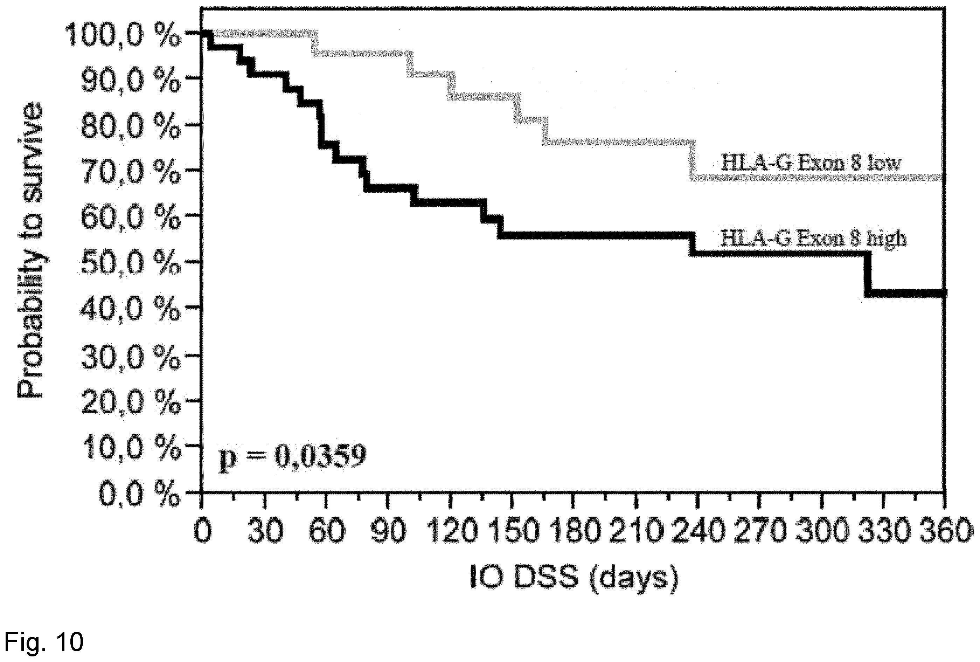

To prove the superiority of the combinatorial HLA diagnostics, the prognostic value of single gene determination was compared. As displayed in **Figure 10** the determination of HLA-G Exon 8 alone is also of general prognostic value (p=0,0359). However as shown in Figure 7 there is a multitude of patients (n=20 low risk as being HLA-A Exon 8 high in n=33 "high risk" based on high HLA-G Exon 8 mRNA expression) in the "bad prognosis" group of high single gene HLA-G Exon 8 mRNA expression as simultaneously exhibiting high expression of HLA-A Exon 8. Therefore almost 2/3 of the patients in the "bad prognosis" group can be spared additional treatment when performing more precise tissue diagnostics for HLA gene combinations.

**Figure 10** depicts a Kaplan Meier Plot displaying disease specific survival (DSS) probability from FFPE tissues of muscle invasive bladder cancer patients (n=61) based on stratification by single gene determination of only HLA-G exon 8 and exon 5 mRNA expression as quantified by RT-qPCR assay. Relative mRNA expression is determined by the 40-DCT method using CALM2 as reference gene. Patients, whose tumor exhibited high HLA-G Exon 8 mRNA (>28,43) and high HLA-A exon 8 mRNA (<28,43) expression, exhibited good survival (grey, dotted line). Patients, whose tumor exhibited high HLA-G Exon 8 mRNA (>28,43), exhibited worse survival (black, solid line).

### Example 4: Determination of HLA sense and HLA antisense mRNA expression levels by reverse transcription (RT) quantitative PCR (RT-qPCR) in a neoadjuvantly treated ovarian cancer patient cohort

Further, inventors determined whether combinatorial use of more than one HLA group gene sequence would be also applicable for other types of tumors apart from bladder cancer such as gynecologic cancer, in particular ovarian cancer. In addition, the inventors determined whether the combinatorial use nt only of HLA group genes with HLA group antisense expression can be determined to predict outcome in cancer. Moreover, the investigators evaluated whether the HLA sense and antisense combinations can be used to predict response/non response to the therapeutic regimen apart from immune-oncology treatment such as chemotherapy and/or hormonal therapy.

Forty-five newly diagnosed patients with histologically confirmed FIGO stage III-IV epithelial ovarian or peritoneal carcinoma unsuitable for optimal upfront surgery and candidate for neo-adjuvant chemotherapy (said carcinoma also referred to herein below as ovarian cancer) were enrolled in the study between September 2004 and December 2007. Other inclusion criteria were age >18 years, hematological, renal, hepatic and cardiac function adequate for platinum-based chemotherapy. Exclusion criteria were a Karnofsky performance status (KPS) lower than 70%, a history of other malignancies and contraindications for surgery. The possibility of optimal debulking surgery was excluded at baseline by open laparoscopy. The initial study population of 45 patients was restricted to 35, after excluding nine patients whose biopsy samples were not adequate for the microarray analysis and one patient found to be ineligible because of diagnosis of peritoneal mesothelioma after histological revision. A standard regimen of carboplatin AUC 5 and paclitaxel 175 mg/m2 Q3 over 3 h every 3 weeks was administered as neo-adjuvant treatment for six cycles. In three patients older than 75 years and in one patient with poor performance status (KPS 70%), single-agent carboplatin was preferred to the combination chemotherapy.

Histopathological response was evaluated after surgery, with surgical samples analysis. To date, no histopathological criteria have been firmly established to describe treatment response after neo-adjuvant chemotherapy in ovarian cancer. According to the literature concerning response to primary chemotherapy in ovarian (Le et al. 2007, Sassen et al. 2007) and breast cancer (Ogston et al. 2003), as complete pathological response the absence of cancer cells in surgical specimens, and as very good partial remission the persistence of only small clusters (<1 cm) or individual cancer cells and no macroscopic residual after surgery was considered. Partial pathological remission was defined as a tumor burden reduction between 30% and 90% at surgery, while stable disease was defined as no tumor burden reduction or reduction lower than 30% at surgery, compared with initial diagnostic laparoscopy. Only patients with complete and very good partial remissions were considered as pathological responders, while all the other cases were considered as pathological non-responders. For survival analysis, the time from initial diagnosis until progression (PFS) or until death (OS) or the time between progression and death (PDT) were used for Kaplan Meier survival estimates and cox regression analysis. Complete survival data were available from 40 patients. At time of data closure, the median PFS was at 14.7 months and the median OS was at 33.5 months, which is high given the very advanced stage of the disease at study entry (unresectable FIGO III - IV).

For mRNA detection, tissues collected were snap frozen and stored in liquid nitrogen until analysis. Approximately 20-100 mg of frozen ovarian tumor tissue was crushed in liquid nitrogen. RNA was extracted using commercial kits (Qiagen), RNA integrity was assessed on the Agilent 2100 Bioanalyzer (Agilent Technologies, Palo Alto, CA, USA), cDNA was synthesized from 1 mg of total RNA using Invitrogen kits (Invitrogen Corp.) and analyzed on Affymetrix HG-U133A microarrays (Affymetrix Inc., Santa Clara, CA, USA) as described elsewhere (Ihnen et al. 2008).

For validation purposes, RT-qPCR was applied to the total RNA isolated from identical fresh tissue biopsies as described above to validate the array data by an independent technical approach. Gene specific TaqMan-based Primer/Probe sets for the assessment of the expression of HLA-F or HLA-F AS were used. For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant or selected splice variants as appropriate. All primer pairs were checked for specificity by conventional PCR reactions. After further optimization of the primers/probes, the primers and probes listed in Table 6 gave the best results. These primers/probes are superior to primers/probes known from the prior art, e.g., in terms of specificity and amplification efficiency. To standardize the amount of sample RNA, the CALM2 was selected as reference gene, since they were not differentially regulated in the samples analyzed. Paired samples having low RNA content (i.e. Raw CT values for CALM2 of less than 22) for pretreatment biopsy or post treatment resectate were excluded.

**Table 6. Used primers and probes for HLA-F and HLA-F AS mRNA quantitation**

| Gene | NM-Nr | Forward Primer | Probe | Reverse Primer |
|---|---|---|---|---|
| HLA-F2+3 (MP781) | NM _018950+ NM _001098478 | TGTGAGACAGCTTCCTTGTGTG (SEQ ID NO. 37) | AATTCTGCTACATTGATATCTTGCTTCTCAGTCC (SEQ ID NO. 38) | TATGTATGTTCGTGAGGCACAAGTG (SEQ ID NO. 39) |
| HLA-F1 (MP782) | NM_001098479 | CTTTGGCTTCGGCTTTAGGA (SEQ ID NO. 40) | CTTCGTTCTTGGCACCATCTTATGAAAAGGGT (SEQ ID NO. 41) | CATGGGTCTTGCAACTTACTTTAGAAT (SEQ ID NO. 42) |
| HLA-F AS1 (MP839) | NR_026972 | CCAAGAAGGCAGTGTTGAAAGAT (SEQ ID NO. 43) | ATCCACATGTCACCCACCTTCCGG (SEQ ID NO. 44) | GGGTGCTCTTCCAAGGATATTTG (SEQ ID NO. 45) |
| HLA-F AS1 (MP840) | NR_026972 | AGGATTGCGGCCTGTTG (SEQ ID NO. 46) | AGAGTAGTGTCTTGGGCCCCAGCTGA (SEQ ID NO. 47) | CAGGGCATTGGATGTTGATATTC (SEQ ID NO. 48) |
| HLA-F AS1 (MP841) | NR_026972 | ACTCCCATGCAGAGAAGAAGCTC (SEQ ID NO. 49) | CACCTGTGGAGAGAGGATTGCGGC (SEQ ID NO. 50) | CTGGGGCCCAAGACACTACTC (SEQ ID NO. 51) |
| HLA-F3 spez. (MP842) | NM_001098478 | TGCTCCGCAGATACTTGGAGAAT (SEQ ID NO. 52) | CTACAGCGC GCAGAGCAGT CTCCC (SEQ ID NO. 53) | GCCAGCAACGATGCCCAC (SEQ ID NO. 54) |
| HLA-F1 spez. (MP843) | NM_001098479 | TCAGATAGAAACAGAGGGAGCTACTCT (SEQ ID NO. 55) | CTGCAGCCTACTCAGTGGTCAGCGG (SEQ ID NO. 56) | GAGAAATAAGCTTGACCACCATGTTA (SEQ ID NO. 57) |
| HLA-F2 (+1) spez. (MP844) | NM_018950 | TGGAGTTGCTCCGCAGATACT (SEQ ID NO. 58) | CCTTTGGAGGATCTGCGCGCTG (SEQ ID NO. 59) | AGATGGGGTGGTGGGCA (SEQ ID NO. 60) |
| HLA-F2 (+3) spez. (MP845) | NM_018950 | TCAGATAGAAACAGAGGGAGCTACTC (SEQ ID NO. 61) | CAGGCTGCAGTGTGAGACAGCTTCCTTG (SEQ ID NO. 62) | TTTATGTATGTTCGTGAGGCACAA (SEQ ID NO. 63) |
| HLA-F3 spez. (MP846) | NM_001098478 | AGGAATATGCAGAGGAGTTCAGGA (SEQ ID NO. 64) | AGTATCTGCGGAGCAACTCCAGGCACTC (SEQ ID NO. 65) | GACTGCTCTGCGCGCTGT (SEQ ID NO. 66) |
| HLA-F2 (+1) spez. (MP850) | NM_001098478 | AGGAATATGCAGAGGAGTTCAGGA (SEQ ID NO. 67) | AGTATCTGCGGAGCAACTCCAGGCACTC (SEQ ID NO. 68) | GGAGGATCTGCGCGCTGT (SEQ ID NO. 69) |

TaqMan^{®} validation experiments were performed showing that the efficiencies of the target and the control amplifications were approximately equal, which is a prerequisite for the relative quantification of gene expression by the comparative ΔCT method. To perform the expression analysis of genes of interest within a biological sample, 4x duplex assay-mixtures were prepared by mixing the respective primers/probes of two specific assays. For separate detection of CT values, the assay probes were modified with different fluorescent probes. Each 4x assay-mix contained 2 µM of unmodified forward and reverse primers and 1.2 µM of probe. For each reaction, 2.5 µl total RNA extracted from FFPE sections (see above) were mixed with 2.5 µl assay-mix, 2.5 µl enzyme-mix and 2.5 µl water in one well of a 96-well-optical reaction plate. Measurements of the PCR reaction were done according to the instructions of the manufacturer with a Versant qPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles).

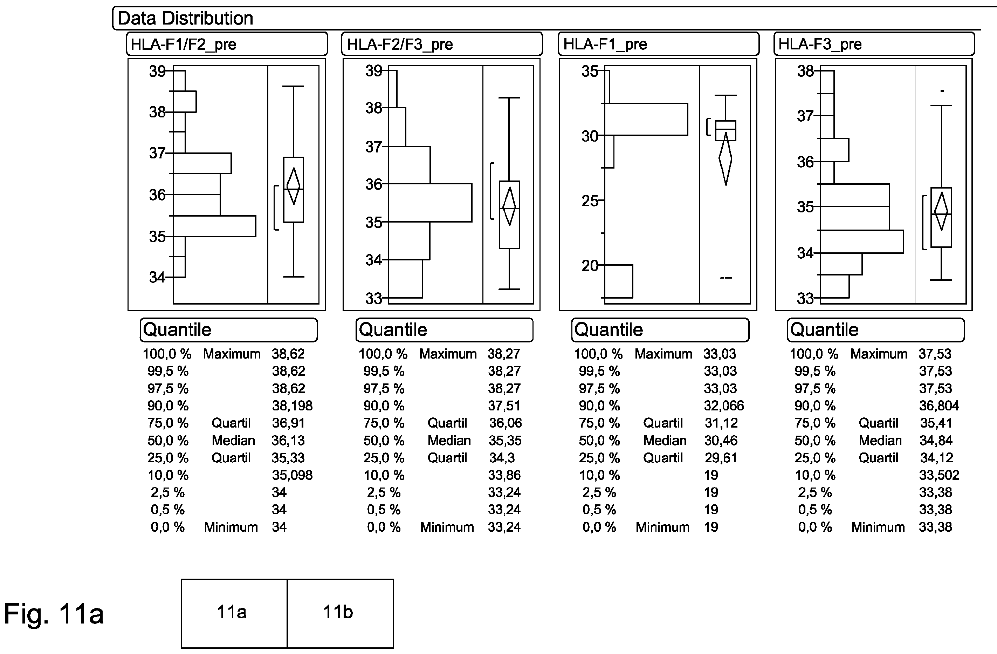

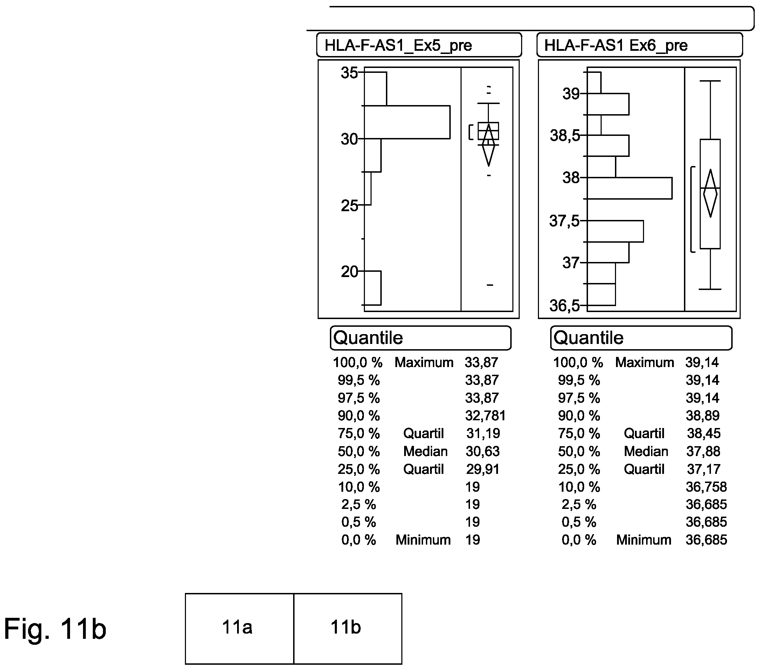

**Figure 11** depicts a data distribution of relative mRNA expression (40-DCT) of HLA-F isoforms and anti-sense HLA-F expression as determined by RT-qPCR. It depicts the relative mRNA expression levels of defined sense and anti-sense regions of HLA genes as exemplified for HLA-F. The three known HLA-F isoforms HLA-F 1, HLA-F2 and HLA-F3 as well as exons of the HLA-antisense isoforms AS1 and AS2 were determined by RT-qPCR after DNAse digestion of the nucleic acid extracts. Interestingly the expression level of different HLA-F AS regions differed markedly, with HLA-F AS1 Exon 6 expression being highest with a median 40-DCT before neoadjuvant chemotherapy of 37,88. Moreover subtractive analysis of the Isoform comparison revealed particularly high expression of HLA-F2 and HLA-F3 in pre-treatment biopsies of ovarian cancer samples.

The HLA-F expression was set into the context of molecular subtyping into hormone dependent luminal and hormone-independent ovarian cancer, which had been published before (Zamagni et al. Oestrogen receptor 1 mRNA is a prognostic factor in ovarian cancer patients treated with neo-adjuvant chemotherapy: determination by array and kinetic PCR in fresh tissue biopsies. ERC 2009). For this purpose the mRNA expression of ESR1, HLA-F3 and HLA_F AS1 exon 6 were combined by building decision tree models, gene ratios and linear combinations.

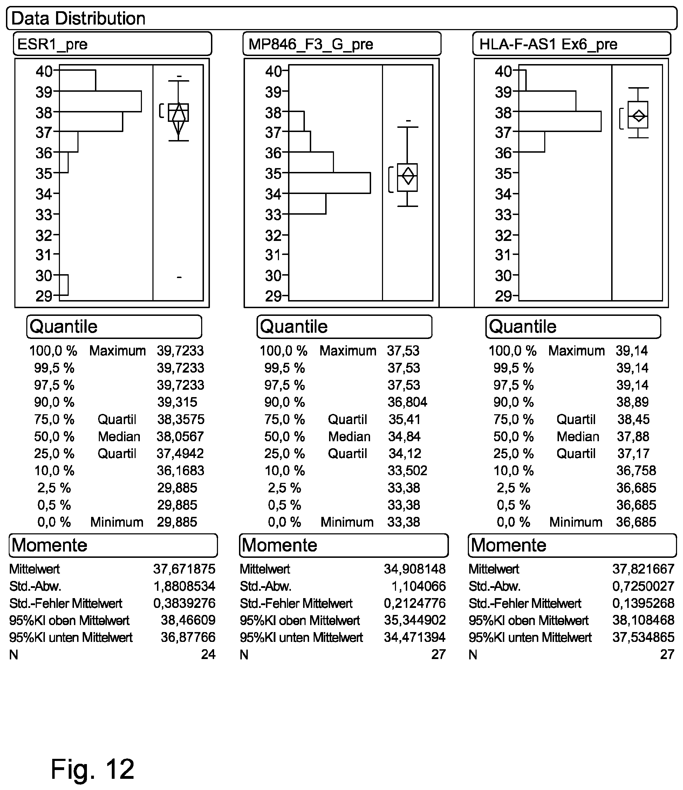

**Figure 12** depicts a data distribution of relative mRNA expression (40-DCT) of ESR1, HLA-F3 and HLA-F AS1 expression as determined by RT-qPCR.

HLA-F3 is a non-classical MHC I molecule harboring the extracellular alpha 1 and alpha 2 domains for formation of the peptide presenting protein groove for antigen presentation, but lacking the alpha 3 domain for interaction with immune cells such as activating T-cells or natural killer cells. As all known isoforms of HLA-F isoforms also HLA-F3 contains a transmembrane domain and is therefore thought to be present on the cell surface for immune cell interactions.

The predictive value of HLA-F3 mRNA expression was tested by partitioning test for progression free survival as endpoint.

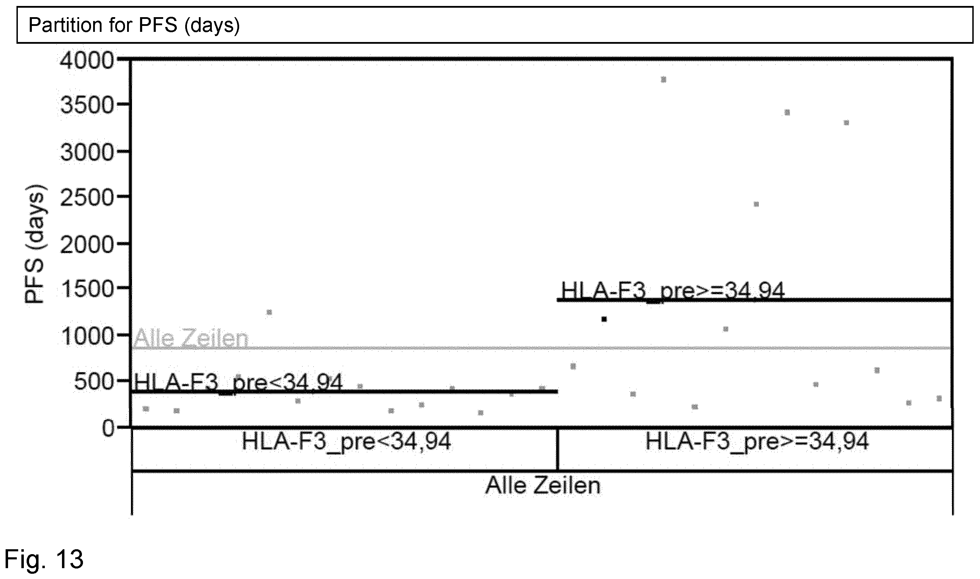

**Figure 13** depicts a partition test for HLA-F3 mRNA expression in pre-treatment biopsy samples of neoadjuvantly treated ovarian cancer patients determined by RT-qPCR to predict progression free survival.

As shown in **Figure 13****,** a cut-off close to the median expression of pre-treatment HLA-F3 (DCT 34,94) divided the neoadjuvant ovarian cancer cohort into two equally sized groups with markedly different median survival and high expression of HLA-F3 being associated with prolonged survival (1.392 days of progression-free survival) versus reduced survival upon low expression of HLA-F3 (400 days of progression-free survival).

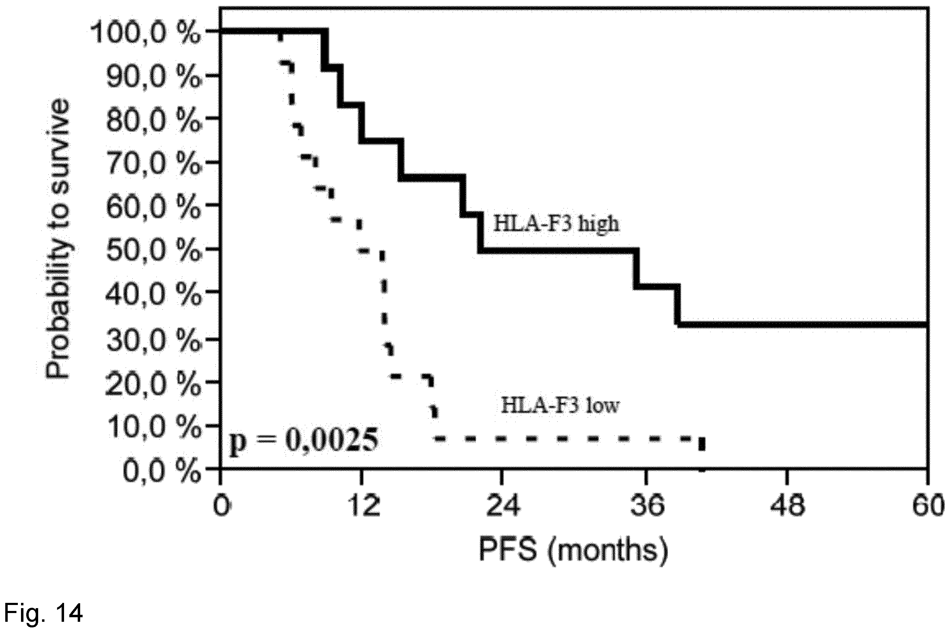

**Figure 14** depicts a Kaplan Meier Plot displaying progression free survival (PFS) probability based on stratification by single gene determination of only HLA-F3 as quantified by RT-qPCR assay from fresh tissues of advanced ovarian cancer patients (n=27). Relative mRNA expression is determined by the 40-DCT method using CALM2 as reference gene. Patients, whose tumor exhibited high HLA-F3 mRNA expression (>= 34,94) exhibited good survival (black, solid line). Patients, whose tumor exhibited low HLA-F3 mRNA expression (< 34,94) exhibited worse survival (black, dashed line).

Kaplan Meier analysis proved the significance of predicting survival by HLA-F3 mRNA expression. As depicted in **Figure 14****,** patients exhibiting high HLA-F3 mRNA expression in the primary ovarian cancer tissue had a median progression free survival of 28,5 months, while patients with low HLA-F3 mRNA expression exhibited diminished median progression free survival of 12,5 months.

Similarly, the overall survival analysis by Kaplan Meier method revealed significant survival differences when stratifying based on HLA-F3 mRNA. As depicted in **Figure 14****,** patients exhibiting high HLA-F3 mRNA expression in the primary ovarian cancer tissue had a median overall survival of 52,5 months, while patients with low HLA-F3 mRNA expression exhibited diminished median overall survival of 22,9 months.

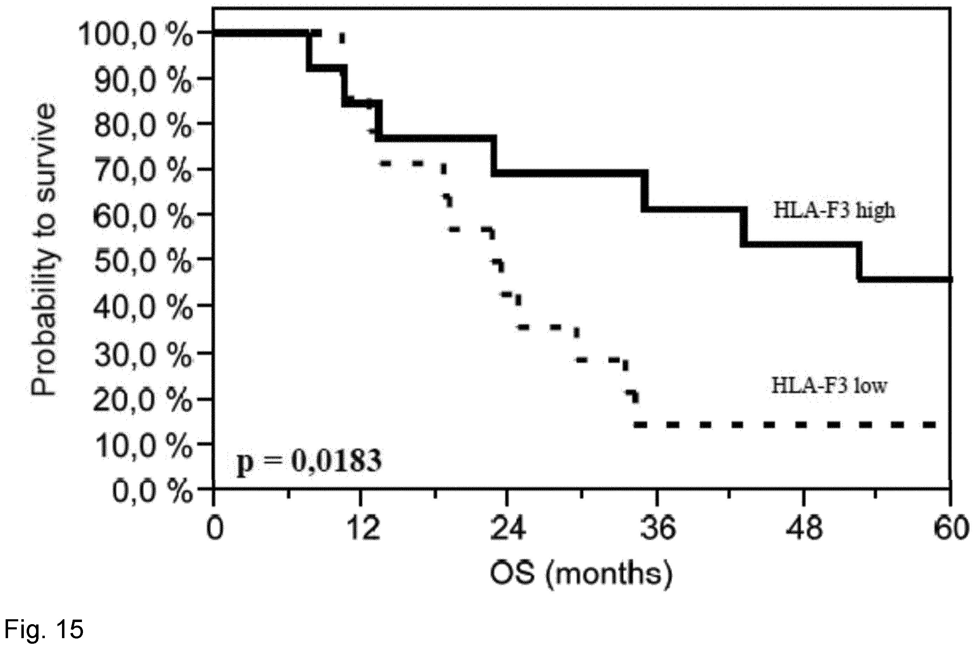

**Figure 15** depicts a Kaplan Meier Plot displaying overall survival (OS) probability based on stratification by single gene determination of only HLA-F3 as quantified by RT-qPCR assay from fresh tissues of advanced ovarian cancer patients (n=27). Relative mRNA expression is determined by the 40-DCT method using CALM2 as reference gene. Patients, whose tumor exhibited high HLA-F3 mRNA expression (>= 34,94) exhibited good survival (black, solid line). Patients, whose tumor exhibited low HLA-F3 mRNA expression (< 34,94) exhibited worse survival (black, dashed line).

When adjusting for clinical parameters such as Grade, FIGO stage and primary site (ovary versus peritoneum) the mRNA stratification into high and low mRNA expression remained to be an independent factor for predicting progression free survival with a L-R Chi² value of 6.14 (p=0,0132) and a hazard ratio of 0,22, while all other clinical factors were not significant.

**Figure 15** is based on a multivariate analysis for PFS using cox proportional hazards models including Grade, FIGO stage, Primary site and HLA-F3 mRNA expression. When adjusting prediction of overall survival for clinical parameters such as Grade, FIGO stage and primary site (ovary versus peritoneum) the mRNA stratification into high and low mRNA expression remained to be an independent factor for predicting progression free survival with a L-R Chi² value of 3.19 (p=0,0441) and a hazard ratio of 0.31, while all clinical factors were not significant.

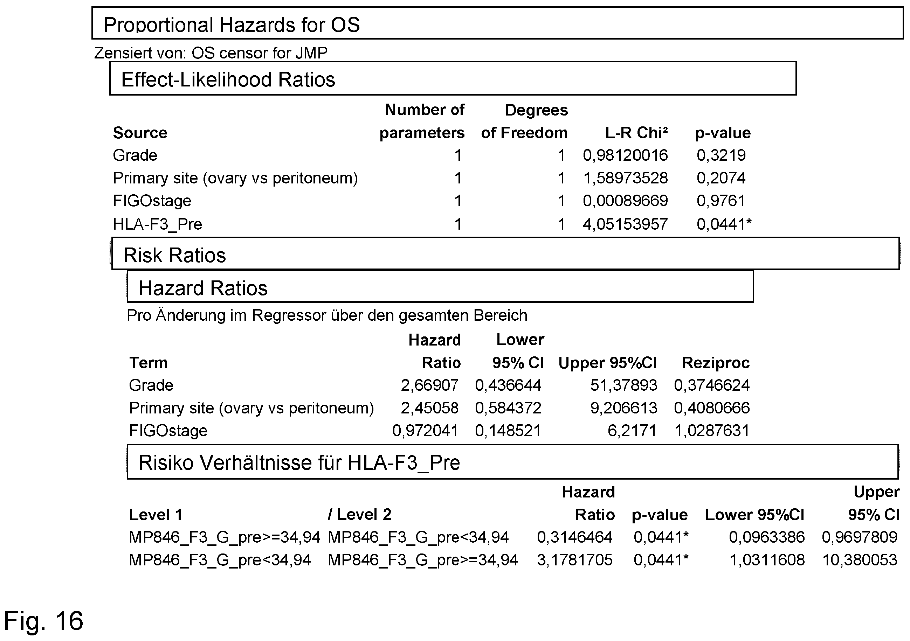

**Figure 16** depicts a multivariate analysis for OS using cox proportional hazards models including Grade, FIGO stage, Primary site and HLA-F3 mRNA expression.

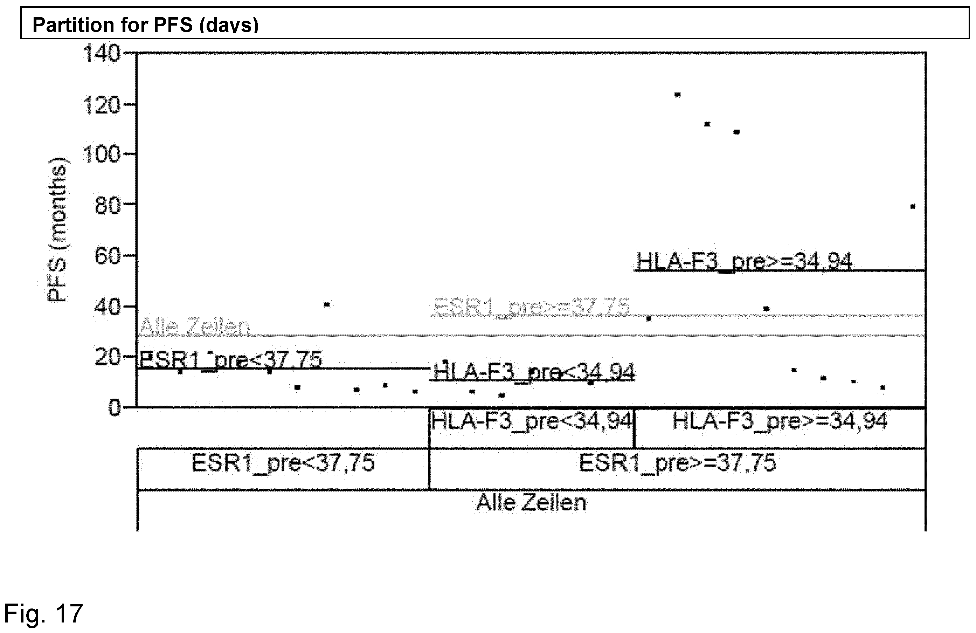

As a next step, the HLA-F3 expression has been set into the context of molecular subtypes by discriminating ESR1 mRNA levels into hormone dependent and hormone independent ovarian carcinomas. ESR1 mRNA stratification using a 40-DCT value if 37,75 discriminated between 37% of ovarian cancers being ERS1 negative and having a median progression free survival of approximately 15,72 month from ESR1 positive ovarian cancer accounting for 63% of all ovarian cancer patients having a median progression free survival of 36.47 months (Fig. 17).

**Figure 17** depicts a Partition test for ESR1 and HLA-F3 mRNA expression in pre-treatment biopsy samples of neoadjuvantly treated ovarian cancer patients determined by RT-qPCR to predict progression free survival.

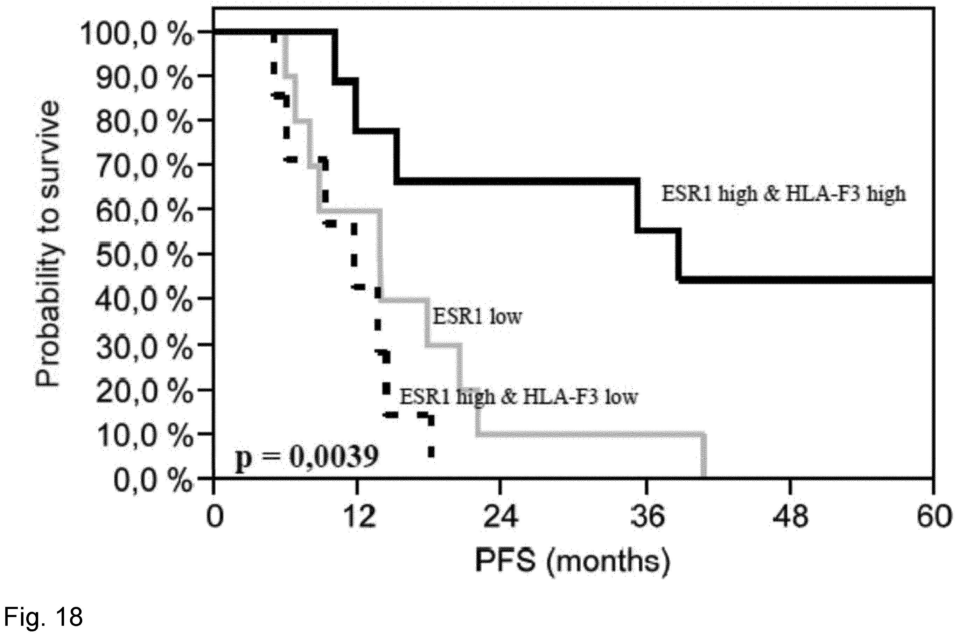

**Figure 18** depicts a Kaplan Meier Plot displaying progression free survival (PFS) probability based on stratification ESR1 and HLA-F3 mRNA expression as quantified by RT-qPCR assay from fresh tissues of advanced ovarian cancer patients (n=27). Relative mRNA expression is determined by the 40-DCT method using CALM2 as reference gene. Patients, whose tumor exhibited high ESR1 mRNA expression (>= 37,75) and high HLA-F3 mRNA expression (>= 34,94) exhibited good survival (black, solid line). Patients, whose tumor exhibited high ESR1 mRNA expression (>= 37,75) and low HLA-F3 mRNA expression (< 34,94) exhibited worse survival (black, dashed line). Patients with low ESR1 mRNA expression (< 37,75) also exhibited a bad prognosis (grey, solid line).

Kaplan Meier analysis proved the significance of predicting progression free survival by combining ESR1 and HLA-F3 mRNA expression. As depicted in **Figure 18****,** patients exhibiting high ESR1 and high HLA-F3 mRNA expression in the primary ovarian cancer tissue had a median progression free survival of 38,7 months, while patients with high ESR1and low HLA-F3 mRNA expression exhibited diminished median progression free survival of 11,6 months. Patients with low ESR1 mRNA expression exhibited similar bad prognosis.

In addition, Kaplan Meier analysis also proved the significance of predicting overall survival by combining ESR1 and HLA-F3 mRNA expression. As depicted in **Figure 18****,** patients exhibiting high ESR1 and high HLA-F3 mRNA expression in the primary ovarian cancer tissue had a median overall survival of 38,7 months, while patients with high ESR1and low HLA-F3 mRNA expression exhibited diminished median progression free survival of 11,6 months. Patients with low ESR1 mRNA expression exhibited similar bad prognosis.

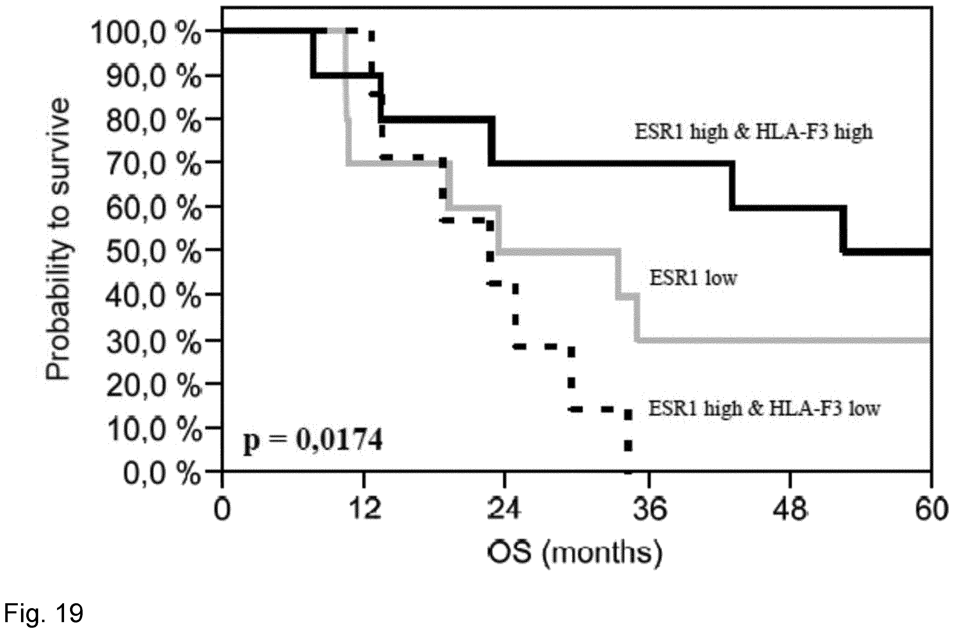

**Figure 19** depicts a Kaplan Meier Plot displaying overall survival (OS) probability based on stratification ESR1 and HLA-F3 mRNA expression as quantified by RT-qPCR assay from fresh tissues of advanced ovarian cancer patients (n=27). Relative mRNA expression is determined by the 40-DCT method using CALM2 as reference gene. Patients, whose tumor exhibited high ESR1 mRNA expression (>= 37,75) and high HLA-F3 mRNA expression (>= 34,94) exhibited good survival (black, solid line). Patients, whose tumor exhibited high ESR1 mRNA expression (>= 37,75) and low HLA-F3 mRNA expression (< 34,94) exhibited worse survival (black, dashed line). Patients with low ESR1 mRNA expression (< 37,75) also exhibited a bad prognosis (grey, solid line).

When adjusting for clinical parameters such as grade, FIGO stage and primary site (ovary versus peritoneum) the combinatorial mRNA stratification based on ESR1 and HLA-F3 mRNA expression remained to be an independent factor for predicting progression free survival with a L-R Chi² value of 9.53 (p=0,0095), while all other clinical factors were not significant. The hazard ratio of high ESR1 and high HLA-F3 mRNA expression reached a hazard ratio of 0.097 and 0.152 when comparing to ESR1 high & HLA-F3 low or ESR1 low (p=0.0042 and p=0.0136, respectively.

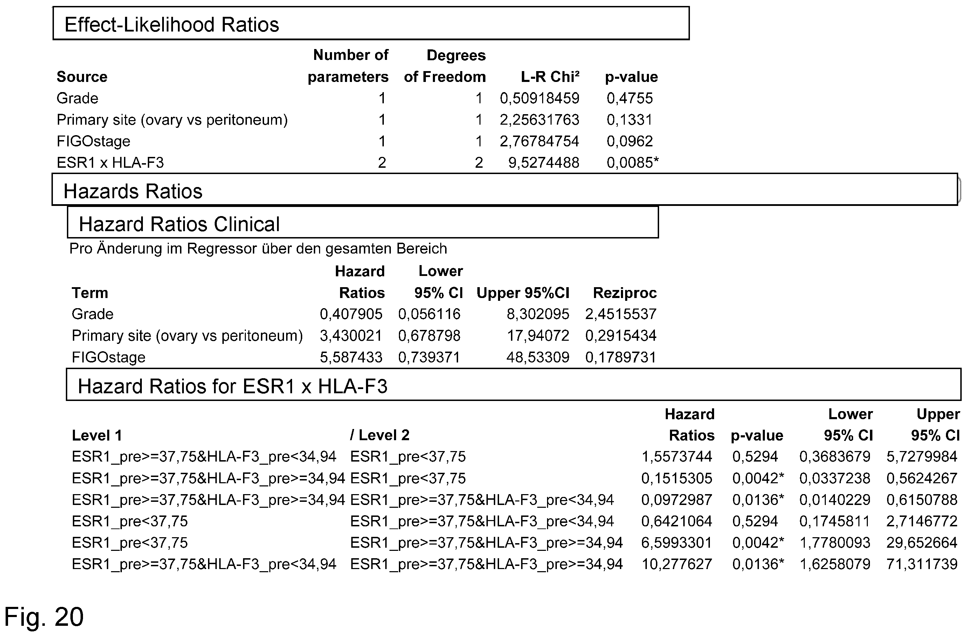

**Figure 20** depicts a multivariate analysis for PFS using cox proportional hazards models including Grade, FIGO stage, Primary site and the combination of ESR1 and HLA-F3 mRNA expression.

In addition, when analyzing overall survival and adjusting for the clinical parameters grade, FIGO stage and primary site (ovary versus peritoneum) the combinatorial mRNA stratification based on ESR1 and HLA-F3 mRNA expression remained to be an independent factor for predicting progression free survival with a L-R Chi² value of 6.53 (p=0,0383), while all other clinical factors were not significant. The hazard ratio of high ESR1 and high HLA-F3 mRNA expression reached a hazard ratio of 0.184 and 0.230 when comparing to ESR1 high & HLA-F3 low or ESR1 low (p=0.0832 and p=0.0182, respectively).

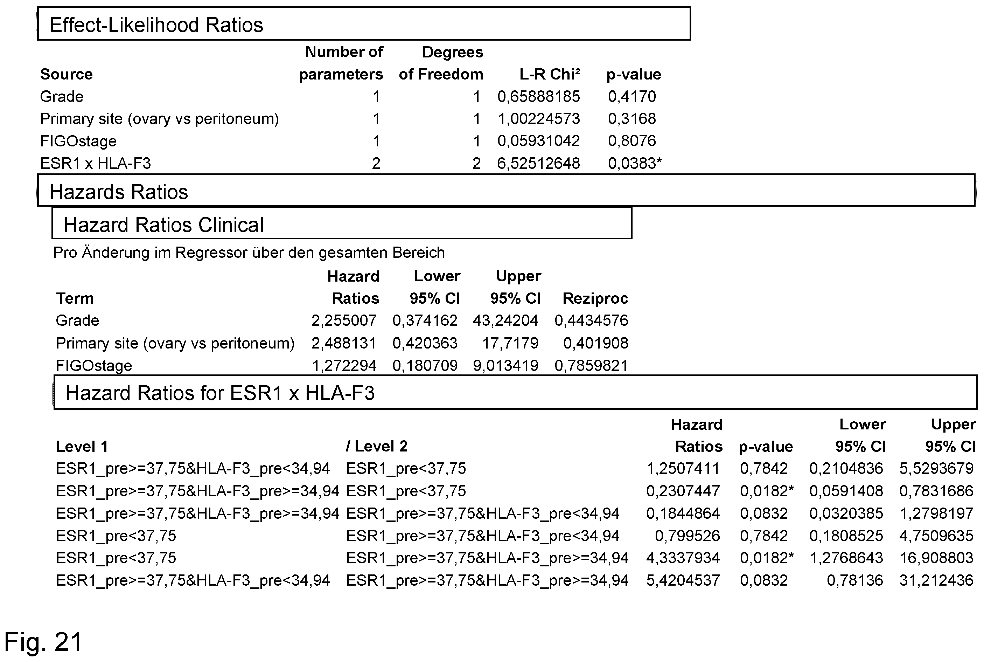

**Figure 21** depicts a multivariate analysis for OS using cox proportional hazards models including Grade, FIGO stage, Primary site and the combination of ESR1 and HLA-F3 mRNA expression.

The above-mentioned data analysis requires the adjustments of the individual HLA-F3 mRNA level to housekeeping gene to get the normalized expression levels depicted as 40-DCT values. Interestingly the genomic locus of HLA-F3 contains at the 3' genomic location at exon 8 on the reverse strand an anti-sense gene denoted as HLA-F3 AS, which might of importance for gene expression regulation and protein translation of the HLA-F3 mRNA. To investigate the relevance of the putative anti-sense transcript and to get rid of the necessity of a housekeeper normalization a gene ratio of HLA-F3 and HLA-F AS1 was investigated.

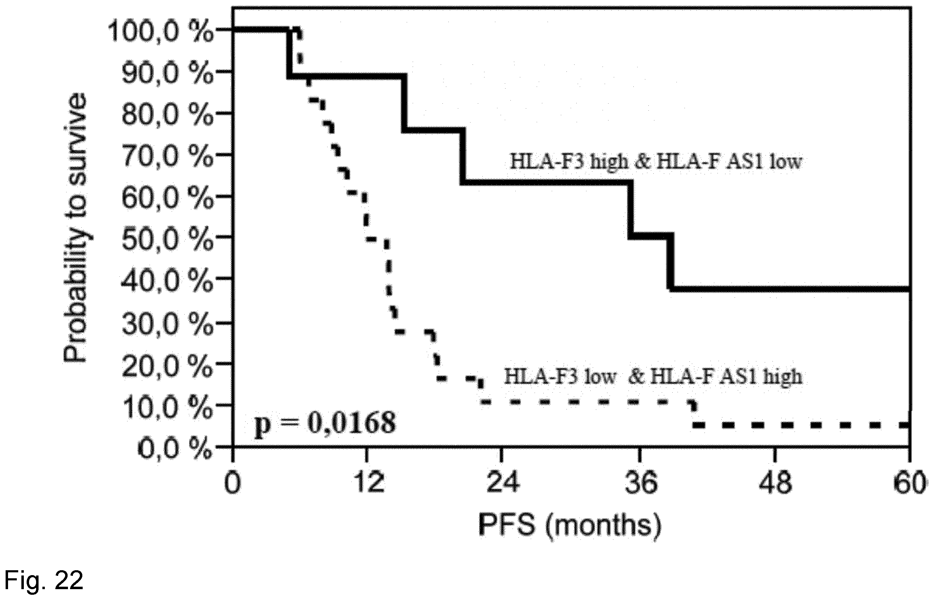

**Figure 22** depicts a Kaplan Meier Plot displaying overall survival (OS) probability based on stratification HLA-F3 and HLA-F AS1 mRNA expression as quantified by RT-qPCR assay from fresh tissues of advanced ovarian cancer patients (n=27). Gene ratios free of housekeeper determination instead of relative mRNA expression is determined by the 40-DCT method using CALM2 as reference gene are displayed. Patients, whose tumor exhibited high HLAF gene ratio expression (>= 2,35) exhibited good survival (black, solid line). Patients, whose tumor exhibited low gene ratio (< 2,35) exhibited worse survival (black, dashed line).

As depicted in **Figure 22****,** Kaplan Meier analysis proved the significance of predicting progression free survival by combining HLA-F3 and HLA-F AS1 mRNA expression. Patients exhibiting high HLA-F3 mRNA and simultaneously lower levels of HLA-F AS1 mRNA expression in the primary ovarian cancer tissue had a median progression free survival of 38,7 months (gene ratio >= 2,35), while patients with low HLA-F3 and high HLA-F AS1 mRNA expression (gene ratio < 2,35) exhibited diminished median progression free survival of 12,6 months.

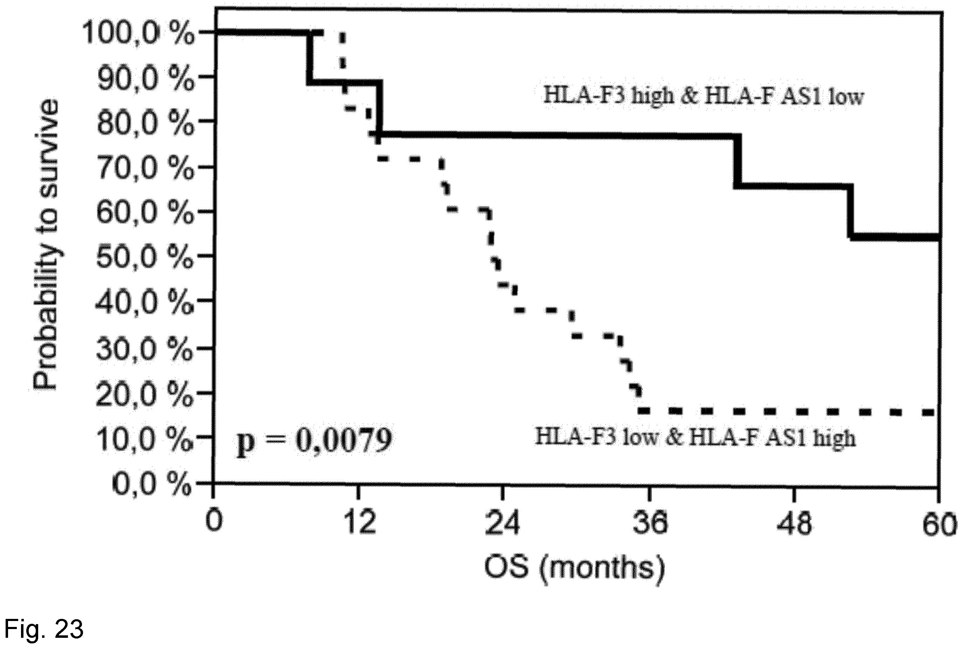

**Figure 23** depicts a Kaplan Meier Plot displaying overall survival (OS) probability based on stratification HLA-F3 and HLA-F AS1 mRNA expression as quantified by RT-qPCR assay from fresh tissues of advanced ovarian cancer patients (n=27). Gene ratios free of housekeeper determination instead of relative mRNA expression is determined by the 40-DCT method using CALM2 as reference gene are displayed. Patients, whose tumor exhibited high HLAF gene ratio expression (>= 2,35) exhibited good survival (black, solid line). Patients, whose tumor exhibited low gene ratio (< 2,35) exhibited worse survival (black, dashed line).

Kaplan Meier analysis also proved the significance of predicting overall survival by the combination of HLA-F3 and HLA-F AS1 as calculated by gene ratio. As depicted in **Figure 23****,** patients exhibiting high HLA-F gene ratio (>= 2,35) expression in the primary ovarian cancer tissue had a median overall survival of 61,5 months, while patients with high ESR1and low HLA-F3 mRNA expression exhibited diminished median progression free survival of 23,1 months.

When adjusting for clinical parameters such as grade, FIGO stage and primary site (ovary versus peritoneum) the combinatorial mRNA stratification based on ESR1 and HLA-F3 mRNA expression remained to be an independent factor for predicting progression free survival with a L-R Chi² value of 4.71 (p=0,0301), while all other clinical factors except for Primary site (p=0,0479) were not significant. The hazard ratio of high HLAF3 and low HLA-F AS1 mRNA expression reached a hazard ratio of 0.2866 (p=0.0301).

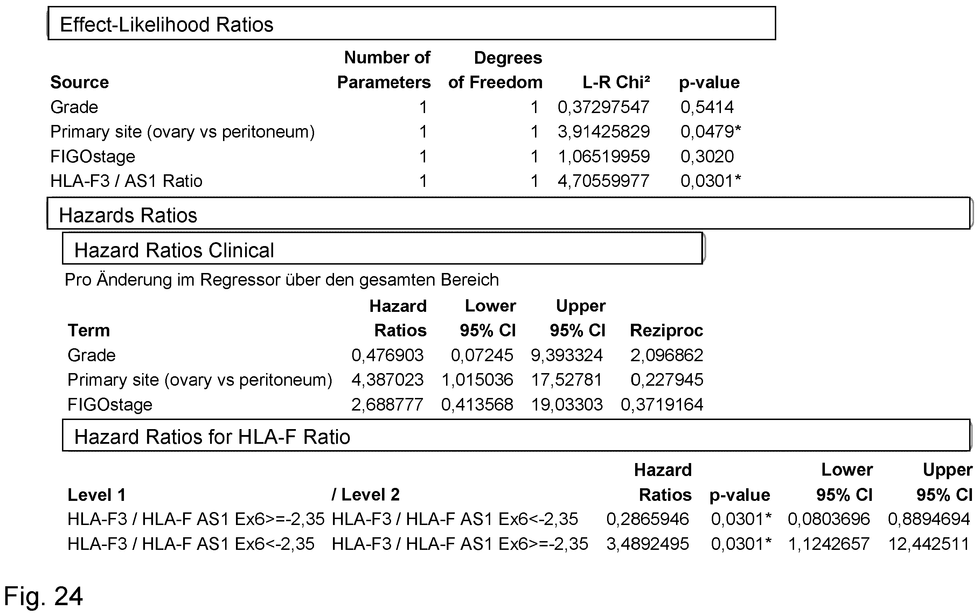

**Figure 24** depicts a multivariate analysis for PFS using cox proportional hazards models including Grade, FIGO stage, Primary site and the combination of HLA-F3 and HLA-F AS1.

In addition, when analyzing overall survival and adjusting for the clinical parameters grade, FIGO stage and primary site (ovary versus peritoneum) the combinatorial mRNA stratification based on HLA-F3 and HLA-F AS1 mRNA expression remained to be an independent factor for predicting progression free survival with a L-R Chi² value of 8.41 (p=0,0037), while all other clinical factors were not significant. The hazard ratio of high HLA-F3 / low HLA-F AS1 versus low HLA-F3 / high HLA-F AS1 reached a hazard ratio of 0.189 (p=0.0037).

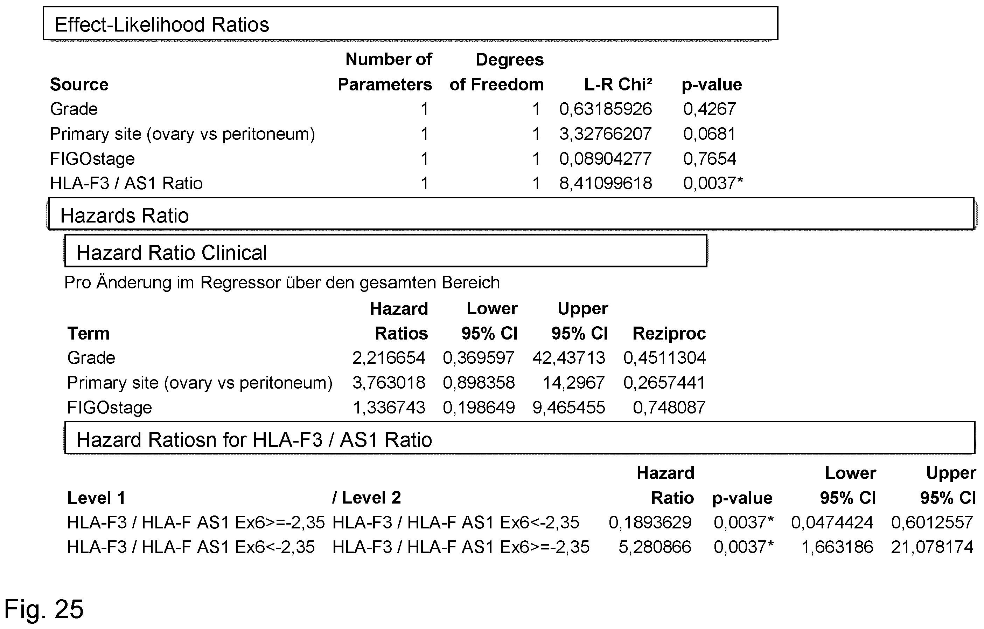

**Figure 25** depicts a multivariate analysis for OS using cox proportional hazards models including Grade, FIGO stage, Primary site and the combination of HLA-F3 and HLA-F AS1.

### Example 5: HLA profiling in advanced, chemotherapy refractory urothelial cancer

TUR biopsies and cystectomy samples from primary tumors being refractory to chemotherapy and thereafter undergoing first or second line immuneoncology ("IO") treatment by PD-1 and PD-L1 checkpoint inhibitor drugs (i.e. Atezolizumab, Nivolumab and Pembrolizumab) were analyzed for HLA expression and associated with histopathological and molecular parameters as well as response to IO treatment and disease specific survival after IO.

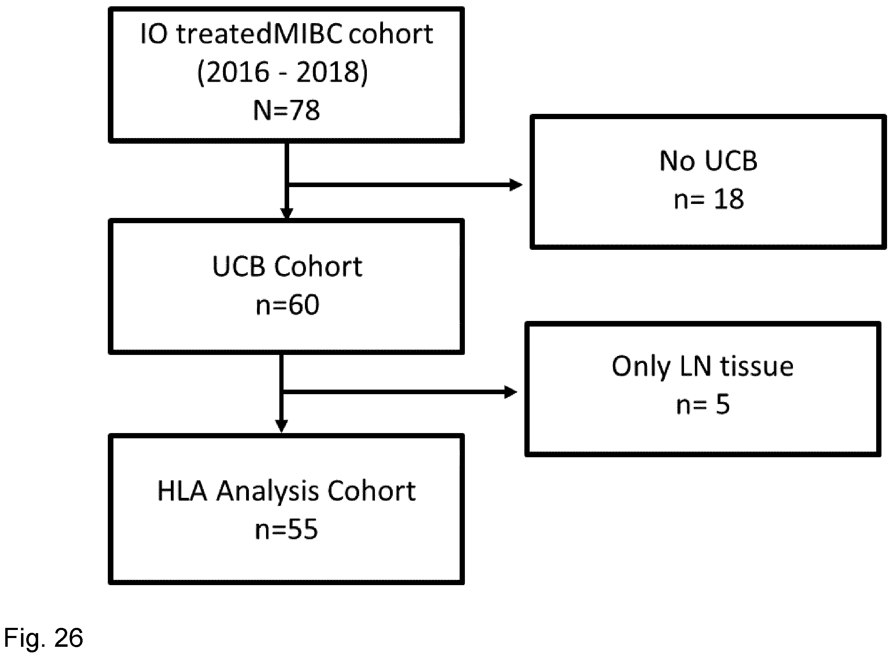

Seventy-two newly diagnosed patients with histologically confirmed urothelial cancer, including bladder cancer and upper urothelial tract carcinoma were enrolled in the study between 2016 and 2018. Nivolumab, Pembrolizumab and Atezumab were given as 1st, 2nd and 3rd line mono-treatment according to approved instructions. All hematoxylin-eosin (HE) stained tumor tissue sections from samples of the cohort were evaluated and classified according to TNM-classification (2017) of the UICC by two uro-pathologists. Rare histological variants were classified according to the World Health Organization (WHO 2016) classification of genitourinary tumors. After central histopathological review 18 tissues were excluded for not having sufficient tumor material or not being urothelial cancer. From 5 patients only lymph node tissue was available and therefor excluded from primary analysis of prognostic and/or predictive effects of HLA gene expression (see Figure 26; Consort Diagram).

**Figure 26** depicts a consort diagram of advanced or metastatic urothelial cancer cohort. After exclusion of FFPE blocks with insufficient and/or lymph node tissues, tissues of 55 patients were available for analysis.

For mRNA detection, RNA was extracted from FFPE tissue from TUR biopsies, cystectomy and corresponding mapping bladder tissue using commercial kits (Xtract, Stratifyer). For each reaction, 2,5 µl total RNA extracted from FFPE sections were mixed with 2,5 µl assay-mix, 2,5 µl enzyme-mix and 2,5 µl water in one well of a 96-well-optical reaction plate. Measurements of the PCR reaction were done according to the instructions of the manufacturer with a Versant kPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles). The relative mRNA expression was associated with response to IO treatment determined based on RECIST criteria as assessed at the individual sites and with disease specific survival as determined from start of IO treatment to cancer specific death. Partition testing using biostatistical JMP SAS 9.0.0 (SAS, Cary, North Carolina, USA) were performed to evaluate the possible differences in response to IO treatment.

For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant or selected splice variants as appropriate. All primer pairs were checked for specificity by conventional PCR reactions. After further optimization of the primers/probes, the primers and probes listed in the Table(s) above gave the best results. These primers/probes are superior to primers/probes known from the prior art, e.g., in terms of specificity and amplification efficiency. To standardize the amount of sample RNA, the CALM2 was selected as reference gene, since they were not differentially regulated in the samples analyzed. TaqMan^{®} validation experiments were performed showing that the efficiencies of the target and the control amplifications were approximately equal, which is a prerequisite for the relative quantification of gene expression by the comparative ΔCT method.

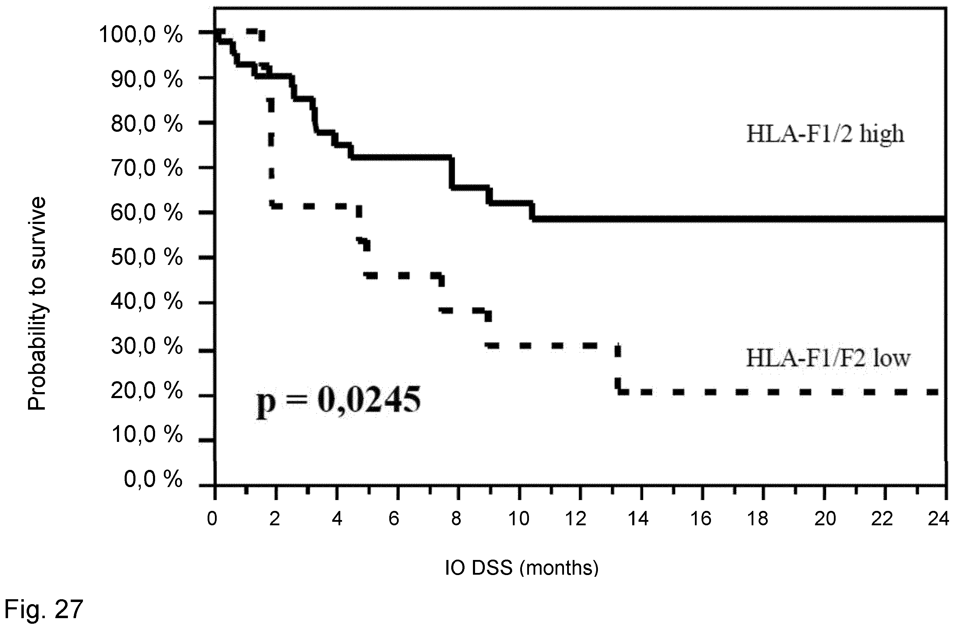

**Figure 27** depicts a Kaplan Meier Plot displaying disease specific survival (DSS) probability from muscle invasive bladder cancer patients having locally advanced or metastatic UBC (n=55) based on stratification by HLA-F1/F2 expression as quantified by RT-qPCR assay. Relative mRNA expression is determined by the 40-DCT method using CALM2 as reference gene.

As depicted in Figure 27 high HLA-F1/2 mRNA expression (>= 34,63) was significantly associated with better disease specific survival with HLA-F1/F2 positive patients having a survival probability of 60% after 2 years, while HLA-F1/F2 Exon negative patients had a survival probability of 20% after 2 years (p=0,0245).

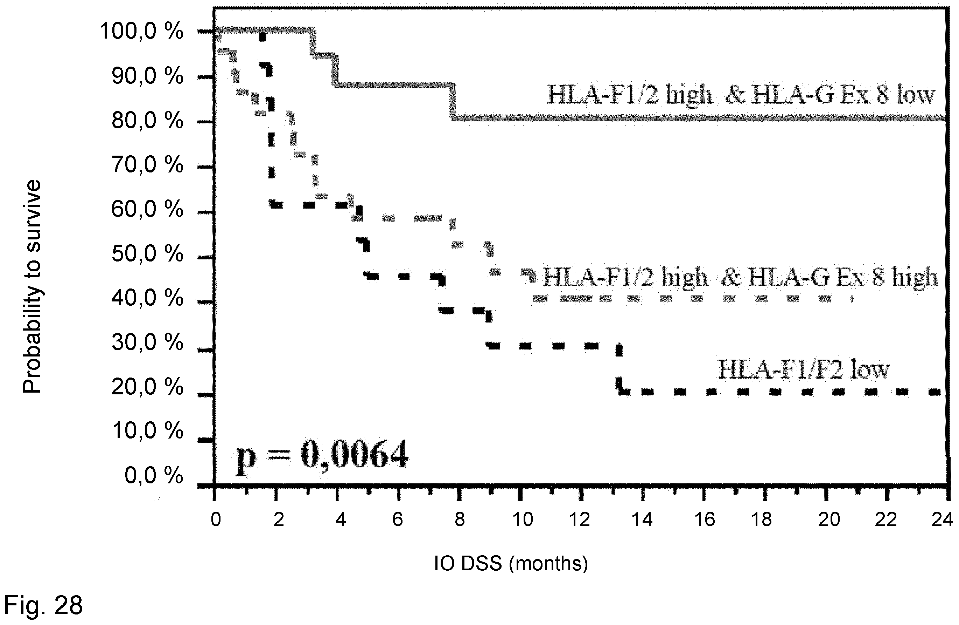

**Figure 28** depicts a Kaplan Meier Plot displaying disease specific survival (DSS) probability from muscle invasive bladder cancer patients having locally advanced or metastatic UBC (n=55) based on stratification by HLA-F 1/F2 and HLA-G Exon 8 mRNA expression as quantified by RT-qPCR assay. Relative mRNA expression is determined by the 40-DCT method using CALM2 as reference gene.

As depicted in Figure 28, when combining high HLA-F1/2 mRNA expression (>= 34,63) with HLA-G Ex8 mRNA expression (>= 30,16) the predictive value could be improved. Interestingly, high HLA-F1/F2 having low HLA-G mRNA expression was significantly associated with better disease specific survival with HLA-F1/F2 positive / HLA-G Ex8 negative patients having a survival probability of 80% after 2 years, while HLA-F1/F2 Exon negative patients had a survival probability of 20% after 2 years and HLA-F1/F2 positive /HLA-G Ex 8 positive patients had a survival probability of 40% after 2 years (p=0,0245).

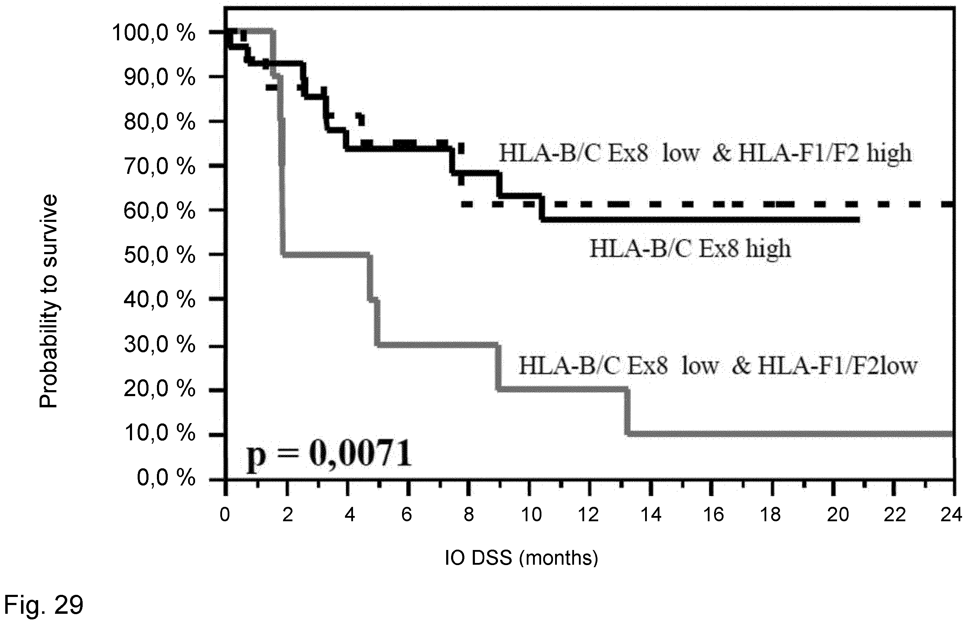

**Figure 29** depicts a Kaplan Meier Plot displaying disease specific survival (DSS) probability from muscle invasive bladder cancer patients having locally advanced or metastatic UBC (n=55) based on stratification by HLA-F1/F2 and HLA-B/C Exon 8 mRNA expression as quantified by RT-qPCR assay. Relative mRNA expression is determined by the 40-DCT method using CALM2 as reference gene.

As depicted in Figure 29, when combining high HLA-F1/2 mRNA expression (>= 34,63) with HLA-B/C Ex8 mRNA expression (>= 34,2) high HLA-B/C was significantly associated with better disease specific survival having a survival probability of 60% after 2 years, while HLA-B/C Exon 8 and HLA-F 1/F2 negative patients had a poor survival probability of 10% after 2 years and HLA-B/C negative & HLA-F 1/F2 positive patients had a good survival probability of 60% after 2 years (p=0,0071).

In contrast, when just looking at HLA-B/C Exon 8 mRNA expression no significant value could be determined (p=0,2127):

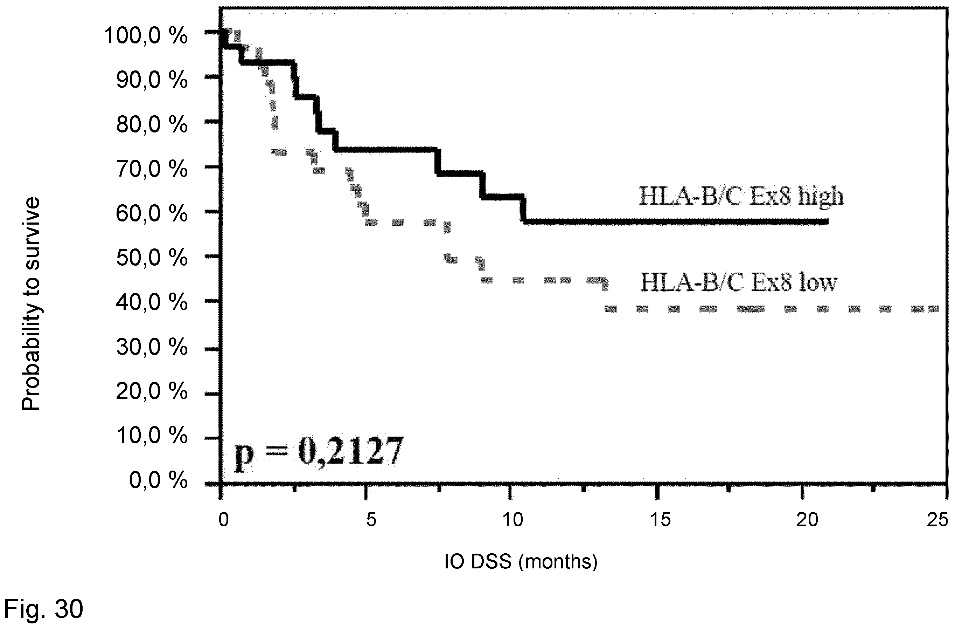

**Figure 30** depicts a Kaplan Meier Plot displaying disease specific survival (DSS) probability from muscle invasive bladder cancer patients having locally advanced or metastatic UBC (n=55) based on stratification by HLA-B/C Exon 8 mRNA expression as quantified by RT-qPCR assay. Relative mRNA expression is determined by the 40-DCT method using CALM2 as reference gene.

As depicted in Figure 30, patients with high HLA-B/C Ex8 mRNA expression (>= 34,2) had not a significantly better disease specific survival than patients with low HLA-B/C expression, having a survival probability of 60% after 2 years, while HLA-B/C Exon 8 negative patients had a poor survival probability of 40% after 2 years. This demonstrates that the combination of HLA expression is superior for survival prediction.

### SEQUENCE LISTING

SEQ ID NO. 1
   gtaacttctt ccttccctat taaaattaga
SEQ ID NO. 2
   tttactttct caaattcttg ccatgagagg ttgatg
SEQ ID NO. 3
   tggactctgg aaggttctca tg
SEQ ID NO. 4
   ccatctctgt ctcaaattca tggt
SEQ ID NO. 5
   cactgagctg caacttctta cttccctaat ga
SEQ ID NO. 6
   caggtcttta tttgctctct caacttc
SEQ ID NO. 7
   ggccggagta ttgggaaga
SEQ ID NO. 8
   caaggcccac gcacagactg aca
SEQ ID NO. 9
   gcagggtctg caggttcatt
SEQ ID NO. 10
   ctgcggctca gatctccaa
SEQ ID NO. 11
   cgcaagtgtg aggcggccaa t
SEQ ID NO. 12
   caggtaggct ctcctttgtt cag
SEQ ID NO. 13
   caccaccctg tctttgacta tgag
SEQ ID NO. 14
   accctgaggt gctgggccct g
SEQ ID NO. 15
   agtatgatct ccgcagggta gaag
SEQ ID NO. 16
   catccccatc atgggtatcg
SEQ ID NO. 17
   tgctggcctg gttgtecttg ca
SEQ ID NO. 18
   ccgcagctcc agtgactaca
SEQ ID NO. 19
   gaccctcttc ctcatgctga ac
SEQ ID NO. 20
   cattccttcc ccaatcacct ttcctgtt
SEQ ID NO. 21
   catcccagcc ccttttctg
SEQ ID NO. 22
   ttcatcgcca tgggctacg
SEQ ID NO. 23
   cgacacgcag ttcgtgcggt tc
SEQ ID NO. 24
   atcctcggac acgccgagt
SEQ ID NO. 25
   ccgaaccctc ttcctgctgc
SEQ ID NO. 26
   cgagacctgg gcgggctccc
SEQ ID NO. 27
   gcgctgaaat acctcatgga
SEQ ID NO. 28
   gagagaacct gcggatcgc
SEQ ID NO. 29
   agcgagggcg gttctcacac catg
SEQ ID NO. 30
   ccacgtcgca gccatacat
SEQ ID NO. 31
   tggccctgac cctgaccc
SEQ ID NO. 32
   agacctgggc gcgctcccac
SEQ ID NO. 33
   cgggccggga catggt
SEQ ID NO. 34
   gagcgagctg agtggttgtg
SEQ ID NO. 35
   tcgcgtctcg gaaaccggta gc
SEQ ID NO. 36
   agtcagttgg tcagccatgc t
SEQ ID NO. 37
   tgtgagacag cttccttgtg tg
SEQ ID NO. 38
   aattctgcta cattgatate ttgcttctca gtcc
SEQ ID NO. 39
   tatgtatgtt cgtgaggcac aagtg
SEQ ID NO. 40
   ctttggcttc ggctttagga
SEQ ID NO. 41
   cttcgttctt ggcaccatct tatgaaaagg gt
SEQ ID NO. 42
   catgggtctt gcaacttact ttagaat
SEQ ID NO. 43
   ccaagaaggc agtgttgaaa gat
SEQ ID NO. 44
   atccacatgt cacccacctt ccgg
SEQ ID NO. 45
   gggtgctctt ccaaggatat ttg
SEQ ID NO. 46
   aggattgcgg cctgttg
SEQ ID NO. 47
   agagtagtgt cttgggcccc agctga
SEQ ID NO. 48
   cagggcattg gatgttgata ttc
SEQ ID NO. 49
   actcccatgc agagaagaag ctc
SEQ ID NO. 50
   cacctgtgga gagaggattg cggc
SEQ ID NO. 51
   ctggggccca agacactact c
SEQ ID NO. 52
   tgctccgcag atacttggag aat
SEQ ID NO. 53
   ctacagcgc gcagagcagt ctccc
SEQ ID NO. 54
   gccagcaacg atgcccac
SEQ ID NO. 55
   tcagatagaa acagagggag ctactct
SEQ ID NO. 56
   ctgcagccta ctcagtggtc agcgg
SEQ ID NO. 57
   gagaaataag cttgaccacc atgtta
SEQ ID NO. 58
   tggagttgct ccgcagatac t
SEQ ID NO. 59
   cctttggagg atctgcgcgc tg
SEQ ID NO. 60
   agatggggtg gtgggca
SEQ ID NO. 61
   tcagatagaa acagagggag ctactc
SEQ ID NO. 62
   caggctgcag tgtgagacag cttccttg
SEQ ID NO. 63
   tttatgtatg ttcgtgaggc acaa
SEQ ID NO. 64
   aggaatatgc agaggagttc agga
SEQ ID NO. 65
   agtatctgcg gagcaactcc aggcactc
SEQ ID NO. 66
   gactgctctg cgcgctgt
SEQ ID NO. 67
   aggaatatgc agaggagttc agga
SEQ ID NO. 68
   agtatctgcg gagcaactcc aggcactc
SEQ ID NO. 69
   ggaggatctg cgcgctgt

## Claims

1. Method of determining individual HLA patterns of a tumor, comprising:
- determining a first expression level of RNA transcript encoding a first region of a first HLA gene;
- determining at least a second expression level of sense or antisense RNA transcript of at least one second region of at least one second HLA gene; and
- comparing the determined first and second expression levels to obtain an individual HLA pattern,
wherein the first HLA gene is selected from the group consisting of genes encoding HLA-A, HLA-B, HLA-C; and
wherein the second HLA gene is selected from the group consisting of genes encoding HLA-D, HLA-E, HLA-F, HLA-G, HLA-H, HLA-J.

2. Method according to claim 1, wherein
- one of the first region and second region comprises an exon-exon-boundary and the other one of the first region and second region comprises no exon-exon-boundary, or wherein
- the first region comprises no exon-exon-boundary and the second region comprises no exon-exon-boundary, or wherein
- the first region comprises an exon-exon-boundary and the second region comprises an exon-exon-boundary.

3. Method according to one of the preceding claims, wherein
- at least one of the first region and second region encodes a signal peptide region of a HLA group, and/or
- at least one of the first region and second region encodes a transmembrane region of a HLA group.

4. Method according to one of the preceding claims, further comprising
- determining whether the individual HLA pattern is predominantly soluble or membrane-bound based on the comparison of the first and second expression levels.

5. Method according to one of the preceding claims, further comprising
- determining HLA isoforms based on the comparison of the first and second expression levels.

6. Method according to one of the preceding claims, further comprising determining one or more further expression levels for one or more further regions of a gene encoding for a HLA group and wherein the comparing is further based on the determined further expression levels to obtain the individual HLA pattern.

7. Method according to one of the preceding claims, wherein the comparing includes the formation of an expression level ratio.

## Patentansprüche

1. Verfahren zur Bestimmung individueller HLA-Muster eines Tumors, umfassend:
- Bestimmen eines ersten Expressionsniveaus eines RNA-Transkripts, das für eine erste Region eines ersten HLA-Gens kodiert;
- Bestimmen mindestens eines zweiten Expressionsniveaus eines Sense- oder Antisense-RNA-Transkripts mindestens einer zweiten Region mindestens eines zweiten HLA-Gens; und
- Vergleichen der ermittelten ersten und zweiten Expressionsniveaus, um ein individuelles HLA-Muster zu erhalten,
wobei das erste HLA-Gen aus der Gruppe ausgewählt ist, die aus Genen besteht, die für HLA-A, HLA-B, HLA-C kodieren; und
wobei das zweite HLA-Gen aus der Gruppe ausgewählt ist, die aus Genen besteht, die für HLA-D, HLA-E, HLA-F, HLA-G, HLA-H und HLA-J kodieren.

2. Verfahren nach Anspruch 1, wobei
- eine von der ersten Region und der zweiten Region eine Exon-Exon-Grenze umfasst und die andere der ersten Region und der zweiten Region keine Exon-Exon-Grenze umfasst, oder wobei
- die erste Region keine Exon-Exon-Grenze umfasst und die zweite Region keine Exon-Exon-Grenze umfasst, oder wobei
- die erste Region eine Exon-Exon-Grenze umfasst und die zweite Region eine Exon-Exon-Grenze umfasst.

3. Verfahren gemäß einem der vorstehenden Ansprüche, wobei
- mindestens eine der ersten Region und der zweiten Region eine Signalpeptidregion einer HLA-Gruppe kodiert, und/oder
- mindestens eine der ersten Region und der zweiten Region eine Transmembranregion einer HLA-Gruppe kodiert.

4. Verfahren nach einem der vorstehenden Ansprüche, ferner umfassend
- Bestimmen, ob das individuelle HLA-Muster überwiegend löslich oder membrangebunden ist, basierend auf dem Vergleich des ersten und des zweiten Expressionsniveaus.

5. Verfahren nach einem der vorstehenden Ansprüche, ferner umfassend
- Bestimmen von HLA-Isoformen auf der Grundlage des Vergleichs der ersten und zweiten Expressionsniveaus.

6. Verfahren nach einem der vorstehenden Ansprüche, ferner umfassend Bestimmen eines oder mehrerer weiterer Expressionsniveaus für eine oder mehrere weitere Regionen eines Gens, das für eine HLA-Gruppe kodiert, und wobei der Vergleich ferner auf den bestimmten weiteren Expressionsniveaus basiert, um das individuelle HLA-Muster zu erhalten.

7. Verfahren nach einem der vorstehenden Ansprüche, wobei der Vergleich die Bildung eines Expressionsniveau-Verhältnisses aufweist.

## Revendications

1. Méthode de détermination de motifs HLA individuels d'une tumeur, comprenant :
- la détermination d'un premier niveau d'expression de transcription d'ARN codant pour une première région d'un premier gène HLA ;
- la détermination d'au moins un second niveau d'expression d'une transcription d'ARN sens ou antisens d'au moins une seconde région d'au moins un second gène HLA ; et
- la comparaison des premier et second niveaux d'expression déterminés pour obtenir un motif HLA individuel,
dans laquelle le premier gène HLA est sélectionné dans le groupe consistant en gènes codant pour HLA-A, HLA-B, HLA-C ; et
dans laquelle le second gène HLA est sélectionné dans le groupe consistant en gènes codant pour HLA-D, HLA-E, HLA-F, HLA-G, HLA-H, HLA-J.

2. Méthode selon la revendication 1, dans laquelle
- l'une de la première région et de la seconde région comprend une limite exon-exon et l'autre de la première région et de la seconde région ne comprend pas de limite exon-exon, ou dans laquelle
- la première région ne comprend pas de limite exon-exon et la seconde région ne comprend pas de limite exon-exon, ou dans laquelle
- la première région comprend une limite exon-exon et la seconde région comprend une limite exon-exon.

3. Méthode selon l'une des revendications précédentes, dans laquelle
- au moins l'une de la première région et de la seconde région code pour une région de peptide signal d'un groupe HLA, et/ou
- au moins l'une de la première région et de la seconde région code pour une région transmembranaire d'un groupe HLA.

4. Méthode selon l'une des revendications précédentes, comprenant en outre
- le fait de déterminer si le motif HLA individuel est principalement soluble ou lié à la membrane sur la base de la comparaison des premier et second niveaux d'expression.

5. Méthode selon l'une des revendications précédentes, comprenant en outre
- la détermination d'isoformes HLA sur la base de la comparaison des premier et second niveaux d'expression.

6. Méthode selon l'une des revendications précédentes, comprenant en outre la détermination d'un ou plusieurs niveaux d'expression supplémentaires pour une ou plusieurs régions supplémentaires d'un gène codant pour un groupe HLA et dans laquelle la comparaison est en outre basée sur les niveaux d'expression supplémentaires déterminés pour obtenir le motif HLA individuel.

7. Méthode selon l'une des revendications précédentes, dans laquelle la comparaison comporte la formation d'un rapport de niveau d'expression.
